# EUROPEAN PATENT SPECIFICATION

(11) **EP 1 307 234 B1**
(45) Date of publication and mention of the grant of the patent: **02.04.2008**
(21) Application number: 00950382.2
(22) Date of filing: 13.07.2000
(51) Int. Cl.: A61K 48/00

(54) **METHODS FOR TREATMENT OF HYPERPROLIFERATIVE DISEASES USING HUMAN MDA-7**
METHODEN ZUR BEHANDLUNG VON HYPERPROLIFERATIVEN KRANKHEITEN MIT MENSCHLICHEM MDA-7
METHODES DE TRAITEMENT DE MALADIES HYPERPROLIFERATIVES, AU MOYEN DE LA PROTEINE HUMAINE MDA-7

(30) Priority: 15.07.1999 US 144354 P; 28.04.2000 US 200768 P
(43) Date of publication of application: 07.05.2003
(60) Divisional application: 06022661.0
(73) Proprietor: Introgen Therapeutics, Inc., Austin, TX 78701 (US)
(72) Inventor: MHASHILKAR, Abner, Houston, TX 77031 (US); SCHROCK, Bob, Houston TX 77025 (US); CHADA, Sunil, Missouri City TX 77459 (US)
(74) Representative: Bösl, Raphael Konrad
(86) International application number: PCT/US2000/019392
(87) International publication number: WO 2001/005437

(56) References cited:
- WO-A-95/11986
- WO-A-98/28425
- SU ZAO-ZHONG ET AL: "The cancer growth suppressor gene mda-7 selectively induces apoptosis in human breast cancer cells and inhibits tumor growth in nude mice." PROCEEDINGS OF THE NATIONAL ACADEMY OF SCIENCES OF THE UNITED STATES, vol. 95, no. 24, 24 November 1998 (1998-11-24), pages 14400-14405, XP002151807 Nov. 24, 1998 ISSN: 0027-8424 cited in the application
- JIANG HONGPING ET AL: "The melanoma differentiation associated gene mda-7 suppresses cancer cell growth." PROCEEDINGS OF THE NATIONAL ACADEMY OF SCIENCES OF THE UNITED STATES, vol. 93, no. 17, 1996, pages 9160-9165, XP002151808 1996 ISSN: 0027-8424 cited in the application

### SEQUENCE LISTING

<110> MHASHILKAR, ABNER
   SCHROCK, BOB
   CHADA, SUNIL
<120> METHODS FOR TREATMENT OF HYPERPROLIFERATIVE DISEASES
   USING HUMAN MDA-7
<130> INGN:090P
<140> UNKNOWN
   <141> 2000-07-13
<150> 60/200,768
   <151> 2000-04-28
<160> 60/144,354
   <161> 1999-07-15
<170> 2
<171> PatentIn Ver. 2.0
<210> 1
   <211> 718
   <212> DNA
   <213> Human
<400> 1 .
   acaagacatg actgtgatga ggagctgctt tcgccaattt aacaccaaga agaattgagg 60
   ctgcttggga ggaaggccag gaggaacacg agactgagag atgaattttc aacagaggct 120
   gcaaagcctg tggactttag ccagaccctt ctgccctcct ttgctggcga cagcctctca 180
   aatgcagatg gttgtgctcc cttgcctggg ttttaccctg cttctctgga gccaggtatc 240
   aggggcccag ggccaagaat tccactttgg gccctgccaa gtgaaggggg ttgttcccca 300
   gaaactgtgg gaagccttct gggctgtgaa agacactatg caagctcagg ataacatcac 360
   gagtgcccgg ctgctgcagc aggaggttct gcagaacgtc tcggatgctg agagctgtta 420
   ccttgtccac accctgctgg agttctactt gaaaactgtt ttcaaaaact accacaatag 480
   aacagttgaa gtcaggactc tgaagtcatt ctctactctg gccaacaact ttgttctcat 540
   cgtgtcacaa ctgcaaccca gtcaagaaaa tgagatgttt tccatcagag acagtgcaca 600
   caggcggttt ctgctattcc ggagagcatt caaacagttg gacgtagaag cagctctgac 660
   caaagccctt ggggaagtgg acattcttct gacctggatg cagaaattct acaagctc   718
<210> 2
   <211> 206
   <212> PRT
   <213> Human
<400> 2
   Met Asn Phe Gln Gln Arg Leu Gln Ser Leu Trp Thr Leu Ala Arg Pro
    1               5                  10                  15
   Phe Cys Pro Pro Leu Leu Ala Thr Ala Ser Gln Met Gln Met Val Val
               20                  25                  30
   Leu Pro Cys Leu Gly Phe Thr Leu Leu Leu Trp Ser Gln Val Ser Gly
           35                  40                  45
   Ala Gln Gly Gln Glu Phe His Phe Gly Pro Cys Gln Val Lys Gly Val
        50                  55                  60
   Val Pro Gln Lys Leu Trp Glu Ala Phe Trp Ala Val Lys Asp Thr Met
    65                  70                  75                  80
   Gln Ala Gln Asp Asn Ile Thr Ser Ala Arg Leu Leu Gln Gln Glu Val
                    85                  90                  95
   Leu Gln Asn Val Ser Asp Ala Glu Ser Cys Tyr Leu Val His Thr Leu
               100                 105                 110
   Leu Glu Phe Tyr Leu Lys Thr Val Phe Lys Asn Tyr His Asn Arg Thr
           115                 120                 125
   Val Glu Val Arg Thr Leu Lys Ser Phe Ser Thr Leu Ala Asn Asn Phe
       130                 135                 140
   Val Leu Ile Val Ser Gln Leu Gln Pro Ser Gln Glu Asn Glu Met Phe
   145                 150                 155                 160
   Ser Ile Arg Asp Ser Ala His Arg Arg Phe Leu Leu Phe Arg Arg Ala
                   165                 170                 175
   Phe Lys Gln Leu Asp Val Glu Ala Ala Leu Thr Lys Ala Leu Gly Glu
               180                 185                 190
   Val Asp Ile Leu Leu Thr Trp Met Gln Lys Phe Tyr Lys Leu
           195                 200                 205

### SEQUENCE LISTING

<110> MASHILKAR, ABNER
   SCHROCK, BOB
   CHADA, SUNIL
<120> METHODS FOR TREATMENT OF HYPERPROLIFERATIVE DISEASES
   USING HUMAN MDA-7
<130> INGN:090-WO
<140> PCT/US 00/19392
   <141> 2000-07-13
<140> SN 60/200,768
   <141> 1999-07-15
<160> 2
<170> PatentIn Ver. 2.0
<210> 1
   <211> 718
   <212> DNA
   <213> Homo Sapiens
<400> 1
   acaagacatg actgtgatga ggagctgctt tcgccaattt aacaccaaga agaattgagg 60
   ctgcttggga ggaaggccag gaggaacacg agactgagag atgaattttc aacagaggct 120
   gcaaagcctg tggactttag ccagaccctt ctgccctcct ttgctggcga cagcctctca 180
   aatgcagatg gttgtgctcc cttgcctggg ttttaccctg cttctctgga gccaggtatc 240
   aggggcccag ggccaagaat tccactttgg gccctgccaa gtgaaggggg ttgttcccca 300
   gaaactgtgg gaagccttct gggctgtgaa agacactatg caagctcagg ataacatcac 360
   gagtgcccgg ctgctgcagc aggaggttct gcagaacgtc tcggatgctg agagctgtta 420
   ccttgtccac accctgctgg agttctactt gaaaactgtt ttcaaaaact accacaatag 480
   aacagttgaa gtcaggactc tgaagtcatt ctctactctg gccaacaact ttgttctcat 540
   cgtgtcacaa ctgcaaccca gtcaagaaaa tgagatgttt tccatcagag acagtgcaca 600
   caggcggttt ctgctattcc ggagagcatt caaacagttg gacgtagaag cagctctgac 660
   caaagccctt ggggaagtgg acattcttct gacctggatg cagaaattct acaagctc   718
<210> 2
   <211> 206
   <212> PRT
   <213> Homo Sapiens
<400> 2
   Met Asn Phe Gln Gln Arg Leu Gln Ser Leu Trp Thr Leu Ala Arg Pro
   1               5                   10                  15
   Phe Cys Pro Pro Leu Leu Ala Thr Ala Ser Gln Met Gln Met Val Val
               20                  25                  30
   Leu Pro Cys Leu Gly Phe Thr Leu Leu Leu Trp Ser Gln Val Ser Gly
           35                  40                  45
   Ala Gln Gly Gln Glu Phe His Phe Gly Pro Cys Gln Val Lys Gly Val
       50                  55                  60
   Val Pro Gln Lys Leu Trp Glu Ala Phe Trp Ala Val Lys Asp Thr Met
   65                  70                  75                  80
   Gln Ala Gln Asp Asn Ile Thr Ser Ala Arg Leu Leu Gln Gln Glu Val
                   85                  90                  95
   Leu Gln Asn Val Ser Asp Ala Glu Ser Cys Tyr Leu Val His Thr Leu
               100                 105                 110
   Leu Glu Phe Tyr Leu Lys Thr Val Phe Lys Asn Tyr His Asn Arg Thr
           115                 120                 125
   Val Glu Val Arg Thr Leu Lys Ser Phe Ser Thr Leu Ala Asn Asn Phe
       130                 135                 140
   Val Leu Ile Val Ser Gln Leu Gln Pro Ser Gln Glu Asn Glu Met Phe
   145                 150                 155                 160
   Ser Ile Arg Asp Ser Ala His Arg Arg Phe Leu Leu Phe Arg Arg Ala
                   165                 170                 175
   Phe Lys Gln Leu Asp Val Glu Ala Ala Leu Thr Lys Ala Leu Gly Glu
               180                 185                 190
   Val Asp Ile Leu Leu Thr Trp Met Gln Lys Phe Tyr Lys Leu
           195                 200                 205

## Description

### BACKGROUND OF THE INVENTION

### A. FIELD OF THE INVENTION

The present invention relates generally to the field of gene therapy. More particularly, it concerns a method of administering a therapeutic nucleic acid for the treatment of hyperproliferative diseased as specified in the claims. In one embodiment, the invention relates to the expression of a nucleic acid encoding a truncated form of the human MDA-7 (mda7^{TF}) protein for the treatment of hyperproliferative diseases, while in other embodiments the invention involves the full-length form of the human MDA-7 polypeptide for the treatment of hyperproliferative diseases.

### B. DESCRIPTION OF RELATED ART

### 1. Gene Therapy

Gene therapy is an emerging field in biomedical research with a focus on the treatment of disease by the introduction of therapeutic recombinant nucleic acids into somatic cells of patients. Various clinical trials using gene therapies have been initiated and include the treatment of various cancers, AIDS, cystic fibrosis, adenosine deaminase deficiency, cardiovascular disease, Gaucher's disease, rheumatoid arthritis, and others. Currently, adenovirus is the preferred vehicle for the delivery of gene therapy agents. Advantages in using adenovirus as a gene therapy agent are high transduction efficiency, infection of non-dividing cells, easy manipulation of its genome, and low probability of non-homologous recombination with the host genome. The present invention describes a novel nucleic acid, encoding a truncated form of human MDA-7 (mda7^{TF}), for the treatment of hyperproliferative disease in humans. Furthermore, the present invention also describes a nucleic acid that encodes a soluble form of the MDA-7 protein and uses thereof.

### 2. MDA-7

The cDNA encoding the MDA-7 protein has been described by Jiang et al., 1995 (WO 9511986). The protein encoded by the mda-7 cDNA was recognized as a potential regulator of melanoma progression. Jiang *et al.* used a subtractive hybridization technique (Jiang *et al.,* 1995) to identify genes involved in the regulation of growth and differentiation in human melanoma cells. A cDNA library prepared by subtraction hybridization of cDNAs prepared form actively proliferating human HO-1 melanoma cells against cDNAs prepared from interferon-beta (IFN-β) and mezerin-differentiated human HO-1 melanoma cells was used to identify several melanoma differentiation associated (mda) cDNAs, including mda-7. The expression of mda-7 mRNA is inversely correlated with melanoma progression as demonstrated by increased mRNA levels in normal melanocytes as compared to primary and metastatic melanomas as well as decreased mda-7 mRNA expression in early vertical growth phase melanoma cells selected for enhanced tumor formation in nude mice.

The mda-7 cDNA encodes a novel, evolutionarily conserved protein of 206 amino acids with a predicted size of 23.8 kDa. The deduced amino acid sequence contains a hydrophobic stretch from about amino acid 26 to 45, which has characteristics of a signal sequence. The protein sequence shows no significant amino-acid sequence homology to known proteins or protein motifs with the exception of a 42 amino acid stretch that is 54% identical to interleukin 10 (IL-10). Structural analysis performed by Bazan *et al.* has determined that mda-7 (IL-BKW or IL-20) displays the structural characteristics of the cytokine family (WO 9828425). The structural characteristics and limited identity across a small stretch of amino acids implies an extracellular function for MDA-7.

Additional studies have shown that elevated expression of MDA-7 suppressed cancer cell growth *in vitro* and selectively induced apoptosis in human breast cancer cells as well as inhibiting tumorigenicity in nude mice (Jiang *et al.,* 1996; Su *et al.,* 1998). Jiang *et al.* (1996) report findings that MDA-7 is a potent growth suppressing gene in cancer cells of diverse origins including breast, central nervous system, cervix, colon, prostate, and connective tissue. A colony inhibition assay was used to demonstrate that elevated expression of mda-7 enhanced growth inhibition in human cervical carcinoma (HeLa), human breast carcinoma (MCF-7 and T47D), colon carcinoma (LS174T and SW480), nasopharyngeal carcinoma (HONE-1), prostate carcinoma (DU-145), melanoma (HO-1 and C8161), glioblastome multiforme (GBM-18 and T98G), and osteosarcoma (Saos-2). MDA-7 overexpression in normal cells (HMECs, HBL-100, and CREF-Trans6) showed limited growth inhibition indicating that mda-7 transgene effects are not manifest in normal cells. In summary, growth inhibition by elevated expression of MDA-7 is more effective *in vitro* in cancer cells than in normal cells. Su *et al.* (1998) reported investigations into the mechanism by which MDA-7 suppressed cancer cell growth. The studies reported that ectopic expression of MDA-7 in breast cancer cell lines MCF-7 and T47D induced apoptosis as detected by cell cycle analysis and TUNEL assay without an effect on the normal HBL-100 cells. Western blot analysis of cell lysates from cells infected with adenovirus mda-7 ("Ad-mda-7") showed an upregulation of the apoptosis stimulating protein BAX. Ad-mda-7 infection elevated levels of BAX protein only in MCF-7 and T47D cells and not normal HBL-100 or HMEC cells.

The bax gene plays an important role in inducing apoptosis. Increases in bax transcription may be in part responsible for the p53-regulated pathway of apoptosis-induction (Miyashita *et al.,* 1995). Overexpression of BAX and an increase in the Bax/Bel-2 protein ratio results in dissipation of mitochondrial membrane potential and release of cytochrome c (Rosse, 1998). The BAX protein binds directly to the mitochondrial porin channel (called voltage dependent anion channel, VDAC) and allows cytochrome c to pass through VDAC (Shimizu *et al.,* 1999). Cytochrome c complexes with Apaf-1 and this complex cleaves and activates caspase-9, an initiator caspase. The caspase cascade is activated from this initiator caspase. In some reported pathways of cell death, the caspase and Bcl-2 protein families play a key role in the regulation and execution of apoptosis.

Based on the established interactions among the known mediators of apoptosis, three classic pathways of apoptotic signaling in mammalian cell have emerged (Dragovich, 1998). The first one is initiated by the withdrawal of growth factors and is regulated by the Bcl-2 family of proteins. This pathway results in cytochrome c release from mitochondria, activation of Apaf-1 and triggering of the caspase cascade. The other well-established apoptosis pathway involves signaling by cell surface death receptors such as TNF or Fas which, through adapter molecules, can recruit and activate caspases. The third and least well-characterized pathway is initiated by DNA damage. This is regulated in part by proteins such as p53 and ATM . In all three of these pathways of cell death, the caspase and Bcl-2 protein families play key roles in regulation and execution of apoptosis.

### 3. Cancer

Normal tissue homeostasis is a highly regulated process of cell proliferation and cell death. An imbalance of either cell proliferation or cell death can develop into a cancerous state (Solyanik *et al.,* 1995; Stokke *et al.,* 1997; Mumby and Walter, 1991; Natoli *et al.,* 1998; Magi-Galluzzi *et al.,* 1998). For example, cervical, kidney, lung, pancreatic, colorectal and brain cancer are just a few examples of the many cancers that can result (Erlandsson, 1998; Kolmel, 1998; Mangray and King, 1998; Gertig and Hunter, 1997; Mougin *et al.,* 1998). In fact, the occurrence of cancer is so high, that over 500,000 deaths per year are attributed to cancer in the United States alone.

The maintenance of cell proliferation and cell death is at least partially regulated by proto-oncogenes. A proto-oncogene can encode proteins that induce cellular proliferation *(e.g., sis, erbB, src, ras and myc),* proteins that inhibit cellular proliferation *(e.g.. Rb, p16. p19, p21, p53, NFI and WT1)* or proteins that regulate programmed cell death *(e.g., bcl-2)* (Ochi *et al.,* 1998; Johnson and Hamdy, 1998; Liebermann *et al.,* 1998). However, genetic rearrangements or mutations to these proto-oncogenes, results in the conversion of a proto-oncogene into a potent cancer-causing oncogene. Often, a single point mutation is enough to transform a proto-oncogene into an oncogene. For example, a point mutation in the p53 tumor suppressor protein results in the complete loss of wild-type p53 function (Vogelstein and Kinzler, 1992; Fulchi *et al.,* 1998) and acquisition of "dominant" tumor promoting function.

Currently, there are few effective options for the treatment of many common cancer types. The course of treatment for a given individual depends on the diagnosis, the stage to which the disease has developed and factors such as age, sex and general health of the patient. The most conventional options of cancer treatment are surgery, radiation therapy and chemotherapy. Surgery plays a central role in the diagnosis and treatment of cancer. Typically, a surgical approach is required for biopsy and to remove cancerous growth. However, if the cancer has metastasized and is widespread, surgery is unlikely to result in a cure and an alternate approach must be taken. Radiation therapy, chemotherapy and immunotherapy are alternatives to surgical treatment of cancer (Mayer, 1998; Ohara, 1998; Ho *et al.,* 1998). Radiation therapy involves a precise aiming of high energy radiation to destroy cancer cells and much like surgery, is mainly effective in the treatment of non-metastasized, localized cancer cells. Side effects of radiation therapy include skin irritation, difficulty swallowing, dry mouth, nausea, diarrhea, hair loss and loss of energy (Curran, 1998; Brizel, 1998).

Chemotherapy, the treatment of cancer with anti-cancer drugs, is another mode of cancer therapy. The effectiveness of a given anti-cancer drug therapy often is limited by the difficulty of achieving drug delivery throughout solid tumors (el-Kareh and Secomb, 1997). Chemotherapeutic strategies are based on tumor tissue growth, wherein the anti-cancer drug is targeted to the rapidly dividing cancer cells. Most chemotherapy approaches include the combination of more than one anti-cancer drug, which has proven to increase the response rate of a wide variety of cancers (U.S. Patent 5,824,348; U.S. Patent 5,633,016 and U.S. Patent 5,798,339). A major side effect of chemotherapy drugs is that they also affect normal tissue cells, with the cells most likely to be affected being those that divide rapidly (e.g., bone marrow, gastrointestinal tract, reproductive system and hair follicles). Other toxic side effects of chemotherapy drugs are sores in the mouth, difficulty swallowing, dry mouth, nausea, diarrhea, vomiting, fatigue, bleeding, hair loss and infection.

Immunotherapy, a rapidly evolving area in cancer research, is yet another option for the treatment of certain types of cancers. For example, the immune system identifies tumor cells as being foreign and thus they are targeted for destruction by the immune system. Unfortunately, the response typically is not sufficient to prevent most tumor growths. However, recently there has been a focus in the area of immunotherapy to develop methods that augment or supplement the natural defense mechanism of the immune system. Examples of immunotherapies currently under investigation or in use are immune adjuvants *(e.g., Mycobacterium bovis, Plasmodium falciparum,* dinitrochlorobenzene and aromatic compounds) (U.S. Patent 5,801,005; U.S. Patent 5,739,169; Hui and Hashimoto, 1998; Christodoulides *et al.,* 1998), cytokine therapy (e.g., interferons α, β and γ; IL-1, GM-CSF and TNF) (Bukowski *et al.,* 1998; Davidson *et al.,* 1998; Hellstrand *et al.,* 1998) gene therapy (*e.g.,* TNF, IL-1, IL-2, p53) (Qin *et al.,* 1998; Austin-Ward and Villaseca, 1998; U.S. Patent 5,830,880 and U.S. Patent 5,846,945) and monoclonal antibodies (e.g., anti-ganglioside GM2, anti-HER-2, anti-p185) (Pietras *et al.,* 1998; Hanibuchi *et al.,* 1998; U.S. Patent 5,824,311).

As mentioned above, tumor suppressors play an important role in cancer biology. One of these, the p53 tumor suppressor proto-oncogene, is essential for the maintenance of the non-tumorogenic phenotype of cells (reviewed by Soddu and Sacchi, 1998). Approximately 50% of all cancers have been found to be associated with mutations of the p53 gene, which result in the loss of p53 tumor suppressor properties (Levine *et al.,* 1991; Vogelstein and Kinzler, 1992; Hartmann *et al.,* 1996a; Hartmann *et al.,* 1996b). Mutations in the p53 gene also result in the stabilization of the p53 protein in cells with concomitant overexpression of p53 protein. In normal cells, p53 protein is generally undetectable due to its high turnover rate. The high incidence of cancer related mutations in the p53 gene has prompted many research groups to investigate p53 as a route of cancer treatment via gene replacement. Ad-mda7 has been shown to suppress the growth of cancer cells that are p53 wildtype, p53 null and p53 mutant. Also, the upregulation of the apoptosis-related *bax* gene indicates that MDA-7 is capable of using p53 independent mechanisms to induce the destruction of cancer cells. These characteristics suggest that MDA-7 has broad therapeutic potential as an anti-proliferative agent.

### SUMMARY OF THE INVENTION

It is, therefore, an objective of the present invention to provide an alternative medicament for treating a patient with cancer. The object was solved by providing as a medicament a therapeutic nucleic acid, such as DNA, encoding either a full-length or truncated human MDA-7 protein or polypeptide under the control of a promoter operable in eukaryotic cells wherein the medicament is administered in combination with at least one other anticancer treatment, wherein the anticancer treatment is chemotherapy, wherein the chemotherapy comprises a DNA damaging agent, tamoxifen or Herceptin. The therapeutic nucleic acid is comprised in an expression cassette or construct, which is a nucleic acid molecule capable of allowing the expression of at least a portion of the nucleic acid sequence. In uses of the present invention, preferably the patient is a human. The sequence of a full-length MDA-7 polypeptide is provided in SEQ ID. NO:2. A truncated version of MDA-7 would comprise a portion or portions of contiguous amino acid regions of the full-length sequence as specified in the claims, but would not contain the entire sequence. The truncated version may be truncated by any number of contiguous amino acids at any site in the polypeptide.

The uses for treating a patient with cancer in the present invention comprise the transfer of a nucleic acid encoding either a full-length or truncated form of the human MDA-7 protein or polypeptide. Following the administration of the nucleic acid to a patient with cancer, the nucleic acid, under control of a promoter active in eukaryotic cells, is expressed by hyperproliferative cells thereby stimulating growth arrest or apoptosis of those cells. Alternatively, the nucleic acid encoding all or part of an MDA-7 protein may be expressed in normal, *i.e.*, non-hyperproliferative, cells and secreted to achieve bystander activity in which neighboring hyperproliferative cells are affected by MDA-7. Thus, it is contemplated that cells that are not hyperproliferative (non-hyperproliferative cells), such as normal or noncancerous cells *(i.e.,* cells that do not exhibit characteristics of unregulated cancerous cell growth), may express a population of MDA-7 protein, some of which is processed into a secretable form that is secreted and taken up by non-transduced (or non-transfected) cells that are nearby. Non-transduced or non-transfected cells are cells that have not internalized the exogenous expression cassette, and thereby, such cells do not express the polypeptide encoded by it. Non-transfected cells could include hyperproliferative or non-normal cells, which may uptake a secreted form of the MDA-7 polypeptide or protein such that the hyperproliferative or non-normal cells are induced to undergo apoptosis or growth inhibited. These non-normal cells may be tumor cells. It is envisioned that non-transduced or non-transfected cells affected by transduced or transfected cells would be proximate to, adjacent to (next to), or near each other. Essentially, the non-tranduced or non-transfected cell is close enough to the transduced or transfected cell so that the MDA-7 protein secreted by the transfected cell reaches the non-transfected cell.

In further embodiments, the cancer is melanoma, non-small cell lung, small-cell lung, lung, hepatocarcinoma, retinoblastoma, astrocytoma, glioblastoma, gum, tongue, leukemia, neuroblastoma, head, neck, breast, pancreatic, prostate, renal, bone, testicular, ovarian, mesothelioma, cervical, gastrointestinal, lymphoma, brain, colon, sarcoma or bladder. The cancer may include a tumor comprised of tumor cells. In other embodiments, the hyperproliferative disease is rheumatoid arthritis, inflammatory bowel disease, osteoarthritis, leiomyomas, adenomas, lipomas, hemangiomas, fibromas, vascular occlusion, restenosis, atherosclerosis, pre-neoplastic lesions (such as adenomatous hyperplasia and prostatic intraepithelial neoplasia), carcinoma *in situ,* oral hairy leukoplakia, or psoriasis.

In some embodiments, the nucleic acid molecule encodes amino acids from about 49 to about 206; about 75 to about 206; about 100 to about 206; about 125 to about 206; about 150 to about 206; about 175 to about 206; or about 182 to about 206 of SEQ ID NO:2. In some embodiments the expression cassette or vector encodes a truncated MDA-7 polypeptide as defined above. Thus, it is contemplated that in some embodiments, the nucleic acid sequence encoding a truncated MDA-7 polypeptide as defined above comprises fewer contiguous nucleotides than are in SEQ ID NO:1, *i.e.,* the nucleic acid segment is also less than full-length or truncated. For example, the expression vector or cassette may lack coding sequences corresponding to amino acid 1 to about amino acid 49, amino acid 1 to about amino acid 75, amino acid 1 to about amino acid 100, amino acid 1 to about amino acid 125, amino acid 1 to about amino acid 150, amino acid 1 to about amino acid 175, or amino acid 1 to about amino acid 182, of SEQ ID NO:2.

Nucleic acid molecules used in the present invention may contain sequences encoding a full-length, human mda-7 gene, as disclosed in SEQ ID NO:1. In some embodiments of the invention, a nucleic acid molecule may encode fewer nucleotides than is depicted in SEQ ID NO:1, such that the molecule contains fewer than 700 contigous nucleotides from SEQ ID NO:1 to encode the truncated MDA-7 polypeptide as defined above. In some aspects, a nucleic acid molecule may contain about 100, 200, 300, 400, 500, 600, or 700 contigous nucleotides from SEQ ID NO:1 to encode the truncated MDA-7 polypeptide as defined above. Alternatively the molecule may encode a nucleic acid molecule that encodes a MDA-7 polypeptide missing the first 49 amino acids of SEQ ID NO:2 because the nucleic acid sequence corresponding to the first 49 amino acids is absent.

In certain other embodiments, the nucleic acid further comprises nucleotides encoding a heterologous secretory signal sequence in which the heterologous sequence is derived from a non-MDA-7 nucleic acid sequence or polypeptide. A "signal sequence" refers to a sequence, typically short, that directs a newly translated secretory or membrane polypeptide to and through the endoplasmic reticulum or across a membrane. The signal sequence allows a protein to be secreted from a cell. In further embodiments, the nucleic acid further comprises a heterologous secretory signal sequence defined as a positively charged N-terminal region in combination with a hydrophobic core.

In certain embodiments, the promoter is CMV IE, dectin-1, dectin-2, human CD11c, F4/80, SM22, RSV, SV40, Ad MLP, beta-actin, MHC class I or MHC class II promoter, however any other promoter that is useful to drive expression of the mda-7 gene of the present invention, such as those set forth hereinbelow, is believed to be applicable to the practice of the present invention. In other embodiments, a polyadenylation signal is operatively linked to a MDA-7 coding region.

In certain embodiments, the nucleic acid is a viral vector, wherein the viral vector dose is from about 10³, 10⁴, 10⁵, 10⁶, 10⁷, 10⁸, 10⁹, 10¹⁰, 10¹¹, 10¹², 10¹³ pfu and higher. Alternatively, dosage may be expressed in units of viral particles (vp); thus, the numbers listed above in "pfu" units may be expressed in units of "vp" units or "viral particles." It is contemplated that about 10³ to about 10¹⁵, about 10⁵ to about 10¹², or 10⁷ to about 10¹⁰ viral particles may be administered to a patient.

In some embodiments, the viral vector is an adenoviral vector, a retroviral vector, a vaccinia viral vector, an adeno-associated viral vector, a polyoma viral vector, alpha viral vector or a herpesviral vector. In some aspects, the viral vector is an adenoviral vector. It is contemplated that the viral vector may be replication-deficient or defective. While in other embodiments, an adenovirus vector that contains Ad-5 sequences may be employed; in some aspects, an adenovirus vector construct lacks El-coding regions, which may comprise a deletion of both E1A and E1B sequences.

The uses of the present invention include dispersing expression constructs, vectors, and cassettes in pharmacologically acceptable solution for administration to a patient. In some cases, the pharmacologically acceptable solution comprises a lipid. In further embodiments of the present invention, a nucleic acid molecule encoding a full-length or truncated MDA-7 polypeptide is administered in a lipoplex *(i.e.,* as a lipid-nucleic acid complex), which may contain, as described in some embodiments, DOTAP and at least one cholesterol, cholesterol derivative, or cholesterol mixture. These nucleic acid molecules may be administered to the patient intravenously, intraperitoneally, intratracheally, intratumorally, intramuscularly, endoscopically, intralesionally, percutaneously, subcutaneously, regionally, or by direct injection or perfusion. It is further contemplated that treatment methods may involve multiple administrations.

The nucleic acid used in the present invention may be administered by injection. Other embodiments include the administering of the nucleic acid by multiple injections. In certain embodiments, the injection is performed local, regional or distal to a disease or tumor site. In some embodiments, the administering of nucleic acid is via continuous infusion, intratumoral injection, or intravenous injection. In certain other embodiments, the nucleic acid is administered to the tumor bed prior to or after; or both prior to and after resection of the tumor.

The present invention includes combination therapy methods using a nucleic acid sequence encoding a full-length or truncated MDA-7 polypeptide in combination with a second therapy to treat a cancer. The nucleic acid molecule may be administered to the patient before, during, or after chemotherapy.

While in further embodiments, chemotherapy involving at least one DNA damaging agent is implemented in combination with administration of an MDA-7 encoding nucleic acid molecule. The DNA damaging agent may be adriamycin, 5-fluorouracil (5FU), etoposide (VP-16), camptothecin, actinomycin-D, mitomycin C or cisplatin (CDDP). In further embodiments, the DNA damaging agent is adriamycin. In one aspect of the invention, the chemotherapy comprises tamoxifen, while in another aspect is it comprises adriamycin. Further embodiments involve immunotherapy with Herceptin.

In cases involving a cancerous tumor, a combination treatment may involve administration of a nucleic acid molecule encoding a full-length or truncated MDA-7 polypeptide and tumor resection, which may occur before, after, or during the mda-7 gene therapy administration. If mda-7 treatment occurs after tumor resection, the expression construct or vector encoding MDA-7 may be administered to the tumor bed.

Other uses of the invention include treating a patient with cancer in a process involving at least the following step: administering to the patient an adenovirus composition that contains an adenovirus construct with a human mda-7 gene under the control of a promoter and at least one other anticancer treatment as defined above in an amount effective to confer a therapeutic benefit on the patient. Another aspect includes uses for inducing apoptosis in a cancer cell by administering to a cancer cell in a subject an expression cassette containing a nucleic acid sequence encoding a human MDA-7 protein under the control of a promoter operable in eukaryotic cells and at least one other anticancer treatment as defined above. In yet further embodiments, the invention includes uses for inducing apoptosis in a cancer cell by administering to a noncancerous cell in a subject an expression cassette that contains a nucleic acid sequence encoding a human MDA-7 polypeptide under the control of a promoter operable in eukaryotic cells and at least one other anticancer treatment as defined above, wherein the MDA-7 polypeptide is expressed and secreted. With these types of bystander methods, a transfected noncancerous cell may be adjacent or close enough to a cancer cell such that the noncancerous cell secretes an MDA-7 polypeptide that induces growth arrest or apoptosis in the untransfected cancer cell. Other uses include those directed at treating a patient with cancer by administering to a noncancerous cell in the patient an effective amount of an adenovirus composition to confer a therapeutic benefit on that patient and at least one other anticancer treatment as defined above. This adenovirus composition can include an adenovirus vector construct that contains a mda-7 gene under the control of a promoter.

The uses of the present invention also encompass medicaments for treating a tumor by inducing apoptosis in transfected and untransfected tumor cells comprising administering to the tumor an adenovirus composition comprising an adenovirus vector construct comprising a human mda-7 gene under the control of a promoter and at least one other anticancer treatment as defined above, such that transfected cells express and secrete a truncated MDA-7 polypeptide. And still other embodiments include uses for treating cancer by administering to a subject with cancer an adenovirus composition that contains an adenovirus vector construct with a human mda-7 gene under the control of a promoter to a cell that does not have mutated p53, Rb, ras, or p16 genes and at least one other anticancer treatment as defined above, in an amount effective to induce apoptosis in a cell that does have a mutated p53, Rb, ras, or p16 gene.

Other uses of the invention include treating a subject with a tumor by administering to the subject a nucleic acid molecule comprising a human mda-7 gene under the control of a promoter and at least one other anticancer treatment as defined above in an amount effective to inhibit angiogenesis around the tumor. Such methods may also include steps to evaluate the level of angiogenesis inhibition. It is contemplated that other embodiments of treatment described herein may be implemented with these uses.

Compositions of the present invention include an expression vector encoding a mda-7 coding region under the control of a promoter operable in an eukaryotic cell, such that the coding region contains a deletion corresponding to N-terminal sequences. The expression vector compositions include any expression cassette described with respect to the methods of the present invention. Similarly, any compositions described herein may be utilized in the practice of any of the uses disclosed herein.

The use of the word "a" or "an" when used in conjunction with the term "comprising" in the claims and/or the specification may mean "one," but it is also consistent with the meaning of "one or more," "at least one," and "one or more than one."

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### BRIEF DESCRIPTION OF THE FIGURES

The following drawings form part of the present specification and are included to further demonstrate certain aspects of the present invention. The invention may be better understood by reference to one or more of these drawings in combination with the detailed description of specific embodiments presented herein.
**FIG. 1**. Schematic illustration of Ad-vectors. Replication-deficient human type 5 Adenovirus (Ad5) carrying the mda-7 (or luciferase gene) linked to an internal CMVIE promoter and followed by SV40 polyadenylation (pA) signal were used. In addition, Ad-CMVp(A) (empty vector) was used as control.
**FIG 2A****.** T47D cells treated with Ad-mda7 at varying MOIs (viral particle/cell). **FIG 2B****.** MCF-7 cells treated with Ad-mda7 at varying MOIs (Viral particle/cell).
**FIG. 3A****.** MDA-MB-361 cells treated with Ad-mda7 at varying MOIs (Viral particle/cell). **FIG. 3B****.** BT-20 cells treated with Ad-mda7 at varying MOIs (Viral particle/cell).
**FIG. 4A****.** H1299 cells treated with Ad-mda7 at varying MOIs (Viral particle/cell). **FIG. 4B****.** H322 cells treated with Ad-mda7 at varying MOIs (Viral particle/cell).
**FIG. 5A****.** SW620 cells treated with Ad-mda7 at varying MOIs (Viral particle/cell). **FIG. 5B****.** DLD-1 cells treated with Ad-mda7 at varying MOIs (Viral particle/cell).
**FIG. 6A****.** MJ90 cells treated with Ad-mda7 at varying MOIs (Viral particle/cell). **FIG. 6B****.** HUVEC cells treated with Ad-mda7 at varying MOIs (Viral particle/cell).
**FIG. 7**. Annexin V assay to determine apoptosis induction after Ad-mda7 transduction in breast cancer cell lines. Three breast cancer cell lines (T47D, MDA-MB-468, MCF-7) were infected with Ad-mda7 or control Ad-CMVp(A) empty vector, and evaluated for apoptosis using Annexin V.
**FIG. 8****.** DLD-1 cells were infected with Ad-mda7 or Ad-luc and 48 hours later examined for Annexin V staining by FACS analysis.
**FIG. 9****.** Panel A shows apoptosis induction in H1299 cells infected with Ad-mda7 or Ad-luc. Cells were evaluated at different time points post-infection using Annexin V staining and FACS analysis. Panel B illustrates apoptosis in DLD-1 cells that were infected with Ad-mda7 or Ad-luc at different time points post-infection (as examined by Annexin V staining and FACS analysis).
**FIG. 10**. H460 cells were infected with increasing MOIs of Ad-mda7 or Ad-luc and 48 h later processed for MDA7 surface expression and analyzed by FACS.

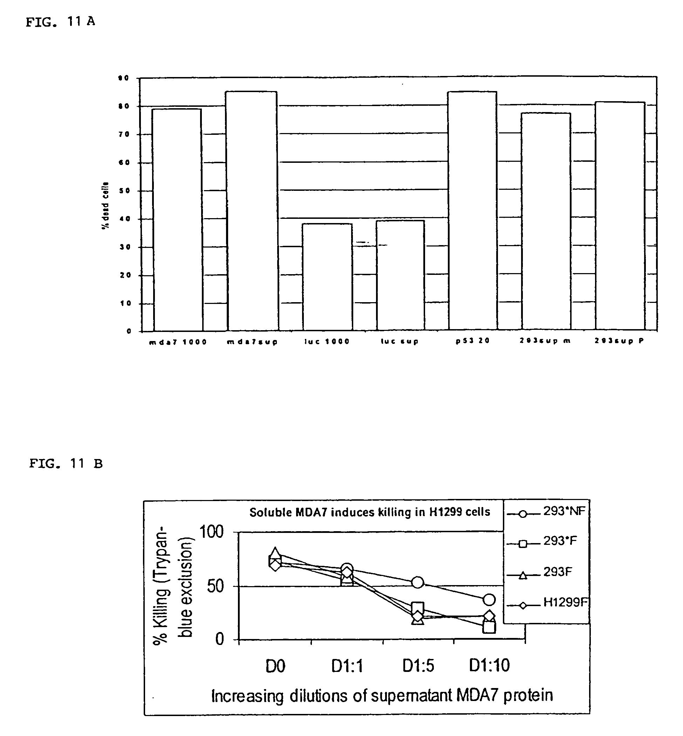

**FIG. 11A**. Soluble MDA-7 (sMDA7) kills tumor cells. H1299 cells were challenged with the following samples and percentage dead cells evaluated after 48 hours: 1) Ad-mda7 virus, positive control infected at 1000 Vp/cell; 2) Soluble MDA7 supernatant from H1299 infected cells with Ad-mda-7 (1000 vp/cell); 3) Ad-luc virus, control infected at 1000 Vp/ cell; 4) supernatant from H1299 infected cells with Ad-luc (1000 vp/cell); 5) Ad-p53 virus, positive control infected at 20 Vp/cell; 6) a separate stock of soluble MDA-7 supernatant obtained from 293 cells infected with Ad-mda-7 (sup M, 500 Vp/cell); and 7) a separate stock of soluble MDA-7 supernatant obtained from modified serum-free 293 cells infected with Ad-mda-7 (sup P, 500 Vp/cell). All the supernatants used in this experiment were filtered through a 0.1 micron filter prior to challenge with H1299 cells. **FIG. 11B****.** H1299 cells were challenged with soluble MDA-7 supernatant from four different stocks and percentage dead cells evaluated after 48 hours: 1) 293*NF: Non-filtered supernatant obtained from modified 293 cells (cells were grown in serum-free conditions); 2) 293*F: 0.1 micron filtered supernatant obtained from modified 293 cells; 3) 293F: 0.1 micron filtered supernatant obtained from regular 293 cells (FBS +); and 4) H1299F: 0.1 micron filtered supernatant obtained from H 1299 cells. D0 is non-diluted material whereas D1:1; D1:5, D1:10 indicate the dilutions used. Control undiluted supernatant from Ad-luc treated H1299 cells demonstrated 20% dead cells.
**FIG. 12****.** Combination with Tamoxifen. Ad-mda7 has been combined with tamoxifen and evaluated for anti-tumor effects in breast cancer cell lines. The graphs demonstrate that combining these two agents provides superior anti-tumor activity compared to either agent alone.
**FIG. 13****.** Combination with Adriamycin. Ad-mda7 has been combined with adriamycin and evaluated for anti-tumor effects in breast cancer cell lines. The graphs demonstrate that combining these two agents provides superior anti-tumor activity compared to either agent alone.
**FIG. 14****. Left Panel:** MDA-7 protein expression in NSCLC cells and normal lung cells after transduction with Ad-mda7. NHFB-normal human bronchial cells. **Right Panel-upper:** Effect of Ad-mda7 on growth of NSCLC cells and normal lung cells. Ad-mda7 (circles), PBS (diamonds), Ad-luc (squares). **Lower Panel:** Cell cycle analysis of NSCLC cells and normal lung cells after transduction with Ad-mda7. Note significant decrease in G 1 and increase in G2/M.
**FIG. 15****.** Combination of Ad-mda7 and Herceptin on breast cancer cell lines. Cell lines treated with Ad-mda7 are enhanced in a Her2-expressing cell line as compared to a non-expressing cell line, demonstrating the increased effectiveness of Herceptin on killing cells following contact with Ad-mda7.

### DESCRIPTION OF ILLUSTRATIVE EMBODIMENTS

The present invention contemplates the treatment of cancer by identifying patients with such a disease and expressing either a full-length or truncated form of human MDA-7 polypeptide in these hyperproliferative cells or normal cells neighboring hyperproliferative cells by means of nucleic acid transfer. The treatment of such a hyperproliferative disease in one embodiment involves the intratumoral administration of a full-length or truncated human MDA-7 expression construct to hyperproliferative cells. The hyperproliferative cells or normal cells then express a full-length or truncated form of human MDA-7, resulting in the growth inhibition or death of the hyperproliferative cells. Furthermore, neighboring hyperproliferative cells that have not taken up the MDA-7 expression construct may also be growth inhibited and/ or killed by the soluble form of MDA-7.

### A. HYPERPROLIFERATIVE DISEASE AND MDA-7

A variety of cancers can be treated according to the methods of the present invention. The present invention has important ramifications with respect to one hyperproliferative disease: cancer.

Cancer has become one of the leading causes of death in the western world, second only behind heart disease. Current estimates project that one person in three in the U.S. will develop cancer, and that one person in five will die from cancer. Cancers can be viewed as altered cells that have lost the normal growth-regulating mechanisms.

There currently are three major categories of oncogenes, reflecting their different activities. One category of oncogenes encodes proteins that induce cellular proliferation. A second category of oncogenes, called tumor-suppressors genes or anti-oncogenes, function to inhibit excessive cellular proliferation. The third category of oncogenes, either block or induce apoptosis by encoding proteins that regulate programmed cell death.

The cDNA encoding the MDA-7 protein has been described by Jiang et al., 1995 (WO 9511986). The protein encoded by the mda-7 cDNA was recognized as a potential regulator of melanoma progression. Jiang *et al.* used a subtractive hybridization technique (Jiang *et al.,* 1995) to identify genes involved in the regulation of growth and differentiation in human melanoma cells. A subtracted cDNA library prepared by subtraction hybridization of cDNAs prepared form actively proliferating human HO-1 melanoma cells against cDNAs prepared from interferon (IFN-β) and mezerin differentiated human HO-1 melanoma cells' was used to identify several melanoma differentiation associated (mda) cDNAs. The cDNA for MDA-7 was identified as having elevated expression levels in the differentiated melanoma cells.

That MDA-7 increased BAX levels in cancer cell lines led to an evaluation of the effect of *ex vivo* Ad-mda-7 transduction on xenograft tumorigenicity of MCF-7 tumor cells. *Ex vivo* transduction resulted in the inhibition of tumor formation and progression in the tumor xenograft model.

Treatment of cells with a mda-7 expression vector results in the secretion of a soluble form of the MDA-7 protein. This soluble protein possesses anti-tumor activity. Therefore, the combination of direct induction of apoptosis and release of soluble mediator with anti-tumor properties will provide enhanced activity against hyperproliferative diseases. The cancer cell-specific anti-proliferative effects of elevated MDA-7 expression make this molecule an ideal gene therapy treatment for hyperproliferative disease, especially cancer, when combined with at least one other anticancer treatment as defined above.

In some embodiments, the treatment of a wide variety of cancerous states or tissue/organ types is within the scope of the invention, for example, melanoma, non-small cell lung, small-cell lung, lung, hepatocarcinoma, retinoblastoma, astrocytoma, glioblastoma, leukemia, blood, brain, skin, eye, tongue, gum, neuroblastoma, head, neck, breast, pancreatic, prostate, renal, bone, testicular, ovarian, mesothelioma, cervical, gastrointestinal, lymphoma, brain, colon or bladder. In still more preferred embodiments the hyperproliferative disease being treated according to the present invention is rheumatoid arthritis, inflammatory bowel disease, osteoarthritis, leiomyomas, adenomas, lipomas, hemangiomas, fibromas, vascular occlusion, restenosis, atherosclerosis, pre-neoplastic lesions, carcinoma *in situ,* oral hairy leukoplakia or psoriasis.

The present invention is directed at the use of at least a part of an MDA-7 protein to treat patients with cancer such that these patients are conferred a therapeutic benefit as a result of the treatment. The term "therapeutic benefit" used throughout this application refers to anything that promotes or enhances the well-being of the patient with respect to the medical treatment of his cancer. A list of nonexhaustive examples of this includes extension of the patient's life by any period of time; decrease or delay in the neoplastic development of the disease; decrease in hyperproliferation; reduction in tumor growth; delay of metastases; reduction in the proliferation rate of a cancer cell or tumor cell; induction of apoptosis in any treated cell or in any cell affected by a treated cell; and a decrease in pain to the patient that can be attributed to the patient's condition.

### 1. MDA Protein, Polypeptides, and Peptides

The mda-7 cDNA encodes a novel, evolutionarily conserved protein of 206 amino acids with a predicted size of 23.8 kDa. The deduced amino acid sequence contains a hydrophobic stretch from about amino acid 26 to 45. The protein sequence shows no significant homology to known proteins or protein motifs with the exception of a 42 amino acid stretch that is 54% identical to interleukin 10 (IL-10). Structural analysis performed by Bazan *et al.* has determined that a shortened soluble form of MDA-7 (IL-BKW or IL-20) displays structural characteristics of the cytokine family (WO 9828425) and antagonizes IL-10 function. Bazan et al. note that the coding region of the mda-7 cDNA was mis-identified (p.6, 1.30). Furthermore, they assert that the "pre-sequence of IL-BKW/mda-7 probably starts at either the M(et) at position 28 or 30..(p.9, 1.3)" of the MDA-7 sequence. The structural characteristics and limited identity across a small stretch of amino acids implies a potential extracellular function for MDA-7. The inventors demonstrated that Ad-mda7, which encodes the full length 206 amino-acid sequence, gives rise to an intracellular protein of approximately 23 kD. Furthermore, the Ad-mda7 vector also causes release of a soluble form of MDA-7 protein from treated cells. The soluble MDA-7 protein is approximately 40kD and is glycosylated. Treatment with glycosidases reduces the molecular mass of the soluble protein. Inhibitors of protein secretion, brefeldin A and tunicamycin, cause an intracellular accumulation of MDA-7 and inhibit release of this protein from cells. The MDA-7 soluble protein causes growth inhibition of tumor cells. Therefore, release of this soluble MDA-7 can give rise to a "bystander" effect wherein tumor cells that are not contacted by a mda-7 expression construct will be growth inhibited.

Additional studies have shown that elevated expression of MDA-7 suppressed cancer cell growth and selectively induced apoptosis in human breast cancer cells as well as inhibiting tumorigenicity in nude mice (Jiang *et al.,* 1996 and Su *et al.,* 1998). Jiang *et al.* (1996) report findings that mda-7 is a potent growth suppressing gene in cancer cells of diverse origins including breast, central nervous system, cervix, colon, prostate, and connective tissue. A colony inhibition assay was used to demonstrate that elevated expression of mda-7 enhanced growth inhibition in human cervical carcinoma (HeLa), human breast carcinoma (MCF-7 and T47D), colon carcinoma (LS174T and SW480), nasopharyngeal carcinoma (HONE-1), prostate carcinoma (DU-145), melanoma (HO-1 and C8161), glioblastome multiforme (GBM-18 and T98G), and osteosarcoma (Saos-2). Mda-7 overexpressed in normal cells (HMECs, HBL-100, and CREF-Trans6) did not show significant effects.

Growth inhibition by elevated expression of MDA-7 is more effective in cancer cells than in normal cells. Su *et al.* (1998) investigated the mechanism by which MDA-7 suppressed cancer cell growth. The studies reported that ectopic expression of MDA-7 in breast cancer cell lines MCF-7 and T47D induced apoptosis as detected by cell cycle analysis and TUNEL assay without an effect on the normal HBL-100 cells. Western blotting of lysates from cells infected with adenovirus mda-7 showed an upregulation of the apoptosis stimulating protein BAX. Ad-mda-7 infection elevated levels of BAX protein only in MCF-7 and T47D cells and not normal HBL-100 or HMEC cells. These data led the investigators to evaluate the effect of *ex vivo* Ad-mda-7 transduction on xenograft tumorigenicity of MCF-7 tumor cells. *Ex vivo* transduction resulted in the inhibition of tumor formation and progression in the tumor xenograft model. MDA-7 has been shown to be effective in tumor cell-specific apoptotic induction. Thus, one embodiment of the present invention is the use of a mda-7 adenoviral construct encoding full-length or truncated MDA-7 for the manufacture of a medicament for the treatment of cancer in combination with at least one other anticancer treatment as defined above.

Of particular interest, according to the present invention, is the use of a soluble form of MDA-7. WO 98/28425 describes a cytokine molecule allegedly related to IL-10. This molecule, designated IL-BKW, appears to be derived from the same gene as MDA-7. However, the authors describe the coding region designation of MDA-7 as "mis-identified". The mature form of IL-BKW was to begin at about residue 47 or 49 of the mda-7 coding region, and continue some 158-160 residues, *i.e.,* to residues 206 of the mda-7 sequence. Thus, a preferred molecule would preferably lack all or part of both the putative signal sequence (residues 1-25) and a putative membrane spanning hydrophobic domain (residues 26-45) of full length MDA-7.

Other even shorter molecules are contemplated. For example, while molecules beginning approximately at MDA-7 residues 46-49 are the largest molecules, further N-terminal truncations are within the scope of the invention. Thus, specifically contemplated are molecules starting at about residue 49, 100, 150, 175 or 182, and terminating at about residue 206.

Though not adhering to a particular theory regarding the operability of these constructs, there is a notable homology between MDA-7 and IL-10, as well as across species in the C, E, and F helical regions and also in the amino acid positions that have been implicated in receptor binding. The tertiary structure of MDA-7 closely models the known structure of IL-10, further suggesting that these molecules are related. Thus, molecules containing any or all of these amino acid regions are particularly preferred.

Alternatively, in other embodiments a full-length or a substantially full-length MDA-7 polypeptide is contemplated to be of use in the treatment of cancer. When used in the context of human MDA-7, the term "full-length" refers to a MDA-7 polypeptide that contains at least the 206 amino acids encoded by the human mda-7 cDNA. The term "substantially full-length" in the context of human MDA-7 refers to a MDA-7 polypeptide that contains at least 80% of the contiguous amino acids of the full-length human MDA-7 polypeptide (SEQ ID N0:2). However, it is also contemplated that MDA-7 polypeptides containing at least about 85%, 90%, and 95% of SEQ ID NO:2 are within the scope of the invention as "substantially fun-length" MDA-7: A "truncated MDA-7 polypeptide" or "truncated MDA-7" refers to an MDA-7 polypeptide that is lacking contiguous amino acids from the full-length MDA-7 amino acid sequence as specified in the claims. The phrase "secreted MDA-7" refers to an MDA-7 polypeptide that is secreted from a cell, *i.e.,* a polypeptide that may or may not be encoded by a full-length mda-7 cDNA and whose N-terminus begins at about amino acid 46 of the full-length MDA-7 polypeptide. The phrases "truncated MDA-7" and "truncated MDA-7 polypeptide" include a secreted MDA-7 polypeptide if, for example, the signal sequence is not missing or if a heterologous signal sequence is attached to the truncated polypeptide.

The term "biologically functional equivalent" is well understood in the art and is further defined in detail herein. Accordingly, a sequence that has between about 70% and about 80%; or more preferably, between about 81 % and about 90%; or even more preferably, between about 91% and about 99%; of amino acids that are identical or functionally equivalent to the amino acids of SEQ ID NO:2 will be a sequence that is "essentially as set forth in SEQ ID NO:2," provided the biological activity of the protein, polypeptide, or peptide is maintained.

The term "functionally equivalent codon" is used herein to refer to codons that encode the same amino acid, such as the six codons for arginine and serine, and also refers to codons that encode biologically equivalent amino acids.

Excepting intronic and flanking regions, and allowing for the degeneracy of the genetic code, nucleic acid sequences that have between about 70% and about 79%; or more preferably, between about 80% and about 89%; or even more particularly, between about 90% and about 99%; of nucleotides that are identical to the nucleotides of SEQ ID NO: 1 will be nucleic acid sequences that are "essentially as set forth in SEQ ID NO:1."

It will also be understood that this invention is not limited to the particular nucleic acid and amino acid sequences of SEQ ID NO: 1 and SEQ ID NO:2, respectively. Recombinant vectors and isolated nucleic acid segments may therefore variously include these coding regions themselves, coding regions bearing selected alterations or modifications in the basic coding region, and they may encode larger polypeptides or peptides that nevertheless include such coding regions or may encode biologically functional equivalent proteins, polypeptide or peptides that have variant amino acids sequences.

### 2. mda-7 Nucleic Acids and Uses Thereof

One embodiment of the present invention is to transfer the nucleic acids encoding the full-length, substantially full-length, or truncated form of human MDA-7 to induce the destruction, apoptosis or lysis of hyperproliferative cells. The expression of MDA-7 is inversely correlated with tumor progression as demonstrated .. by increased mRNA levels in normal melanocytes as compared to primary and metastatic tumors as well as decreased MDA-7 expression in early vertical growth phase tumor cells selected for enhanced tumor formation in nude mice.

Thus, in some embodiments of the present invention, the treatment of involves the administration of a therapeutic nucleic acid expression construct encoding a full-length, substantially full-length, or truncated form of MDA-7 to hyperproliferative cells. It is contemplated that the hyperproliferative cells take up the construct and express the therapeutic polypeptide encoded by nucleic acid, thereby restoring a growth control to or destroying the hyperproliferative cells. Furthermore, the soluble MDA-7 released from transfected or transduced cells will be available locally and provide a bystander effect on neighboring tumor cells. Thus, it is contemplated that the therapeutic mda-7 expression construct may be delivered to normal cells and the released bystander effector (MDA-7, full-length or truncated) would cause anti-tumor effects, particularly with respect to hyperproliferative cells.

Certain aspects of the present invention concern at least one human mda-7 nucleic acid molecule. In certain aspects, the mda-7 nucleic acid comprises a wild-type or mutant mda-7 nucleic acid. In particular aspects, the mda-7 nucleic acid encodes for at least one transcribed nucleic acid. The mda-7 nucleic acid encodes at least one MDA-7 polypeptide. In other aspects, the human mda-7 nucleic acid comprises at least one nucleic acid segment of SEQ ID NO:1.

The present description also discloses the isolation or creation of at least one recombinant construct or at least one recombinant host cell through the application of recombinant nucleic acid technology known to those of skill in the art or as described herein. The recombinant construct or host cell may comprise at least one mda-7 nucleic acid, and may express at least one MDA-7 polypeptide.

As used herein "wild-type" refers to the naturally occurring sequence of a nucleic acid at a genetic locus in the genome of an organism, and sequences transcribed or translated from such a nucleic acid. Thus, the term "wild-type" also may refer to the amino acid sequence encoded by the nucleic acid. As a genetic locus may have more than one sequence or alleles in a population of individuals, the term "wild-type" encompasses all such naturally occurring alleles. As used herein the term "polymorphic" means that variation exists (*i.e.*, two or more alleles exist) at a genetic locus in the individuals of a population. As used herein, "mutant" refers to a change in the sequence of a nucleic acid or its encoded protein, polypeptide, or peptide that is the result of recombinant DNA technology.

A nucleic acid may be made by any technique known to one of ordinary skill in the art. Non-limiting examples of synthetic nucleic acid, particularly a synthetic oligonucleotide, include a nucleic acid made by *in vitro* chemical synthesis using phosphotriester, phosphite or phosphoramidite chemistry and solid phase techniques such as described in EP 266,032, or via deoxynucleoside H-phosphonate intermediates as described by Froehler *et al.,* 1986, and U.S. Patent Serial No. 5,705,629. A non-limiting example of enzymatically produced nucleic acid include one produced by enzymes in amplification reactions such as PCR^{™} (see for example, U.S. Patent 4,683,202 and U.S. Patent 4,682,195), or the synthesis of oligonucleotides described in U.S.. Patent No. 5,645,897. A non-limiting example of a biologically produced nucleic acid includes recombinant nucleic acid production in living cells, such as recombinant DNA vector production in bacteria (see for example, Sambrook *et al.* 1989).

A nucleic acid may be purified on polyacrylamide gels, cesium chloride centrifugation gradients, or by any other means known to one of ordinary skill in the art (see for example, Sambrook *et al.* 1989).

The term "nucleic acid" will generally refer to at least one molecule or strand of DNA, RNA or a derivative or mimic thereof, comprising at least one nucleobase, such as, for example, a naturally occurring purine or pyrimidine base found in DNA (*e.g.,* adenine "A," guanine "G," thymine "T," and cytosine "C") or RNA (*e.g.* A, G, uracil "U," and C). The term "nucleic acid" encompasses the terms "oligonucleotide" and "polynucleotide." The term "oligonucleotide" refers to at least one molecule of between about 3 and about 100 nucleobases in length. The term "polynucleotide" refers to at least one molecule of greater than about 100 nucleobases in length. These definitions generally refer to at least one single-stranded molecule, but in specific embodiments will also encompass at least one additional strand that is partially, substantially or fully complementary to the at least one single-stranded molecule. Thus, a nucleic acid may encompass at least one double-stranded molecule or at least one triple-stranded molecule that comprises one or more complementary strand(s) or "complement(s)" of a particular sequence comprising a strand of the molecule.

In certain embodiments, a "gene" refers to a nucleic acid that is transcribed. As used herein, a "gene segment" is a nucleic acid segment of a gene. In certain aspects, the gene includes regulatory sequences involved in transcription, or message production or composition. In particular embodiments, the gene comprises transcribed sequences that encode for a protein, polypeptide or peptide. In other particular aspects, the gene comprises a mda-7 nucleic acid, and/or encodes a MDA-7 polypeptide or peptide-coding sequences. In keeping with the terminology described herein, an "isolated gene" may comprise transcribed nucleic acid(s), regulatory sequences, coding sequences. or the like, isolated substantially away from other such sequences, such as other naturally occurring genes, regulatory sequences, polypeptide or peptide encoding sequences, etc. In this respect, the term "gene" is used for simplicity to refer to a nucleic acid comprising a nucleotide sequence that is transcribed, and the complement thereof. In particular aspects, the transcribed nucleotide sequence comprises at least one functional protein, polypeptide and/or peptide encoding unit. As will be understood by those in the art, this functional term "gene" includes both genomic sequences, RNA or cDNA sequences, or smaller engineered nucleic acid segments, including nucleic acid segments of a non-transcribed part of a gene, including but not limited to the non-transcribed promoter or enhancer regions of a gene. Smaller engineered gene nucleic acid segments may express, or may be adapted to express using nucleic acid manipulation technology, proteins, polypeptides, domains, peptides, fusion proteins, mutants and/or such like. Thus, a "truncated gene" refers to a nucleic acid sequence that is missing a stretch of contiguous nucleic acid residues that encode a portion of the full-length MDA-7 polypeptide. For example, a truncated gene may not contain the nucleic acid sequence for the N-terminal region of the MDA-7 polypeptide, such as the first 46 amino acids. It is envisioned that the nucleic acid sequences of the present invention may contain fewer than 95% of the contiguous nucleic acid residues of SEQ ID NO:1. Alternatively, these sequences may encode fewer than 90%, 85%, 80%, 75%, or 70% of the contiguous nucleic acid residues of SEQ ID NO:1.

"Isolated substantially away from other coding sequences" means that the gene of interest, in this case the mda-7 gene, forms the significant part of the coding region of the nucleic acid, or that the nucleic acid does not contain large portions of naturally-occurring coding nucleic acids, such as large chromosomal fragments, other functional genes, RNA or cDNA coding regions. Of course, this refers to the nucleic acid as originally isolated. and does not exclude genes or coding regions later added to the nucleic acid by recombinant nucleic acid technology.

In certain embodiments, the nucleic acid is a nucleic acid segment. As used herein, the term "nucleic acid segment," are smaller fragments of a nucleic acid, such as for non-limiting example, those that encode only part of the MDA-7 peptide or polypeptide sequence. Thus, a "nucleic acid segment may comprise any part of the mda-7 gene sequence, of from about 2 nucleotides to the full-length of the MDA-7 peptide- or polypeptide-encoding region. In certain embodiments, the "nucleic acid segment" encompasses the full-length mda-7 gene sequence. In particular embodiments, the nucleic acid comprises any part of SEQ ID NO:1 of from about 2 nucleotides to the full-length of the sequence encoding SEQ ID NO:2.

Various nucleic acid segments may be designed based on a particular nucleic acid sequence, and may be of any length. By assigning numeric values to a sequence, for example, the first residue is 1, the second residue is 2, *etc.,* an algorithm defining all nucleic acid segments can be created:
n to n + y
where n is an integer from 1 to the last number of the sequence and y is the length of the nucleic acid segment minus one, where n + y does not exceed the last number of the sequence. Thus, for a 10-mer, the nucleic acid segments correspond to bases 1 to 10, 2 to 11, 3 to 12 ... and/or so on. For a 15-mer, the nucleic acid segments correspond to bases 1 to 15, 2 to 16, 3 to 17 ... and/or so on. For a 20-mer, the nucleic segments correspond to bases 1 to 20, 2 to 21, 3 to 22 ... and/or so on. In certain embodiments, the nucleic acid segment may be a probe or primer.

The nucleic acid(s), regardless of the length of the sequence itself, may be combined with other nucleic acid sequences, including but not limited to, promoters, enhancers, polyadenylation signals, restriction enzyme sites, multiple cloning sites, coding segments, and the like, to create one or more nucleic acid construct(s). The overall length may vary considerably between nucleic acid constructs. Thus, a nucleic acid segment of almost any length may be employed, with the total length preferably being limited by the ease of preparation or use in the intended recombinant nucleic acid protocol.

In a non-limiting example, one or more nucleic acid constructs may be prepared that include a contiguous stretch of nucleotides identical to or complementary to SEQ ID NO:1 encoding a human MDA-7 polypeptide as specified in the claims. Such a stretch of nucleotides, or a nucleic acid construct, may be about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 210, about 220, about 230, about 240, about 250, about 260, about 270, about 280, about 290, about 300, about 310, about 320, about 330, about 340, about 350, about 360, about 370, about 380, about 390, about 400, about 410, about 420, about 430, about 440, about 450, about 460, about 470, about 480, about 490, about 500, about 510, about 520, about 530, about 540, about 550, about 560, about 570, about 580, about 590, about 600, about 610, about 618, about 650, about 700, about 750, about 1,000, about 2,000, about 3,000, about 5,000, about 10,000, about 15,000, about 20,000, about 30,000, about 50,000, about 100,000, about 250,000, about 500,000, about 750,000, to about 1,000,000 nucleotides in length, as well as constructs of greater size, up to and including chromosomal sizes (including all intermediate lengths and intermediate ranges), given the advent of nucleic acids constructs such as a yeast artificial chromosome are known to those of ordinary skill in the art. It will be readily understood that "intermediate lengths" and "intermediate ranges," as used herein, means any length or range including or between the quoted values (*i.e.,* all integers including and between such values). Non-limiting examples of intermediate lengths include about 101, about 102, about 103, etc.; about 151, about 152, about 153, etc.; about 1,001, about 1002, etc,; about 50,001, about 50,002, etc; about 750,001, about 750,002, etc.; about 1,000,001, about 1,000,002, etc. Non-limiting examples of intermediate ranges include about about 150 to about 500,001, about 3,032 to about 7,145, about 5,000 to about 15,000, about 20,007 to about 1,000,003, etc.

It is further understood that a nucleic acid sequence encoding all or a portion of an MDA-7 polypeptide may be comprised of contiguous complementary or identical nucleic acid sequences of any of the lengths described above and from SEQ ID NO:1.

It is contemplated that the nucleic acid constructs may encode a full-length MDA-7 or encode a truncated version of MDA-7 as specified in the claims, such that the transcript of the coding region represents the truncated version. The truncated transcript may then be translated into a truncated protein. Alternatively, a nucleic acid sequence may encode a full-length MDA-7 protein sequence, which is processed by the cellular machinery to produce a truncated MDA-7 as specified in the claims. Such nucleic acid molecules may contain contiguous nucleotides of the above-lengths from SEQ ID NO:1. Thus the sequences of the present invention may contain contiguous nucleic acids that are complementary or identical to SEQ ID NO: 1, yet be less than the entire sequence of SEQ ID NO:1. For example, the sequence may contain fewer than 718 contiguous nucleic acids from SEQ ID NO:1; it may instead contain, less than 700, 690, 680, 670, 660, 650, 640, 630, 620, 610, 600, 590, 580, 570, 560, 550, 540, 530, 520, 510, 500, 490, 480, 470, 460, 450, 440, 430, 420, 410, 400, 390, 380, 370, 360, 350, 340, 330, 320, 310, 300, 290, 280, 270, 260, 250, 240, 230, 220, 210, 200, or fewer contiguous nucleotides or nucleosides from SEQ ID NO:1.

The term "a sequence essentially as set forth in SEQ ID NO:1" or "a sequence essentially as set forth in SEQ ID NO:1" means that the sequence substantially corresponds to a portion of SEQ ID NO:1 and has relatively few amino acids that are not identical to, or biologically functionally equivalent to, the amino acids of SEQ ID NO:2.

### a. Vectors and Regulatory Signals

Vectors of the present invention are designed, primarily, to transform hyperproliferative cells with a therapeutic mda-7 gene under the control of regulated eukaryotic promoters (*i.e.,* constitutive, inducible, repressable, tissue-specific). Also, the vectors may contain a selectable marker if, for no other reason, to facilitate their manipulation *in vitro.* However, selectable markers may play an important role in producing recombinant cells.

Tables 1 and 2, below, list a variety of regulatory signals for use according to the present invention.

**Table 1 - Inducible Elements**

| Element | Inducer | References |
|---|---|---|
| MT II | Phorbol Ester (TFA) Heavy metals | Palmiter *et al.,* 1982; Haslinger and Karin, 1985; Searle *et al.,* 1985; Stuart *et al.,* 1985; Imagawa *et al.,* 1987; Karin ®, 1987; Angel *et al.,* 1987b; McNeall *et al.,* 1989 |
| MMTV (mouse mammary tumor virus) | Glucocorticoids | Huang *et al.,* 1981; Lee *et al.,* 1981; Majors and Varmus, 1983; Chandler *et al.,* 1983; Lee *et al.,* 1984; Fonta *et al.,* 1985; Sakai *et al.,* 1986 |
| β-interferon | poly(rI)X poly(rc) | Tavernier *et al.,* 1983 |
| Adenovirus 5 E2 | EIA | Imperiale and Nevins, 1984 |
| Collagenase | Phorbol Ester (TPA) | Angel *et al.,* 1987a |
| Stromelysin | Phorbol Ester (TPA) | Angel *et al.,* 1987b |
| SV40 | Phorbol Ester (TFA) | Angel *et al.,* 1987b |
| Murine MX Gene | Interferon, Newcastle Disease Virus | Hug et a., 1988 |
| GRP78 Gene | A23187 | Resendez *et al.,* 1988 |
| α-2-Macroglobulin | IL-6 | Kunz *et al.,* 1989 |
| Vimentin | Serum | Rittling *et al.,* 1989 |
| MHC Class I Gene H-2κb | Interferon | Blanar *et al.,* 1989 |
| HSP70 | EIA, SV40 Large T Antigen | Taylor *et al.,* 1989; Taylor and , Kingston, 1990a,b |
| Proliferin | Phorbol Ester-TPA | Mordacq and Linzer, 1989 |
| Tumor Necrosis Factor | PMA | Hensel *et al.,* 1989 |
| Thyroid Stimulating Hormone α Gene | Thyroid Hormone | Chatterjee *et al.,* 1989 |

**Table 2 - Other Promoter/Enhancer Elements**

| Promoter/Enhancer | References |
|---|---|
| Immunoglobulin Heavy Chain | Banerji *et al.,* 1983; Gilles *et al.,* 1983; Grosschedl and Baltimore, 1985; Atchinson and Perry, 1986, 1987; Imler *et al.,* 1987; Weinberger *et al.,* 1988; Kiledjian *et al.,* 1988; Porton *et al.,* 1990 |
| Immunoglobulin Light Chain | Queen and Baltimore, 1983; Picard and Schaffner, 1984 |
| T-Cell Receptor | Luria *et al.,* 1987, Winoto and Baltimore, 1989; Redondo *et al.,* 1990 |
| HLA DQ α and DQ β | Sullivan and Peterlin, 1987 |
| β-Interferon | Goodbourn *et al.,* 1986; Fujita *et al.,* 1987; Goodbourn and Maniatis, 1985 |
| Interleukin-2 | Greene *et al.,* 1989 |
| Interleukin-2 Receptor | Greene *et al.,* 1989; Lin *et al.,* 1990 |
| MHC Class II | Koch *et al.,* 1989 |
| MHC Class II HLA-DRα | Sherman *et al.,* 1989 |
| β-Actin | Kawamoto *et al.,* 1988; Ng *et al.,* 1989 |
| Muscle Creatine Kinase | Jaynes *et al.,* 1988; Horlick and Benfield, 1989; Johnson *et al.,* 1989a |
| Prealbumin (Transthyretin) | Costa *et al.,* 1988 |
| Elastase I | Omitz *et al.,* 1987 |
| Metallothionein | Karin *et al.,* 1987; Culotta and Hamer, 1989 |
| Collagenase | Pinkert *et al.,* 1987; Angel *et al.,* 1987 |
| Albumin Gene | Pinkert *et al.,* 1987, Tronche *et al.,* 1989, 1990 |
| α-Fetoprotein | Godbout *et al.,* 1988; Campere and Tilghman, 1989 |
| γ-Globin | Bodine and Ley, 1987; Perez-Stable and Constantini, 1990 |
| β-Globin | Trudel and Constantini, 1987 |
| c-fos | Cohen *et al.,* 1987 |
| c-HA-ras | Triesman, 1986; Deschamps *et al.,* 1985 |
| Insulin | Edlund *et al.,* 1985 |
| Neural Cell Adhesion Molecule (NCAM) | Hirsch *et al.,* 1990 |
| a₁-antitrypsin | Latimer *et al.,* 1990 |
| H2B (TH2B) Histone | Hwang *et al.,* 1990 |
| Mouse or Type I Collagen | Ripe *et al.,* 1989 |
| Glucose-Regulated Proteins (GRP94 and GRP78) | Chang *et al.,* 1989 |
| Rat Growth Hormone | Larsen *et al.,* 1986 |
| Human Serum Amyloid A (SAA) | Edbrooke *et al.,* 1989 |
| Troponin I (TN I) | Yutzey *et al.,* 1989 |
| Platelet-Derived Growth Factor | Pech *et al.,* 1989 |
| Duchenne Muscular Dystrophy | Klamut *et al.,* 1990 |
| SV40 | Banerji *et al.,* 1981; Moreau *et al.,* 1981; Sleigh and Lockett, 1985; Firak and Subramanian, 1986; Herr and Clarke. 1986; Imbra and Karin, 1986; Kadesch and Berg, 1986; Wang and Calame, 1986; Ondek *et al.,* 1987; Kuhl *et al.,* 1987 Schaffner *et al.,* 1988 |
| Polyoma | Swartzendruber and Lehman, 1975; Vasseur *et al.,* 1980: Katinka *et al.,* 1980, 1981; Tyndell *et al.,* 1981: Dandolo *et al.,* 1983; de Villiers *et al.,* 1984; Hen *et al.,* 1986; Satake *et al.,* 1988; Campbell and Villarreal, 1988 |
| Retroviruses | Kriegler and Botchan, 1982, 1983; Levinson *et al.,* 1982; Kriegler *et al.,* 1983, 1984a,b, 1988; Bosze *et al.,* 1986; Miksicek *et al.,* 1986; Celander and Haseltine, 1987; Thiesen *et al.,* 1988; Celander *et al.,* 1988; Chol *et al.,* 1988; Reisman and Rotter, 1989 |
| Papilloma Virus | Campo *et al.,* 1983; Lusky *et al.,* 1983; Spandidos and Wilkie, 1983; Spalholz *et al.,* 1985; Lusky and Botchan, 1986; Cripe *et al.,* 1987; Gloss *et al.,* 1987; Hirochika *et al.,* 1987, Stephens and Hentschel, 1987; Glue *et al.,* 1988 |
| Hepatitis B Virus | Bulla and Siddiqui, 1986; Jameel and Siddiqui, 1986; Shaul and Ben-Levy, 1987; Spandau and Lee, 1988 |
| Human Immunodeficiency Virus | Muesing *et al.,* 1987; Hauber and Cullan, 1988; Jakobovits *et al.,* 1988; Feng and Holland, 1988; Takebe *et al.,* 1988; Rowen *et al.,* 1988; Berkhout *et al.,* 1989; Laspia *et al.,* 1989; Sharp and Marciniak, 1989; Braddock *et al.,* 1989 |
| Cytomegalovirus | Weber *et al.,* 1984; Boshart *et al.,* 1985; Foecking and Hofstetter, 1986 |
| Gibbon Ape Leukemia Virus | Holbrook *et al.,* 1987; Quinn *et al.,* 1989 |

The promoters and enhancers that control the transcription of protein encoding genes in eukaryotic cells are composed of multiple genetic elements. The cellular machinery is able to gather and integrate the regulatory information conveyed by each element, allowing different genes to evolve distinct, often complex patterns of transcriptional regulation. A promoter used in the context of the present invention includes constitutive, inducible, and tissue-specific promoters.

The term "promoter" will be used here to refer to a group of transcriptional control modules that are clustered around the initiation site for RNA polymerase II. Much of the thinking about how promoters are organized derives from analyses of several viral promoters, including those for the HSV thymidine kinase (tk) and SV40 early transcription units. These studies, augmented by more recent work, have shown that promoters are composed of discrete functional modules, each consisting of approximately 7-20 bp of DNA, and containing one or more recognition sites for transcriptional activator proteins.

At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between elements is flexible, so that promoter function is preserved when elements are inverted or moved relative to one another. In the tk promoter, the spacing between elements can be increased to 50 bp apart before activity begins to decline. Depending on the promoter, it appears that individual elements can function either co-operatively or independently to activate transcription.

Enhancers were originally detected as genetic elements that increased transcription from a promoter located at a distant position on the same molecule of DNA. This ability to act over a large distance had little precedent in classic studies of prokaryotic transcriptional regulation. Subsequent work showed that regions of DNA with enhancer activity are organized much like promoters. That is, they are composed of many individual elements, each of which binds to one or more transcriptional proteins. of many individual elements, each of which binds to one or more transcriptional proteins.

The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Aside from this operational distinction, enhancers and promoters are very similar entities.

Promoters and enhancers have the same general function of activating transcription in the cell. They are often overlapping and contiguous, often seeming to have a very similar modular organization. Taken together, these considerations suggest that enhancers and promoters are homologous entities and that the transcriptional activator proteins bound to these sequences may interact with the cellular transcriptional machinery in fundamentally the same way.

Preferred for use in the present invention is the cytomegalovirus (CMV) promoter. This promoter is commercially available from Invitrogen in the vector pcDNAIII, which is preferred for use in the present invention. Also contemplated as useful in the present invention are the dectin-l and dectin-2 promoters. Additional viral promoters, cellular promoters/enhancers and inducible promoters/enhancers that could be used in combination with the present invention are listed in Tables 1 and 2. Additionally any promoter/enhancer combination (as per the Eukaryotic Promoter Data Base EPDB) could also be used to drive expression of structural genes encoding oligosaccharide processing enzymes, protein folding accessory proteins, selectable marker proteins or a heterologous protein of interest. Alternatively, a tissue-specific promoter for cancer gene therapy (Table 3) or the targeting of tumors (Table 4) may be employed with the nucleic acid molecules of the present invention.

**Table 3: Candidate tissue-specific promoters for cancer gene therapy**

| **Tissue-specific promoter** | **Cancers in which promoter is active** | **Normal cells in which promoter is active** |
|---|---|---|
| Carcinoembryonic antigen | Most colorectal carcinomas; 50% of | Colonic mucosa; gastric |
| mucosa; | | |
| (CEA)* | lung carcinomas; 40-50% of gastric | lung epithelia; eccrine sweat |
| | carcinomas; most pancreatic | glands; cells in testes |
| | carcinomas; many breast | |
| | carcinomas | |
| Prostate-specific antigen | Most prostate carcinomas | Prostate epithelium |
| (PSA) .. | | |
| Vasoactive intestinal peptide | Majority of non-small cell lung | Neurons; lymphocytes; mast |
| (VIP) | cancers | cells; eosinophils |
| Surfactant protein A (SP-A) | Many lung adenocarcinomas | Type II pneumocytes; Clara |
| cells | | |
| Human achaete-scute | Most small cell lung cancers | Neuroendocrine cells in lung |
| homolog (hASH) | | |
| Mucin-1 (MUC1)** | Most adenocarcinomas (originating | GFandular epithelial cells in |
| breast | | |
| | from any tissue) | and in respiratory, |
| | | gastrointestinal, and |
| | | genitourinary tracts |
| Alpha-fetoprotein | Most hepatocellular carcinomas; | Hepatocytes (under certain |
| | possibly many testicular cancers | conditions); testis |
| Albumin | Most hepatocellular carcinomas | Hepatocytes |
| Tyrosinase | Most melanomas | Melanocytes; astrocytes; |
| | | Schwann cells; some |
| neurons | | |
| Tyrosine-binding protein | Most melanomas | Melanocytes: astmcytes, |
| (TRP) | | Schwann cells; some |
| neurons | | |
| Keratin 14 | Prosumably many squamous cei¹ | Keratinocytes |
| | carcinomas (eg: Head and neck | |
| | cancers) | |
| EBV LD-2 | Many squamous cell carcinomas of | Keratinocytes of upper |
| digestive | | |
| | head and neck | tract |
| Glial fibrillary acidic protein | Many astrocytomas | Astrocytes |
| (GFAP) | | |
| Myelin basic protein (MBP) | Many gliomas | Oligodendrocytes |
| Testis-specific angiotensin- Possibly many testicular cancers | | Spermatazoa |
| converting enzyme (Testis- | | |
| specific ACE) | | |
| Osteocalcin | Possibly many osteosarcomas | Osteoblasts |

**Table 4: Candidate promoters for use with a tissue-specific targeting of tumors**

| **Promoter** | **Cancers in which** | **Normal cells in** |
|---|---|---|
| **which** | | |
| | **Promoter is active** | **Promoter is active** |
| E2F-regulated promoter | Almost all cancers | Proliferating cells |
| HLA-G | Many colorectal carcinomas; many | Lymphocytes; |
| monocytes; | | |
| | melanomas; possibly many other | spermatocytes; |
| trophoblast | | |
| | cancers | |
| FasL | Most melanomas; many pancreatic | Activated leukocytes: |
| neurons: | | |
| | carcinomas; most astrocytomas; | endothelial cells; |
| keratinocytes; | | |
| | possibly many other cancers | cells in |
| immunoprivileged | | |
| | | tissues; some cells in |
| lungs. | | |
| | | ovaries, liver, and |
| prostate | | |
| Myc-regulated promoter | Most lung carcinomas (both small | Proliferating cells (only |
| some | | |
| | cell and non-small cell); most | cell-types): mammary |
| epithelial | | |
| | colorectal carcinomas | cells (including non- |
| proliferating) | | |
| MAGE-1 | Many melanomas; some non-small | Testis |
| | cell lung carcinomas; some breast | |
| | carcinomas | |
| VEGF | 70% of all cancers (constitutive | Cells at sites of |
| | overexpression in many cancers) | neovascularization (but |
| unlike in | | |
| | | tumors, expression is |
| transient, | | |
| | | less strong, and never |
| | | constitutive) |
| bFGF | Presumably many different | cells at sites of ischemia |
| (but | | |
| | cancers, since bFGF expression is | unlike tumors, |
| expression is | | |
| | induced by ischemic conditions | transient, less strong, and |
| never | | |
| | | constitutive) |
| COX-2 | Most colorectal carcinomas; many | Cells at sites of |
| inflammation | | |
| | lung carcinomas; possibly many | |
| | other cancers | |
| IL-10 | Most colorectal carcinomas; many | Leukocytes |
| | lung carcinomas; many squamous | |
| | cell carcinomas of head and neck; | |
| | possibly many other cancers | |
| GRP78/BiP | Presumably many different | Cells at sites of ischemia |
| | cancers, since GRP7S expression | |
| | is induced by tumor-specific | |
| | conditions | |
| CArG elements from Egr-1 | Induced by ionization radiation, so | Cells exposed to ionizing |
| | conceivably most tumors upon | radiation: leukocytes |
| | irradiation | |

Another signal that may prove useful is a polyadenylation signal (hGH, BGH, SV40). The use of internal ribosome binding sites (IRES) elements are used to create multigene, or polycistronic, messages. IRES elements are able to bypass the ribosome scanning model of 5'-methylated cap-dependent translation and begin translation at internal sites (Pelletier and Sonenberg, 1988). IRES elements from two members of the picornavirus family (polio and encephalomyocarditis) have been described (Pelletier and Sonenberg, 1988), as well as an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message.

In any event, it will be understood that promoters are DNA elements which when positioned functionally upstream of a gene leads to the expression of that gene. Most transgene constructs of the present invention are functionally positioned downstream of a promoter element.

### b. Gene Transfer

### i. Viral Transformation

### a) Adenoviral Infection

One method for delivery of the recombinant DNA involves the use of an adenovirus expression vector. Although adenovirus vectors are known to have a low capacity for integration into genomic DNA, this feature is counterbalanced by the high efficiency of gene transfer afforded by these vectors. "Adenovirus expression vector" is meant to include those constructs containing adenovirus sequences sufficient to (a) support packaging of the construct and (b) to ultimately express a recombinant gene construct that has been cloned therein.

The vector comprises a genetically engineered form of adenovirus. Knowledge of the genetic organization or adenovirus, a 36 kb, linear, double-stranded DNA virus, allows substitution of large pieces of adenoviral DNA with foreign sequences up to 7 kb (Grunhaus and Horwitz, 1992). In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification.

Adenovirus is particularly suitable for use as a gene transfer vector because of its mid-sized genome, ease of manipulation, high titer, wide target-cell range and high infectivity. Both ends of the viral genome contain 100-200 base pair inverted repeats (ITRs), which are *cis* elements necessary for viral DNA replication and packaging. The early (E) and late (L) regions of the genome contain different transcription units that are divided by the onset of viral DNA replication. The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and host cell shut-off (Renan, 1990). The products of the late genes, including the majority of the viral capsid proteins, are expressed only after significant processing of a single primary transcript issued by the major late promoter (MLP). The MLP, (located at 16.8 m.u.) is particularly efficient during the late phase of infection, and all the mRNA's issued from this promoter possess a 5'-tripartite leader (TPL) sequence which makes them preferred mRNA's for translation.

In a current system, recombinant adenovirus is generated from homologous recombination between shuttle vector and provirus vector. Due to the possible recombination between two proviral vectors, wild-type adenovirus may be generated from this process. Therefore, it is critical to isolate a single clone of virus from an individual plaque and examine its genomic structure.

Generation and propagation of the current adenovirus vectors, which are replication deficient, depend on a unique helper cell line, designated 293, which was transformed from human embryonic kidney cells by Ad5 DNA fragments and constitutively expresses E1 proteins (Graham *et al.,* 1977). Since the E3 region is dispensable from the adenovirus genome (Jones and Shenk, 1978), the current adenovirus vectors, with the help of 293 cells, carry foreign DNA in either the E1, the E3, or both regions (Graham and Prevec, 1991). In nature, adenovirus can package approximately 105% of the wild-type genome (Ghosh-Choudhury *et al.,* 1987), providing capacity for about 2 extra kb of DNA. Combined with the approximately 5.5 kb of DNA that is replaceable in the E1 and E3 regions, the maximum capacity of the current adenovirus vector is under 7.5 kb, or about 15% of the total length of the vector. More than 80% of the adenovirus viral genome remains in the vector backbone.

Helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, e.g., Vero cells or other monkey embryonic mesenchymal or epithelial cells. As stated above, the preferred helper cell line is 293.

Racher *et al.* (1995) have disclosed improved methods for culturing 293 cells and propagating adenovirus. In one format, natural cell aggregates are grown by inoculating individual cells into 1 liter siliconized spinner flasks (Techne, Cambridge, UK) containing 100-200 ml of medium. Following stirring at 40 rpm, the cell viability is estimated with trypan blue. In another format, Fibra-Cel microcarriers (Bibby Sterlin, Stone, UK) (5 g/l) is employed as follows. A cell inoculum, resuspended in 5 ml of medium, is added to the carrier (50 ml) in a 250 ml Erlenmeyer flask and left stationary, with occasional agitation, for I to 4 h. The medium is then replaced with 50 ml of fresh medium and shaking initiated. For virus production, cells are allowed to grow to about 80% confluence, after which time the medium is replaced (to 25% of the final volume) and adenovirus added at an MOI of 0.05. Cultures are left stationary overnight, following which the volume is increased to 100% and shaking commenced for another 72 h.

The adenovirus vector may be replication defective, or at least conditionally defective, the nature of the adenovirus vector is not believed to be crucial to the successful practice of the invention. The adenovirus may be of any of the 42 different known serotypes or subgroups A-F. Adenovirus type 5 of subgroup C is the preferred starting material in order to obtain the conditional replication-defective adenovirus vector for use in the present invention. This is because Adenovirus type 5 is a human adenovirus about which a great deal of biochemical and genetic information is known, and it has historically been used for most constructions employing adenovirus as a vector.

As stated above, the typical vector according to the present invention is replication defective and will not have an adenovirus E 1 region. Thus, it will be most convenient to introduce the transforming construct at the position from which the E1-coding sequences have been removed. However, the position of insertion of the construct within the adenovirus sequences is not critical to the invention. The polynucleotide encoding the gene of interest may also be inserted in lieu of the deleted E3 region in E3 replacement vectors as described by Karlsson *et al.* (1986) or in the E4 region where a helper cell line or helper virus complements the E4 defect.

Adenovirus growth and manipulation is known to those of skill in the art, and exhibits broad host range *in vitro* and *in vivo.* This group of viruses can be obtained in high titers, e.g., 10⁹-10¹¹ plaque-forming units per ml, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus (Couch *et al.,* 1963; Top *et al.,* 1971), demonstrating their safety and therapeutic potential as *in vivo* gene transfer vectors.

Adenovirus vectors have been used in eukaryotic gene expression (Levrero *et al.,* 1991; Gomez-Foix *et al.,* 1992) and vaccine development (Grunhaus and Horwitz, 1992; Graham and Prevec, 1992). Animal studies have suggested that recombinant adenovirus could be used for gene therapy (Stratford-Perricaudet and Perricaudet, 1991; Stratford-Perricaudet *et al.,* 1990; Rich *et al.,* 1993). Studies in administering recombinant adenovirus to different tissues include trachea instillation (Rosenfeld *et al.,* 1991; Rosenfeld *et al.,* 1992), muscle injection (Ragot *et al.,* 1993), peripheral intravenous injections (Herz and Gerard, 1993) and stereotactic inoculation into the brain (Le Gal La Salle *et al.,* 1993).

### b) Retroviral Infection

The retroviruses are a group of single-stranded RNA viruses characterized by an ability to convert their RNA to double-stranded DNA in infected cells by a process of reverse-transcription (Coffin, 1990). The resulting DNA then stably integrates into cellular chromosomes as a provirus and directs synthesis of viral proteins. The integration results in the retention of the viral gene sequences in the recipient cell and its descendants. The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome (Coffin, 1990).

In order to construct a retroviral vector, a nucleic acid encoding a gene of interest is inserted into the viral genome in the place of certain viral sequences to produce a virus that is replication-defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

Concern with the use of defective retrovirus vectors is the potential appearance of wild-type replication-competent virus in the packaging cells. This can result from recombination events in which the intact sequence from the recombinant virus inserts upstream from the gag, pol, env sequence integrated in the host cell genome. However, packaging cell lines are available that should greatly decrease the likelihood of recombination (Markowitz *et al.,* 1988; Hersdorffer *et al.,* 1990).

### c) AAV Infection

Adeno-associated virus (AAV) is an attractive vector system for use in the present invention as it - has a high frequency of integration and it can infect nondividing cells, thus making it useful for delivery of genes into mammalian cells in tissue culture (Muzyczka, 1992). AAV has a broad host range for infectivity (Tratschin, *et al.,* 1984; Laughlin, *et al.,* 1986; Lebkowski, *et al.,* 1988; McLaughlin, *et al.,* 1988), which means it is applicable for use with the present invention. Details concerning the generation and use of rAAV vectors are described in U.S. Patent No. 5,139,941 and U.S. Patent No. 4,797,368.

Studies demonstrating the use of AAV in gene delivery include LaFace *et al.* (1988); Zhou *et al.* (1993); Flotte *et al.* (1993); and Walsh *et al.* (1994). Recombinant AAV vectors have been used successfully for *in vitro* and *in vivo* transduction of marker genes (Kaplitt *et al.,* 1994; Lebkowski *et al.,* 1988; Samulski *et al.,* 1989; Shelling and Smith, 1994; Yoder *et al.,* 1994; Zhou *et al.,* 1994; Hermonat and Muzyczka, 1984; Tratschin *et al.,* 1985; McLaughlin *et al.,* 1988) and genes involved in human diseases (Flotte *et al.,* 1992; Luo *et al.,* 1994; Ohi *et al.,* 1990; Walsh *et al.,* 1994; Wei *et al.,* 1994). Recently, an AAV vector has been approved for phase I human trials for the treatment of cystic fibrosis.

AAV is a dependent parvovirus in that it requires coinfection with another virus (either adenovirus or a member of the herpes virus family) to undergo a productive infection in cultured cells (Muzyczka, 1992). In the absence of coinfection with helper virus, the wild-type AAV genome integrates through its ends into human chromosome 19 where it resides in a latent state as a provirus (Kotin *et al.,* 1990; Samulski *et al.,* 1991). - rAAV, however, is not restricted to chromosome 19 for integration unless the AAV Rep protein is also expressed (Shelling and Smith, 1994). When a cell carrying an AAV provirus is superinfected with a helper virus, the AAV genome is "rescued" from the chromosome or from a recombinant plasmid, and a normal productive infection is established (Samulski *et al.,* 1989; McLaughlin *et al.,* ' 1988; Kotin *et al.,* 1990; Muzyczka, 1992).

Typically, recombinant AAV (rAAV) virus is made by cotransfecting a plasmid containing the gene of interest flanked by the two AAV terminal repeats (McLaughlin *et al.,* 1988; Samulski *et al.,* 1989; each incorporated herein by reference) and an expression plasmid containing the wild-type AAV coding sequences without the terminal repeats, for example pIM45 (McCarty *et al.,* 1991). The cells are also infected or transfected with adenovirus or plasmids carrying the adenovirus genes required for AAV helper function. rAAV virus stocks made in such fashion are contaminated with adenovirus which must be physically separated from the rAAV particles (for example, by cesium chloride density centrifugation). Alternatively, adenovirus vectors containing the AAV coding regions or cell lines containing the AAV coding regions and some or all of the adenovirus helper genes could be used (Yang *et al.,* 1994; Clark *et al.,* 1995). Cell lines carrying the rAAV DNA as an integrated provirus can also be used (Flotte *et al.,* 1995).

### d) Other Viral Vectors

Other viral vectors may be employed as constructs in the present invention. Vectors derived from viruses such as vaccinia virus (Ridgeway, 1988; Baichwal and Sugden, 1986; Coupar *et al.,* 1988) and herpesviruses may be employed. They offer several attractive features for various mammalian cells (Friedmann, 1989; Ridgeway, 1988; Baichwal and Sugden, 1986; Coupar *et al.,* 1988; Horwich *et al.,* 1990).

A molecularly cloned strain of Venezuelan equine encephalitis (VEE) virus has been genetically refined as a replication competent vaccine vector for the expression of heterologous viral proteins (Davis *et al.,* 1996). Studies have demonstrated that VEE infection stimulates potent CTL responses and has been sugested that VEE may be an extremely useful vector for immunizations (Caley *et al.,* 1997). It is contemplated in the present invention, that VEE virus may be useful in targeting dendritic cells.

With the recent recognition of defective hepatitis B viruses, new insight was gained into the structure-function relationship of different viral sequences. *In vitro* studies showed that the virus could retain the ability for helper-dependent packaging and reverse transcription despite the deletion of up to 80% of its genome (Horwich *et al.,* 1990). This suggested that large portions of the genome could be replaced with foreign genetic material. Chang *et al.* recently introduced the chloramphenicol acetyltransferase (CAT) gene into duck hepatitis B virus genome in the place of the polymerase, surface, and pre-surface coding sequences. It was cotransfected with wild-type virus into an avian hepatoma cell line. Culture media containing high titers of the recombinant virus were used to infect primary duckling hepatocytes. Stable CAT gene expression was detected for at least 24 days after transfection (Chang *et al.,* 1991).

In still further embodiments of the present invention, the nucleic acid encoding human mda-7 is housed within an infective virus that has been engineered to express a specific binding ligand. The virus particle will thus bind specifically to the cognate receptors of the target cell and deliver the contents to the cell. A novel approach designed to allow specific targeting of retrovirus vectors was recently developed based on the chemical modification of a retrovirus by the chemical addition of lactose residues to the viral envelope. This modification can permit the specific infection of hepatocytes via sialoglycoprotein receptors.

For example, targeting of recombinant retroviruses was designed in which biotinylated antibodies against a retroviral envelope protein and against a specific cell receptor were used. The antibodies were coupled via the biotin components by using streptavidin (Roux *et al.,* 1989). Using antibodies against major histocompatibility complex class I and class II antigens, they demonstrated the infection of a variety of human cells that bore those surface antigens with an ecotropic virus *in vitro* (Roux *et al.,* 1989).

### ii. Non-Viral Delivery

In addition to viral delivery of the nucleic acid encoding full length or truncated MDA-7 protein, the following are additional methods of recombinant gene delivery to a given host cell and are thus considered in the present invention.

### a) Electroporation

In certain preferred embodiments of the present invention, the gene construct is introduced into target hyperproliferative cells via electroporation. Electroporation involves the exposure of cells (or tissues) and DNA (or a DNA complex) to a high-voltage electric discharge.

Transfection of eukaryotic cells using electroporation has been quite successful. Mouse pre-B lymphocytes have been transfected with human kappa-immunoglobulin genes (Potter *et al.,* 1984), and rat hepatocytes have been transfected with the chloramphenicol acetyltransferase gene (Tur-Kaspa *et al.,* 1986) in this manner.

It is contemplated that electroporation conditions for hyperproliferative cells from different sources may be optimized. One may particularly wish to optimize such parameters as the voltage, the capacitance, the time and the electroporation media composition. The execution of other routine adjustments will be known to those of skill in the art. *See e.g.,* Hoffman, 1999; *Heller et al.,* 1996.

### b) Particle Bombardment

Another embodiment of the invention for transferring a naked DNA construct into cells involves particle bombardment. This method depends on the ability to accelerate DNA-coated microprojectiles to a high velocity allowing them to pierce cell membranes and enter cells without killing them (Klein *et al.,* 1987). The microprojectiles used have consisted of biologically inert substances such as tungsten, platinum, or gold beads.

It is contemplated that in some instances DNA precipitation onto metal particles would not be necessary for DNA delivery to a recipient cell using particle bombardment. It is contemplated that particles may contain DNA rather than be coated with DNA. Hence it is proposed that DNA-coated particles may increase the level of DNA delivery via particle bombardment but are not, in and of themselves, necessary.

Several devices for accelerating small particles have been developed. One such device relies on a high voltage discharge to generate an electrical current, which in turn provides the motive force (Yang *et al.,* 1990). Another method involves the use of a Biolistic Particle Delivery System, which can be used to propel particles coated with DNA through a screen, such as stainless steel or Nytex screen, onto a filter surface covered with cells in suspension. The screen disperses the particles so that they are not delivered to the recipient cells in large aggregates. It is believed that a screen intervening between the projectile apparatus and the cells to be bombarded reduces the size of projectile aggregates and may contribute to a higher frequency of transformation by reducing the damage inflicted on the recipient cells by projectiles that are too large.

For the bombardment, cells in suspension are preferably concentrated on filters, or alternatively on solid culture medium. The cells to be bombarded are positioned at an appropriate distance below the macroprojectile stopping plate. If desired, one or more screens are also positioned between the acceleration device and the cells to be bombarded.

In bombardment transformation, one may optimize the prebombardment culturing conditions and the bombardment parameters to yield the maximum numbers of stable transformants. Both the physical and biological parameters for bombardment are important in this technology. Physical factors are those that involve manipulating the DNA/microprojectile precipitate or those that affect the flight and velocity or either the macro- or microprojectiles. Biological factors include all steps involved in manipulation of cells before and immediately after bombardment, the osmotic adjustment of target cells to help alleviate the trauma associated with bombardment, and also the nature of the transforming DNA, such as linearized DNA or intact supercoiled plasmids. Recently, results from a clinical trial evaluating utility of this delivery system for vaccination was published. The study was designed to determine the safety and immunogenicity in volunteers of a DNA vaccine consisting of a plasmid encoding hepatitis B surface antigen delivered by the PowderJect XR1 gene delivery system into human skin *(Tacket et al.,* 1999).

Accordingly, it is contemplated that one may wish to adjust various bombardment parameters in small scale studies to fully optimize the conditions. One may particularly wish to adjust physical parameters such as gap distance, flight distance, tissue distance and helium pressure. One also may optimize the trauma reduction factors by modifying conditions which influence the physiological state of the recipient cells and which may therefore influence transformation and integration efficiencies. For example, the osmotic state, tissue hydration and the subculture stage or cell cycle of the recipient cells may be adjusted for optimum transformation. The execution of other routine adjustments will be known to those of skill in the art.

### c) Calcium Phosphate Co-Precipitation or DEAE-Dextran Treatment

In other embodiments of the present invention, the transgenic construct is introduced to the cells using calcium phosphate co-precipitation. Mouse primordial germ cells have been transfected with the SV40 large T antigen, with excellent results (Watanabe *et al.,* 1997). Human KB cells have been transfected with adenovirus 5 DNA (Graham and Van Der Eb, 1973) using this technique. Also in this manner, mouse L(A9), mouse C127, CHO, CV-1, BHK, NIH3T3 and HeLa cells were transfected with a neomycin marker gene (Chen and Okayama, 1987), and rat hepatocytes were transfected with a variety of marker genes (Rippe *et al.,* 1990).

In another embodiment, the expression construct is delivered into the cell using DEAE-dextran followed by polyethylene glycol. In this manner, reporter plasmids were introduced into mouse myeloma and erythroleukemia cells (Gopal, 1985).

### d) Direct Microinjection or Sonication Loading

Further embodiments of the present invention include the introduction of the nucleic acid construct by direct microinjection or sonication loading. Direct microinjection has been used to introduce nucleic acid constructs into *Xenopus* oocytes (Harland and Weintraub, 1985), and LTK- fibroblasts have been transfected with the thymidine kinase gene by sonication loading (Fechheimer *et al.,* 1987).

### e) Lipid-Mediated Transformation

In a further embodiment of the invention, the gene construct may be entrapped in a liposome or lipid formulation. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated is a gene construct complexed with Lipofectamine (Gibco BRL).

Lipid-mediated nucleic acid delivery and expression of foreign DNA *in vitro* has been very successful (Nicolau and Sene, 1982; Fraley *et al.,* 1979; Nicolau *et al.,* 1987). Wong *et al.* (1980) demonstrated the feasibility of lipid-mediated delivery and expression of foreign DNA in cultured chick embryo, HeLa and hepatoma cells.

Lipid based non-viral formulations provide an alternative to adenoviral gene therapies. Although many cell culture studies have documented lipid based non-viral gene transfer, systemic gene delivery via lipid based formulations has been limited. A major limitation of non-viral lipid based gene delivery is the toxicity of the cationic lipids that comprise the non-viral delivery vehicle. The *in vivo* toxicity of liposomes partially explains the .discrepancy between *in vitro* and *in vivo* gene transfer results. Another factor contributing to this contradictory data is the difference in lipid vehicle stability in the presence and absence of serum proteins. The interaction between lipid vehicles and serum proteins has a dramatic impact on the stability characteristics of lipid vehicles (Yang and Huang, 1997). Cationic lipids attract and bind negatively charged serum proteins. Lipid vehicles associated with serum proteins are either dissolved or taken up by macrophages leading to their removal from circulation. Current *in vivo* lipid delivery methods use subcutaneous, intradermal, intratumoral, or intracranial injection to avoid the toxicity and stability problems associated with cationic lipids in the circulation. The interaction of lipid vehicles and plasma proteins is responsible for the disparity between the efficiency of *in vitro* (Felgner *et al.,* 1987) and *in vivo* gene transfer (Zhu *el al.,* 1993; Philip *et al.,* 1993; Solodin *et al.,* 1995; Liu *et al.,* 1995; Thierry *et al.,* 1995; Tsukamoto *et al.,* 1995; Aksentijevich *et al.,* 1996).

Recent advances in lipid formulations have improved the efficiency of gene transfer *in vivo* (Templeton *et al.* 1997; WO 98/07408). A novel lipid formulation composed of an equimolar ratio of 1,2-bis(oleoyloxy)-3-(trimethyl ammonio)propane (DOTAP) and cholesterol significantly enhances systemic *in vivo* gene transfer, approximately 150 fold. The DOTAP:cholesterol lipid formulation forms unique structure termed a "sandwich liposome". This formulation is reported to "sandwich" DNA between an invaginated bi-layer or 'vase' structure. Beneficial characteristics of these lipid structures include a positive ρ, colloidal stabilization by cholesterol, two dimensional DNA packing and increased serum stability. Patent Application Nos. 60/135,818 and 60/133,116 discuss formulations that may be used with the present invention.

The production of lipid formulations often is accomplished by sonication or serial extrusion of liposomal mixtures after (I) reverse phase evaporation (II) dehydration-rehydration (III) detergent dialysis and (IV) thin film hydration. Once manufactured, lipid structures can be used to encapsulate compounds that are toxic (chemotherapeutics) or labile (nucleic acids) when in circulation. Lipid encapsulation has resulted in a lower toxicity and a longer serum half-life for such compounds (Gabizon *et al.,* 1990). Numerous disease treatments are using lipid based gene transfer strategies to enhance conventional or establish novel therapies, in particular therapies for treating hyperproliferative diseases.

In certain embodiments of the invention, the lipid vehicle may be complexed with a hemagglutinating virus (HVJ). This has been shown to facilitate fusion with the cell membrane and promote cell entry of lipid-encapsulated DNA (Kaneda *et al.,* 1989). In other embodiments, the lipid vehicle may be complexed or employed in conjunction with nuclear non-histone chromosomal proteins (HMG-1) (Kato *et al.,* 1991). In yet further embodiments, the lipid vehicle may be complexed or employed in conjunction with both HVJ and HMG-1.

A contemplated method for commercial scale preparation of a lipid composition is, generally speaking, mixing of the components, drying the mixture, rehydrating the mixture, dispersing the mixture, extruding the lipid composition through filters of decreasing pore size and mixing of the lipid composition with a therapeutic agent to form a lipoplex. The following provides additional information about manufacturing and formulating a lipoplex for use in the delivery of an mda-7 polynucleotide to a cell.

Powdered components are weighed, mixed, and dissolved in an acceptable solvent, such as tertiary butanol, chloroform, methanol, ethylene chloride, ethanol, or mixtures of these solvents. It is contemplated that any two of the solvent are present in a ration of about 1:1000, 1:500,1:100, 1:50, 1:25, 1:10, 1:5 or 1:1. Solublization with tertiary butanol may be employed, due to the carcinogenic properties of chloroform. Although, chloroform can be used if steps are taken to limit the residual chloroform present in the lipid composition to acceptable levels. It is envisioned that other lipophilic solvents may be used for the solubilization of the lipid components. The lipid mixture may be solubilized in *tert*-butanol at a temperature of about 0°C, 2°C, 4°C, 6°C, 8°C, 10°C, 12°C, 14°C, 16°C, 18°C, 20°C, 22°C, 24°C, 26°C, 28°C, 30°C, 32°C, 34°C, 36°C, 38°C, 40°C, 42°C, 44°C, 46°C, 48°C, 50°C, 52°C, 54°C, 56°C, 58°C, 60°C, 62°C, 64°C, 66°C, 68°C, 70°C, 72°C, 74°C, 76°C, 78°C, 80°C, 82°C, 84°C, 86°C, 88°C, or 90°C. A range of temperatures, such as 5°C to 80°C, 10°C to 70°C, 20°C to 60°C, and 30°C to 50°C, is also contemplated for use with the present invention.

Following preparation of the lipid composition a lipid complex is formulated by combining the lipid composition and a therapeutic agent in a pharmaceutically acceptable carrier solution by rapid mixing. Rapid mixing can be done by using a T-mixing apparatus or ethanol injection of the therapeutic agent. In certain embodiments a DOTAP:Cholesterol lipid composition is prepared by the methods described and combined with a polynucleotide that encodes for a therapeutic protein. The lipid composition is provided in an amount to encapsulate the polynucleotide and result in a colloidal suspension of the lipoplex.

DOTAP (cationic lipid) may be mixed with cholesterol at equimolar concentrations. This mixture of powdered lipids is then dissolved with tert-butanol, the solution dried to a thin film and the film hydrated in water containing 5% dextrose (w/v) to give a final concentration of about 20 mM DOTAP and about 20 mM cholesterol. The hydrated lipid film is rotated in a heated water bath for about 45 minutes, then at about 35°C for an additional 10 minutes and left standing at room temperature overnight. The following day the mixture is sonicated for 5 minutes at about 50°C. The sonicated mixture is heated for 10 minutes at about 50°C. This mixture is sequentially extruded through filters of decreasing pore size (1 µm, 0.45 µm, 0.2 µm, 0.1 µm). This lipid composition is then mixed with DNA to produce a lipid complex.

A lipid composition may comprise DOTAP and cholesterol, a cholesterol derivative or a cholesterol mixture and a polynucleotide encoding a therapeutic protein, such as MDA-7, antisense RNA or ribozyme for delivery into disease cells or into cells near the disease cells. The treatment of a disease, in one embodiment, involves the intravenous administration of a polynucleotide lipoplex to a patient, which subsequently express a therapeutic encoded by the polynucleotide. The lipoplex treatment of the patient delivers the polynucloetide to normal and hyperproliferative cells that express the therapeutic, resulting in the inhibition or destruction of the hyperproliferative cells.

The initial lipid mixture will preferably be of powdered lipid components that can be weighed and mixed to appropriate molar ratios. The components can be anionic lipids, cationic lipids, neutral lipids, sterols, and/or other hydrophobic molecules in ratios necessary to produce the desired characteristics of the final lipid composition or complex. The actual composition of the lipid mixture will be determined by the properties required for efficient delivery of the agent(s) to the desired target cells by the desired means of administration, described in detail below. Components of the composition can be mixed to provide various molar ratios. The molar concentrations of any component of the lipid composition can be from about 0.5mM, 1mM, 2mM, 3mM, 4mM, 5mM, 6mM, 7mM, 8mM, 9mM, 10mM, 15mM, 20mM, 25mM, 30mM, 35mM, 40mM, 45mM, and 50mM. The molar ratio of any two of the components can be from about 1:100. 1:50, 1:25, 1:20:1:18, 1:16, 1:15, 1:14, 1:13, 1:12, 1:11, 1:10, 1:9, 1:8, 1:7, 1:6, 1:5, 1:4, 1:3, 1:2, 1:1, 1:0.5, 1:0.25, 1:0.1, 1:0.05, or 1:0.01.

The lipid compositions are capable of carrying biologically active agents. The lipid composition can sequester toxic compounds to reduce free concentrations in the serum, protect compounds from degradative agents in the body, and/or mask antigenic components to reduce the immunogenicity of the agent. For example, DOTP:Cholesterol lipid compositions complex with polynucleotides such that the polynucleotides are sequestered and resistant to degradation in the serum. The lipid composition may be complexed with a variety of therapeutic agents, including but not limited to polynucleotides, proteins, peptides, chemotherapeutics, small molecule, peptonucleotides, and carbohydrate therapeutics.

A preferred lipid composition is DOTAP and cholesterol or a cholesterol derivative. The ratio of DOTAP to cholesterol, cholesterol derivative or cholesterol mixture may about 4:1 to 1:4, 3 :1 to 1:3, more preferably 2:1 to 1:2, or 1:1. The DOTAP or cholesterol concentrations may be between about 1 to 8 mM, 2 to 7 mM, 3 to 6 mM, or 4 to 5 mM. Cholesterol derivatives may be readily substituted for the cholesterol or mixed with the cholesterol in the present invention. A number of cholesterol derivatives are known to the skilled artisan. Cholesterol acetate and cholesterol oleate may be used. Polynucleotides are preferably added to the liposomes at a concentration of 20 to 200 µg per 200 µL final volume.

The lipoplex is prepared by diluting a given polynucleotide and lipid composition in 5% dextrose in water to obtain an appropriate concentration of nucleic acid and lipids. Equal volumes of nucleic acid and lipids, at a concentration to obtain 100 µg of nucleic acid / 5mM lipids / 100 µl, is mixed by adding the nucleic acid rapidly to the surface of the lipid composition by rapid mixing or with an impinging jet.

### 3. Pharmaceutical Formulations and Delivery

According to the present invention the use of an expression construct encoding either a full length or truncated human mda-7 protein as specified in the claims for the manufacture of a medicament for the treatment of cancer is contemplated wherein the medicament is administered in combination with at least one other anticancer treatment as defined above. Cancers that are most likely to be treated in the present invention are those that result from mutations in an oncogene and/ or the reduced expression of a wild-type protein in the hyperproliferative cells. Examples of cancers contemplated for treatment include lung cancer, head and neck cancer, breast cancer, pancreatic cancer, prostate cancer, renal cancer, bone cancer, testicular cancer, cervical cancer, gastrointestinal cancer, lymphomas, pre-neoplastic lesions in the lung, colon cancer, ' melanoma, bladder cancer and any other cancers that may be treated by administering a nucleic acid encoding either a full length or truncated human mda-7 protein as specified in the claims.

An effective amount of the pharmaceutical composition, generally, is defined as that amount sufficient to detectably and repeatedly to ameliorate, reduce, minimize or limit the extent of the disease or its symptoms. More rigorous definitions may apply, including- elimination, eradication or cure of disease.

Preferably, patients will have adequate bone marrow function (defined as a peripheral absolute granulocyte count of > 2,000 / mm³ and a platelet count of 100,000 / mm³), adequate liver function (bilirubin < 1.5 mg / dl) and adequate renal function (creatinine < 1.5 mg / dl).

### a. Administration

To kill cells, inhibit cell growth, inhibit metastasis, decrease tumor or tissue size and otherwise reverse or reduce the malignant phenotype of tumor cells, using the present invention, one would generally contact a hyperproliferative cell with the therapeutic expression construct. The routes of administration will vary; naturally, with the location and nature of the lesion, and include, e.g., intradermal, transdermal, parenteral, intravenous, intramuscular, intranasal, subcutaneous, percutaneous, intratracheal, intraperitoneal, intratumoral, perfusion, lavage, direct injection, and oral administration and formulation.

Intratumoral injection, or injection into the tumor vasculature is specifically contemplated for discrete, solid, accessible tumors. Local, regional or systemic administration also may be appropriate. For tumors of >4 cm, the volume to be administered will be about 4-10 ml (preferably 10 ml), while for tumors of <4 cm, a volume of about 1-3 ml will be used (preferably 3 ml). Multiple injections delivered as single dose comprise about 0.1 to about 0.5 ml volumes. The viral particles may advantageously be contacted by administering multiple injections to the tumor, spaced at approximately 1 cm intervals.

In the case of surgical intervention, the present invention may be used preoperatively, to render an inoperable tumor subject to resection. Alternatively, the present invention may be used at the time of surgery, and/or thereafter, to treat residual or metastatic disease. For example, a resected tumor bed may be injected or perfused with a formulation comprising mda-7 or an mda-7-encoding construct. The perfusion may be continued post-resection, for example, by leaving a catheter implanted at the site of the surgery. Periodic post-surgical treatment also is envisioned.

Continuous administration also may be applied where appropriate, for example, where a tumor is excised and the tumor bed is treated to eliminate residual, microscopic disease. Delivery *via* syringe or catherization is preferred. Such continuous perfusion may take place for a period from about 1-2 hours, to about 2-6 hours, to about 6-12 hours, to about 12-24 hours, to about 1-2 days, to about 1-2 wk or longer following the initiation of treatment. Generally, the dose of the therapeutic composition via continuous perfusion will be equivalent to that given by a single or multiple injections, adjusted over a period of time during which the perfusion occurs. It is further contemplated that limb perfusion may be used to administer therapeutic compositions of the present invention, particularly in the treatment of melanomas and sarcomas.

Treatment regimens may vary as well, and often depend on tumor type, tumor location, disease progression, and health and age of the patient. Obviously, certain types of tumor will require more aggressive treatment, while at the same time, certain patients cannot tolerate more taxing protocols. The clinician will be best suited to make such decisions based on the known efficacy and toxicity (if any) of the therapeutic formulations.

In certain embodiments, the tumor being treated may not, at least initially, be resectable. Treatments with therapeutic viral constructs may increase the resectability of the tumor due to shrinkage at the margins or by elimination of certain particularly invasive portions. Following treatments, resection may be possible. Additional treatments subsequent to resection will serve to eliminate microscopic residual disease at the tumor site.

A typical course of treatment, for a primary tumor or a post-excision tumor bed, will involve multiple doses. Typical primary tumor treatment involves a 6 dose application over a two-week period. The two-week regimen may be repeated one, two, three, four, five, six or more times. During a course of treatment, the need to complete the planned dosings may be re-evaluated.

The treatments may include various "unit doses." Unit dose is defined as containing a predetermined-quantity of the therapeutic composition. The quantity to be administered, and the particular route and formulation, are within the skill of those in the clinical arts. A unit dose need not be administered as a single injection but may comprise continuous infusion over a set period of time. Unit dose of the present invention may conveniently be described in terms of plaque forming units (pfu) for a viral construct. Unit doses range from 10³, 10⁴, 10⁵, 10⁶, 10⁷, 10⁸, 10⁹, 10¹⁰, 10¹¹, 10¹², 10¹³ pfu and higher. Alternatively, depending on the kind of virus and the titer attainable, one will deliver 1 to 100, 10 to 50, 100-1000, or up to about 1 x 10⁴, 1 x 10⁵, 1 x 10⁶, 1 x 10⁷, 1 x 10⁸ , 1 x 10⁹, 1 x 10¹⁰, 1 x 10¹¹, 1 x 10¹², 1 x 10¹³, 1 x 10¹⁴, or 1 x 10¹⁵ or higher infectious viral particles (vp) to the patient or to the patient's cells.

### b. Injectable Compositions and Formulations

The preferred method for the delivery of an expression construct encoding either a full length or truncated human MDA-7 protein to hyperproliferative cells in the present invention is via intratumoral injection. However, the pharmaceutical compositions disclosed herein may alternatively be administered parenterally, intravenously, intradermally, intramuscularly, transdermally or even intraperitoneally as described in U.S. Patent 5,543,158; U.S. Patent 5,641,515 and U.S. Patent 5,399,363.

Injection of nucleic acid constructs may be delivered by syringe or any other method used for injection of a solution, as long as the expression construct can pass through the particular gauge of needle required for injection. A novel needleless injection system has recently been described (U.S. Patent 5,846,233) having a nozzle defining an ampule chamber for holding the solution and an energy device for pushing the solution out of the nozzle to the site of delivery. A syringe system has also been described for use in gene therapy that permits multiple injections of predetermined quantities of a solution precisely at any depth (U.S. Patent 5,846,225).

Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions (U.S. Patent 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous, intratumoral and intraperitoneal administration. In this connection, sterile aqueous media that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vaccuum-drying and freeze-drying techniques which yield a powder of the active ingredient plus any additional desired ingredient from a previously sterile-filtered solution thereof.

The compositions disclosed herein may be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug release capsules and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" or "pharmacologically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared.

### 4. Combination Treatments

In order to increase the effectiveness of a full-length, substantially full-length, or truncated mda-7 polypeptide, or expression construct coding therefor, it combined with other agents effective in the treatment of cancer, namely an anticancer treatment as defined above. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. Anti-cancer agents are chemotherapy agents, namely a DNA damaging agent, tamoxifen or Herceptin. More generally, these other compositions would be provided in a combined amount effective to kill or inhibit proliferation of the cell. This process may involve contacting the cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

Tumor cell resistance to chemotherapy and radiotherapy agents represents a major problem in clinical oncology. One goal of current cancer research is to find ways to improve the efficacy of chemo- and radiotherapy by combining it with gene therapy. For example, the herpes simplex-thymidine kinase (HS-tK) gene, when delivered to brain tumors by a retroviral vector system, successfully induced susceptibility to the antiviral agent ganciclovir (Culver, *et al.,* 1992). In the context of the present invention, mda-7 gene therapy used similarly in conjunction with chemotherapeutic intervention as defined above, in addition to other pro-apoptotic or cell cycle regulating agents.

Alternatively, the gene therapy may precede or follow the other agent treatment by intervals ranging from minutes to weeks. In embodiments where the other agent and expression construct are applied separately to the cell, one would generally ensure that a significant period of time did not expire between the time of each delivery, such that the agent and expression construct would still be able to exert an advantageously combined effect on the cell. In such instances, it is contemplated that one may contact the cell with both modalities within about 12-24 h of each other and, more preferably, within about 6-12 h of each other. In some situations, it may be desirable to extend the time period for treatment significantly, however, where several d (2, 3, 4, 5, 6 or 7) to several wk (1, 2, 3, 4, 5, 6, 7 or 8) lapse between the respective administrations.

Various combinations may be employed, gene therapy is "A" and the secondary agent, i.e. chemotherapy, is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of the therapeutic expression constructs of the present invention to a patient will follow general protocols for the administration of chemotherapeutics, taking into account the toxicity, if any, of the vector. It is expected that the treatment cycles would be repeated as necessary. It also is contemplated that various standard therapies, as well as surgical intervention, may be applied in combination with the described hyperproliferative cell therapy.

### a. Chemotherapy

Cancer therapies in general also include a variety of combination therapies with both chemical and radiation based treatments. Combination chemotherapies include, for example, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, famesyl-protein transferase inhibitors, transplatinum, 5-fluorouracil, vincristine, vinblastine and methotrexate, Temazolomide (an aqueous form of DTIC), or any analog or derivative variant of the foregoing. The combination of chemotherapy with biological therapy is known as biochemotherapy.

### b. Radiotherapy

Other factors that cause DNA damage and have been used extensively include what are commonly known as y-rays, X-rays, and/or the directed delivery of radioisotopes to tumor cells. Other forms of DNA damaging factors are also known such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### c. Immunotherapy

Immunotherapeutics, generally, rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionuclide, ricin A chain, cholera toxin, pertussis toxin, etc.) and serve merely as a targeting agent. Alternatively, the effector may be a lymphocyte carrying a surface molecule that interacts, either directly or indirectly, with a tumor cell target. Various effector cells include cytotoxic T cells and NK cells. Mda-7 gene transfer to tumor cells causes tumor cell death and apoptosis. The apoptotic tumor cells are scavenged by reticuloendothelial cells including dendritic cells and macrophages and presented to the immune system to generate anti-tumor immunity (Rovere *et al.,* 1999; Steinman *et al.,* 1999). The soluble form of MDA-7 protein has cytokine-like structure and activities, such as activation of immune cells. The combination of therapeutic modalities, i.e., direct cytotoxic activity and immune activation by MDA-7 would provide therapeutic benefit in the treatment of cancer.

The general approach for combined therapy is discussed below. In one aspect of immunotherapy, the tumor cell must bear some marker that is amenable to targeting, *i.e.,* is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, *erb* B and p155. An alternative aspect of immunotherapy is to combine pro-apoptotic effect, mediated by Ad-mda7 treatment with immune stimulatory effects. The latter may be inherent in the soluble MDA-7 protein. However, alternate immune stimulating molecules also exist including: cytokines such as IL-2, LL-4, IL-12, GM-CSF, gamma-IFN, chemokines such as MIP-1, MCP-1, IL-8 and growth factors such as FLT3 ligand. Combining immune stimulating molecules, either as proteins or using gene delivery in combination with Ad-mda7 will enhance anti-tumor effects (Ju *et al.,* 2000).

As discussed earlier, examples of immunotherapies currently under investigation or in use are immune adjuvants (*e.g., Mycobacterium bovis, Plasmodium falciparum,* dinitrochlorobenzene and aromatic compounds) (U.S. Patent 5,801,005; U.S. Patent 5,739,169; Hui and Hashimoto, 1998; Christodoulides *et al.,* 1998), cytokine therapy (e.g., interferons α, β and γ; IL-1, GM-CSF and TNF) (Bukowski *et al.,* 1998; Davidson *et al.,* 1998; Hellstrand *et al.,* 1998) gene therapy (e.g., TNF, IL-1, IL-2, p53) (Qin *et al*., 1998; Austin-Ward and Villaseca, 1998; U.S. Patent 5,830,880 and U.S. Patent 5,846,945) and monoclonal antibodies (e.g., anti-ganglioside GM2, anti-HER-2, anti-p185) (Pietras *et al.,* 1998; Hanibuchi *et al.,* 1998; U.S. Patent 5,824,311). Herceptin (trastuzumab) is a chimeric (mouse-human) monoclonal antibody that blocks the HER2-neu receptor. It possesses anti-tumor activity and has been approved for use in the treatment of malignant tumors (Dillman, 1999). Combination therapy of cancer with herceptin and chemotherapy has been shown to be more effective than the individual therapies. Thus, it is contemplated that one or more anti-cancer therapies may be employed with the Ad-mda7 therapy described herein.

### i. Passive Immunotherapy

A number of different approaches for passive immunotherapy of cancer exist. They may be broadly categorized into the following: injection of antibodies alone; injection of antibodies coupled to toxins or chemotherapeutic agents; injection of antibodies coupled to radioactive isotopes; injection of anti-idiotype antibodies; and finally, purging of tumor cells in bone marrow.

Preferably, human monoclonal antibodies are employed in passive immunotherapy, as they produce few or no side effects in the patient. However, their application is somewhat limited by their scarcity and have so far only been administered intralesionally. Human monoclonal antibodies to ganglioside antigens have been administered intralesionally to patients suffering from cutaneous recurrent melanoma (Irie & Morton, 1986). Regression was observed in six out of ten patients, following, daily or weekly, intralesional injections. In another study, moderate success was achieved from intralesional injections of two human monoclonal antibodies (Irie *et al.,* 1989).

It may be favorable to administer more than one monoclonal antibody directed against two different antigens or even antibodies with multiple antigen specificity. Treatment protocols also may include administration of lymphokines or other immune enhancers as described by Bajorin *et al.* (1988). The development of human monoclonal antibodies is described in further detail elsewhere in the specification.

### ii. Active Immunotherapy

In active immunotherapy, an antigenic peptide, polypeptide or protein, or an autologous or allogenic tumor cell composition or "vaccine" is administered, generally with a distinct bacterial adjuvant (Ravindranath & Morton, 1991; Morton & Ravindranath, 1996; Morton *et al.,* 1992; Mitchell *et al.,* 1990; Mitchell *et al.,* 1993). In melanoma immunotherapy, those patients who elicit high IgM response often survive better than those who elicit no or low IgM antibodies (Morton *et al.,* 1992). IgM antibodies are often transient antibodies and the exception to the rule appears to be anti-ganglioside or anticarbohydrate antibodies.

### iii. Adoptive Immunotherapy

In adoptive immunotherapy, the patient's circulating lymphocytes, or tumor infiltrated lymphocytes, are isolated *in vitro,* activated by lymphokines such as IL-2 or transduced with genes for tumor necrosis, and readministered (Rosenberg *et al.,* 1988; 1989). To achieve this, one would administer to an animal, or human patient, an immunologically effective amount of activated lymphocytes in combination with an adjuvant-incorporated anigenic peptide composition as described herein. The activated lymphocytes will most preferably be the patient's own cells that were earlier isolated from a blood or tumor sample and activated (or "expanded") *in vitro.* This form of immunotherapy has produced several cases of regression of melanoma and renal carcinoma, but the percentage of responders were few compared to those who did not respond.

### d. Genes

Delivery of a vector encoding either a full length or truncated MDA-7 in conjuction with a second vector encoding one of the following gene products will have a combined anti-hyperproliferative effect on target tissues. Alternatively, a single vector encoding both genes may be used. A variety of proteins are encompassed within the invention, some of which are described below. Table 6 lists various genes that may be targeted for gene therapy of some form in combination with the present invention.

### i. Inducers of Cellular Proliferation

The proteins that induce cellular proliferation further fall into various categories dependent on function. The commonality of all of these proteins is their ability to regulate cellular proliferation. For example, a form of PDGF, the sis oncogene, is a secreted growth factor. Oncogenes rarely arise from genes encoding growth factors, and at the present, sis is the only known naturally-occurring oncogenic growth factor. In one embodiment of the present invention, it is contemplated that anti-sense mRNA directed to a particular inducer of cellular proliferation is used to prevent expression of the inducer of cellular proliferation.

The proteins FMS, ErbA, ErbB and neu are growth factor receptors. Mutations to these receptors result in loss of regulatable function. For example, a point mutation affecting the transmembrane domain of the Neu receptor protein results in the neu oncogene. The erbA oncogene is derived from the intracellular receptor for thyroid hormone. The modified oncogenic ErbA receptor is believed to compete with the endogenous thyroid hormone receptor, causing uncontrolled growth.

The largest class of oncogenes includes the signal transducing proteins (e.g., Src, Abl and Ras). The protein Src is a cytoplasmic protein-tyrosine kinase, and its transformation from proto-oncogene to oncogene in some cases, results via mutations at tyrosine residue 527. In contrast, transformation of GTPase protein ras from proto-oncogene to oncogene, in one example, results from a valine to glycine mutation at amino acid 12 in the sequence, reducing ras GTPase activity.

The proteins Jun, Fos and Myc are proteins that directly exert their effects on nuclear functions as transcription factors.

### ii. Inhibitors of Cellular Proliferation

The tumor suppressor oncogenes function to inhibit excessive cellular proliferation. The inactivation of these genes destroys their inhibitory activity, resulting in unregulated proliferation. The tumor suppressors p53, p16 and C-CAM are described below.

High levels of mutant p53 have been found in many cells transformed by chemical carcinogenesis, ultraviolet radiation, and several viruses. The p53 gene is a frequent target of mutational inactivation in a wide variety of human tumors and is already documented to be the most frequently mutated gene in common human cancers. It is mutated in over 50% of human NSCLC (*Hollstein et al.,* 1991) and in a wide spectrum of other tumors.

The p53 gene encodes a 393-amino acid phosphoprotein that can form complexes with host proteins such as large-T antigen and E1B. The protein is found in normal tissues and cells, but at concentrations which are minute by comparison with transformed cells or tumor tissue

Wild-type p53 is recognized as an important growth regulator in many cell types. Missense mutations are common for the p53 gene and are essential for the transforming ability of the oncogene. A single genetic change prompted by point mutations can create carcinogenic p53. Unlike other oncogenes, however, p53 point mutations are known to occur in at least 30 distinct codons, often creating dominant alleles that produce shifts in cell phenotype without a reduction to homozygosity. Additionally, many of these dominant negative alleles appear to be tolerated in the organism and passed on in the germ line. Various mutant alleles appear to range from minimally dysfunctional to strongly penetrant, dominant negative alleles (Weinberg, 1991).

Another inhibitor of cellular proliferation is p16. The major transitions of the eukaryotic cell cycle are triggered by cyclin-dependent kinases, or CDK's. One CDK, cyclin-dependent kinase 4 (CDK4), regulates progression through the G₁. The activity of this enzyme may be to phosphorylate Rb at late G₁. The activity of CDK4 is controlled by an activating subunit, D-type cyclin, and by an inhibitory subunit, the p16^{INK4} has been biochemically characterized as a protein that specifically binds to and inhibits CDK4, and thus may regulate Rb phosphorylation (Serrano *et al.,* 1993; Serrano *et al.,* 1995). Since the p16^{INK4} protein is a CDK4 inhibitor (Serrano, 1993), deletion of this gene may increase the activity of CDK4, resulting in hyperphosphorylation of the Rb protein. p16 also is known to regulate the function of CDK6.

p16^{INK4} belongs to a newly described class of CDK-inhibitory proteins that also includes p16^{B}, p19, p21^{WAF1}, and p27^{KIP1}, The p16^{INK4} gene maps to 9p21, a chromosome region frequently deleted in many tumor types. Homozygous deletions and mutations of the p16^{INK4} gene are frequent in human tumor cell lines. This evidence suggests that the p16^{INK4} gene is a tumor suppressor gene. This interpretation has been challenged, however, by the observation that the frequency of the p16^{INK4} gene alterations is much lower in primary uncultured tumors than in cultured cell lines (Caldas *et al.,* 1994; Cheng *et al.,* 1994; Hussussian *et al.,* 1994; Kamb *et al.,* 1994; Kamb *et al.,* 1994; Mori *et al.,* 1994; Okamoto *et al.,* 1994; Nobori *et al.,* 1995; Orlow *et al.,* 1994; Arap *et al.,* 1995). Restoration of wild-type p16^{INK4} function by transfection with a plasmid expression vector reduced colony formation by some human cancer cell lines (Okamoto, 1994; Arap, 1995).

Other genes that may be employed include Rb, APC, DCC, NF-1, NF-2, WT-1, MEN-1, MEN-II, zac1, p73, VHL, MMAC1 / PTEN, DBCCR-1, FCC, rsk-3, p27, p27/p16 fusions, p21/p27 fusions, antithrombotic genes *(e.g.,* COX-1, TFPI), PGS, Dp, E2F, *ras, myc, neu, raf, erb, fms, trk, ret, gsp, hst, abl,* E1A, p300, genes involved in angiogenesis (*e.g.*, VEGF, FGF, thrombospondin, BAI-1, GDAIF, or their receptors) and MCC.

### iii. Regulators of Programmed Cell Death

Apoptosis, or programmed cell death, is an essential process for normal embryonic development, maintaining homeostasis in adult tissues, and suppressing carcinogenesis (Kerr *et al.,* 1972). The Bcl-2 family of proteins and ICE-like proteases have been demonstrated to be important regulators and effectors of apoptosis in other systems. The Bcl-2 protein, discovered in association with follicular lymphoma, plays a prominent role in controlling apoptosis and enhancing cell survival in response to diverse apoptotic stimuli (Bakhshi *et al.,* 1985; Cleary and Sklar, 1985; Cleary *et al.,* 1986; Tsujimoto *et al.,* 1985; Tsujimoto and Croce, 1986). The evolutionarily conserved Bcl-2 protein now is recognized to be a member of a family of related proteins, which can be categorized as death agonists or death antagonists.

Subsequent to its discovery, it was shown that Bcl-2 acts to suppress cell death triggered by a variety of stimuli. Also, it now is apparent that there is a family of Bcl-2 cell death regulatory proteins which share in common structural and sequence homologies. These different family members have been shown to either possess similar functions to Bcl-2 (e.g., Bcl_{xL}, Bcl_{w}, Bclₛ, Mcl-1, Al, Bfl-1) or counteract Bcl-2 function and promote cell death (e.g., Bax, Bak, Bik, Bim, Bid, Bad, Harakiri).

### e. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present invention, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present invention may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### f. Other agents

It is contemplated that other agents may be used in combination with the present invention to improve the therapeutic efficacy of treatment. These additional agents include immunomodulatory agents, agents that affect the upregulation of cell surface receptors and GAP junctions, cytostatic and differentiation agents, inhibitors of cell adehesion, agents that increase the sensitivity of the hyperproliferative cells to apoptotic inducers, or other biological agents. Immunomodulatory agents include tumor necrosis factor; interferon alpha, beta, and gamma; IL-2 and other cytokines; F42K and other cytokine analogs; or MIP-1, MIP-lbeta, MCP-1, RANTES, and other chemokines. It is further contemplated that the upregulation of cell surface receptors or their ligands such as Fas / Fas ligand, DR4 or DR5 / TRAIL (Apo-2 ligand) would potentiate the apoptotic inducing abililties of the present invention by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with the present invention to improve the anti-hyerproliferative efficacy of the treatments. Inhibitors of cell adehesion are contemplated to improve the efficacy of the present invention. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present invention to improve the treatment efficacy.

Apo2 ligand (Apo2L, also called TRAIL) is a member of the tumor necrosis factor (TNF) cytokine family. TRAIL activates rapid apoptosis in many types of cancer cells, yet is not toxic to normal cells. TRAIL mRNA occurs in a wide variety of tissues. Most normal cells appear to be resistant to TRAIL's cytotoxic action, suggesting the existence of mechanisms that can protect against apoptosis induction by TRAIL. The first receptor described for TRAIL, called death receptor 4 (DR4), contains a cytoplasmic "death domain"; DR4 transmits the apoptosis signal carried by TRAIL. Additional receptors have been identified that bind to TRAIL. One receptor, called DR5, contains a cytoplasmic death domain and signals apoptosis much like DR4. The DR4 and DR5 mRNAs are expressed in many normal tissues and tumor cell lines. Recently, decoy receptors such as DcR1 and DcR2 have been identified that prevent TRAIL from inducing apoptosis through DR4 and DR5. These decoy receptors thus represent a novel mechanism for regulating sensitivity to a pro-apoptotic cytokine directly at the cell's surface. The preferential expression of these inhibitory receptors in normal tissues suggests that TRAIL may be useful as an anticancer agent that induces apoptosis in cancer cells while sparing normal cells. (Marsters *et al.* 1999).

There have been many advances in the therapy of cancer following the introduction of cytotoxic chemotherapeutic drugs. However, one of the consequences of chemotherapy is the development/acquisition of drug-resistant phenotypes and the development of multiple drug resistance. The development of drug resistance remains a major obstacle in the treatment of such tumors and therefore, there is an obvious need for alternative approaches such as gene therapy.

Studies from a number of investigators have demonstrated that tumor cells that are resistant to TRAIL can be sensitized by subtoxic concentrations of drugs/cytokines and the sensitized tumor cells are significantly killed by TRAIL. (Bonavida *et al.,* 1999; Bonavida *et al.,* 2000; Gliniak *et al.,* 1999; Keane *et al.,* 1999). Ad-mda7 treatment of cancer cells results in the up-regulation of mRNA for TRAIL and TRAIL receptors. Therefore, administration of the combination of Ad-mda7 with recombinant TRAIL can be used as a treatment to provide enhanced anti-tumor activity. Furthermore, the combination of chemotherapeutics, such as CPT-11 or doxorubicin, with TRAIL also lead to enhanced anti-tumor activity and an increase in apoptosis. The combination of Ad-mda7 with chemotherapeutics and radiation therapy, including DNA damaging agents, will also provide enhanced anti-tumor effects. Some of these effects may be mediated via up-regulation of TRAIL or cognate receptors, whereas others may not. For example, enhanced anti-tumor activity with the combinations of Ad-mda7 and tamoxifen or doxorubicin (adriamycin) has been observed. Neither tamoxifen nor adriamycin are known to up-regulate TRAIL or cognate receptors.

Another form of therapy for use in conjunction with chemotherapy, radiation therapy or biological therapy includes hyperthermia, which is a procedure in which a patient's tissue is exposed to high temperatures (up to 106°F). External or internal heating devices may be involved in the application of local, regional, or whole-body hyperthermia. Local hyperthermia involves the application of heat to a small area, such as a tumor. Heat may be generated externally with high-frequency waves targeting a tumor from a device outside the body. Internal heat may involve a sterile probe , including thin, heated wires or hollow tubes filled with warm water, implanted microwave antennae, or radiofrequency electrodes.

A patient's organ or a limb is heated for regional therapy, which is accomplished using devices that produce high energy, such as magnets. Alternatively, some of the patient's blood may be removed and heated before being perfused into an area that will be internally heated. Whole-body heating may also be implemented in cases where cancer has spread throughout the body. Warm-water blankets, hot wax, inductive coils, and thermal chambers may be used for this purpose.

Hormonal therapy may also be used in conjunction with the present invention or in combination with any other cancer therapy previously described. The use of hormones may be employed in the treatment of certain cancers such as breast, prostate, ovarian, or cervical cancer to lower the level or block the effects of certain hormones such as testosterone or estrogen. This treatment is often used in combination with at least one other cancer therapy as a treatment option or to reduce the risk of metastases.

**TABLE 6: Oncogenes**

| ***Gene*** | ***Source*** | ***Human Disease*** | ***Function*** |
|---|---|---|---|
| **Growth Factors¹** | | | FGF family member |
| *HSTIKS* | Transfection | | |
| *INT-2* | MMTV promoter | | FGF family member |
| | Insertion | | |
| *INTI*/*WNTI* | MMTV promoter | | Factor-like |
| | Insertion | | |
| *SIS* | Simian sarcoma | | PDGF B |
| | virus | | |

| **Receptor Tyrosine Kinases^{1,2}** | | | |
|---|---|---|---|
| *ERBB*/*HER* | Avian | Amplified, deleted | EGF/TGF-α/ |
| | erythroblastosis | squamous cell | amphiregulin/ |
| | virus; ALV | cancer; | hetacellulin receptor |
| | promoter | glioblastoma | |
| | insertion; | | |
| | amplified | | |
| | human tumors | | |
| *ERBB-2*/*NEU*/*HER-2* | Transfected from | Amplified breast, | Regulated by NDF/ |
| | rat | ovarian, gastric | heregulin and EGF- |
| | Glioblastomas | cancers | related factors |
| *FMS* | SM feline sarcoma | | CSF-1 receptor |
| | virus | | |
| *KIT* | HZ feline sarcoma | | MGF/Steel receptor |
| | virus | | Hematopoieis |
| *TRK* | Transfection from | | NGF (nerve growth |
| | human colon | | factor) receptor |
| | cancer | | |
| *MET* | Transfection from | | Scatter factor/HGF |
| | human | | Receptor |
| | osteosarcoma | | |
| RET | Translocations | Sporadic thyroid | Orphan receptor Tyr |
| | and point | cancer; | Kinase |
| | mutations | familial medullary | |
| | | thyroid cancer; | |
| | | multiple endocrine | |
| | | neoplasias 2A and | |
| | | 2B | |
| *ROS* | URII avian | | Orphan receptor Tyr |
| | sarcoma | | Kinase |
| | Virus | | |
| *PDGF* receptor | Translocation | Chronic | TEL(ETS-like |
| | | Myelomonocytic | transcription factor)/ |
| | | Leukemia | PDGF receptor gene |
| | | | Fusion |
| *TGF-β* receptor | | Colon carcinoma | |
| | | mismatch mutation | |
| | | target | |

| **NONRECEPTOR TYROSINE KINASES¹** | | | |
|---|---|---|---|
| *ABI.* | Abelson Mul.V | Chronic | Interact with RB, |
| | | myelogenous | RNA |
| | | leukemia | polymerase, CRK, |
| | | translocation | CBL |
| | | with BCR | |
| *FPS*/*FES* | Avian Fujinami | | |
| | SV;GA | | |
| | FeSV | | |
| *LCK* | Mul.V (murine | | Src family; T cell |
| | leukemia | | signaling; interacts |
| | virus) promoter | | CD4/CD8 T cells |
| | insertion | | |
| *SRC* | Avian Rous | | Membrane-associated |
| | sarcoma | | Tyr kinase with |
| | Virus | | signaling function; |
| | | | activated by receptor |
| | | | kinases |
| *YES* | Avian Y73 virus | | Src family; signaling |

| **SER/THR PROTEIN KINASES¹** | | | |
|---|---|---|---|
| *AKT* | AKT8 murine | | Regulated by |
| | retrovirus | | PI(3)K?; |
| | | | regulate 70-kd S6 k? |
| *MOS* | Maloney murine | | GVBD; cystostatic |
| | SV | | factor; MAP kinase |
| | | | kinase |
| *PIM 1* | Promoter insertion | | |
| | Mouse | | |
| *RAF*/*MlL* | 3611 murine SV; | | Signaling in RAS |
| | MH2 | | Pathway |
| | avian SV | | |

| **MISCELLANEOUS CELL SURFACE¹** | | | |
|---|---|---|---|
| *APC* | Tumor suppressor | Colon cancer | Interacts with |
| | | | catenins |
| *DCC* | Tumor suppressor | Colon cancer | CAM domains |
| E-cadherin | Candidate tumor | Breast cancer | Extracellular |
| | Suppressor | | homotypic |
| | | | binding; |
| | | | intracellular |
| | | | interacts with |
| | | | catenins |
| *PTC*/*NBCCS* | Tumor suppressor | Nevoid basal cell | 12 transmembrane |
| | and | cancer | domain; signals |
| | *Drosophilia* | syndrome (Gorline | through Gli |
| | homology | syndrome) | homogue |
| | | | CI to antagonize |
| | | | hedgehog pathway |
| *TAN-1* Notch | Translocation | T-ALI. | Signaling? |
| homologue | | | |

| **MISCELLANEOUS SIGNALING^{1,3}** | | | |
|---|---|---|---|
| *BCL-2* | Translocation B-cell lymphoma Apoptosis | | |
| *CBL* | Mu Cas NS-1 V | | Tyrosine- |
| | | | Phosphorylated |
| | | | RING |
| | | | finger interact Ab1 |
| CRK | CT1010 ASV | | Adapted SH2/SH3 |
| | | | interact Abl |
| *DPC4* | Tumor suppressor Pancreatic cancer TGF-β-related | | |
| | | | signaling |
| | | | Pathway |
| *MAS* | Transfection and | | Possible angiotensin |
| | Tumorigenicity | | Receptor |
| *NCK* | | | Adaptor SH2/SH3 |

| **GUANINE NUCLEOTIDE EXCHANGERS AND BINDING** | | | |
|---|---|---|---|
| **PROTEINS^{3,4}** | | | |
| *BCR* | | Translocated | Exchanger; protein |
| | | with ABL | Kinase |
| | | in CML | |
| *DBL* | Transfection | | Exchanger |
| *GSP* | | | |
| *NF-1* | Hereditary tumor | Tumor | RAS GAP |
| | Suppressor | suppressor | |
| | | neurofibroma | |
| | | tosis | |
| *OST* | Transfection | | Exchanger |
| Harvey-Kirsten, N-*RAS* | HaRat SV; Ki RaSV; | Point | Signal cascade |
| | Balb-MoMuSV; | mutations in | |
| | Transfection | many | |
| | | human | |
| | | tumors | |
| *VAV* | Transfection | | S 112/S 113 ; |
| | | | exchanger |

| **NUCLEAR PROTEINS AND TRANSCRIPTION FACTORS^{1,5-9}** | | | |
|---|---|---|---|
| *BRCA1* | Heritable suppressor | Mammary | Localization unsettled |
| | | cancer/ovaria | |
| | | n cancer | |
| *BRCA2* | Heritable suppressor | Mammary | Function unknown |
| | | cancer | |
| *ERBA* | Avian erythroblastosis | | thyroid hormone |
| | Virus | | receptor |
| | | | (transcription) |
| *ETS* | Avian E26 virus | | DNA binding |
| *EVII* | MuLV promotor | AML | Transcription factor |
| | Insertion | | |
| *FOS* | FBI/FBR murine | | 1 transcription factor |
| | osteosarcoma viruses | | with c-JUN |
| *GLI* | Amplified glioma | Glioma | Zinc finger; cubitus |
| | | | interruptus |
| | | | homologue |
| | | | is in hedgehog |
| | | | signaling pathway; |
| | | | inhibitory link PTC |
| | | | and hedgehog |
| *HMGI*/*LIM* | Translocation t(3:12) | Lipoma | Gene fusions high |
| | *t*(12:15) | | mobility group |
| | | | HMGI-C (XT-hook) |
| | | | and transcription |
| | | | factor |
| | | | LIM or acidic |
| | | | domain |
| *JUN* | ASV-17 | | Transcription factor |
| | | | AP-1 with FOS |
| *MLL*/*VHRX + ELI*/*MEN* | Translocation/fusion | Acute | Gene fusion of DNA- |
| | ELL with MLL | myeloid | binding and methyl |
| | Trithorax-like gene | leukemia | transferase MLL |
| | | | with |
| | | | ELI RNA pol II |
| | | | elongation factor |
| *MYB* | Avian myeloblastosis | | DNA binding |
| | Virus | | |
| *MYC* | Avian MC29; | Burkitt's | DNA binding with |
| | Translocation B-cell | lymphoma | MAX partner; cyclin |
| | Lymphomas; promoter | | regulation; interact |
| | Insertion avian leukosis | | RB?; regulate |
| | Virus | | apoptosis? |
| *N-MYC* | Amplified | Neuroblastom | |
| | | a | |
| *L-MYC* | | Lung cancer | |
| *REL* | Avian | | NF-κB family |
| | Retriculoendotheliosis | | transcription factor |
| | Virus | | |
| *SKI* | Avian SKV770 | | Transcription factor |
| | Retrovirus | | |
| *VHL* | Heritable suppressor | Von Hippel- | Negative regulator or |
| | | Landau | elongin; |
| | | syndrome | transcriptional |
| | | | elongation complex |
| *WT-1* | | Wilm's tumor | Transcription factor |

| **CELL CYCLE/DNA DAMAGE RESPONSE¹⁰⁻²¹** | | | |
|---|---|---|---|
| *ATM* | Hereditary disorder | Ataxia- | Protein/lipid kinase |
| | | telangiectasia | homology; DNA |
| | | | damage response |
| | | | upstream in P53 |
| | | | pathway |
| *BCL-2* | Translocation | Follicular | Apoptosis |
| | | lymphoma | |
| *FACC* | Point mutation | Fanconi's | |
| | | anemia group | |
| | | C | |
| | | (predispositio | |
| | | n | |
| | | leukemia | |
| *FHIT* | Fragile site 3p14.2 | Lung | Histidine triad-related |
| | | carcinoma | diadenosine 5',3""- |
| | | | P¹.p⁴ tetraphosphate |
| | | | asymmetric |
| | | | hydrolase |
| *hMLI*/*MutL* | | HNPCC | Mismatch repair; |
| | | | MutL |
| | | | Homologue |
| *hMSH2*/*MutS* | | HNPCC | Mismatch repair; |
| | | | MutS |
| | | | Homologue |
| *hPMS1* | | HNPCC | Mismatch repair; |
| | | | MutL |
| | | | Homologue |
| *hPMS2* | | HNPCC | Mismatch repair; |
| | | | MutL |
| | | | Homologue |
| *INK4*/*MTS1* | Adjacent INK-4B at | Candidate | p16 CDK inhibitor |
| | 9p21; CDK complexes | MTS1 | |
| | | suppressor | |
| | | and MLM | |
| | | melanoma | |
| | | gene | |
| *INK4B*/*MTS2* | | Candidate | p15 CDK inhibitor |
| | | suppressor | |
| *MDM-2* | Amplified | Sarcoma | Negative regulator |
| | | | p53 |
| p53 | Association with SV40 | Mutated | Transcription factor; |
| | T antigen | >50% human | checkpoint control; |
| | | tumors, | apoptosis |
| | | including | |
| | | hereditary | |
| | | Li-Fraumeni | |
| | | syndrome | |
| *PRAD1*/*BCG1* | Translocation with | Parathyroid | Cyclin D |
| | Parathyroid hormone | adenoma; | |
| | or IgG | B-CLL | |
| RB | Hereditary | Retinoblasto | Interact cyclin/cdk: |
| | Retinoblastoma; | ma; | regulate E2F |
| | Association with many | | transcription factor |
| | DNA virus tumor | osteosarcoma | |
| | Antigens | ; breast | |
| | | cancer; | |
| | | other | |
| | | sporadic | |
| | | cancers | |
| *XPA* | | xeroderma | Excision repair: |
| | | | photo- |
| | | pigmentosum; | product recognition; |
| | | skin | zinc finger |
| | | cancer | |
| | | predisposition | |

### B. EXAMPLES

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1: TRUNCATED MDA-7

### 1. Materials and Methods

### a. Animals

3-6 wk-old female/male BALB/c nude mice were purchased from Harlan Inc. (Indianapolis, IN).

### b. Virus

Control adenovirus (Ad-c) was prepared by deletion of E1 and partial E3 regions from adenovirus serotype 5. An adenovirus encoding human extracellular MDA-7 (Ad-mda7^{EC}) will be constructed by Introgen Therapeutics Inc., Houston, TX. Extracellular MDA-7 refers to the secreted portion of the MDA-7 protein.

### c. Cell preparation and infection with adenovirus

Where possible, cell lines are obtained from the American Type Culture Collection (ATTC, Rockville, MD). The cells are grown in DMEM medium (GIBCO/BRL, Life Technologies, Grand island, NY) with 100 IU/ml penicillin, 0.1 mg/mL streptomycin and 10% fetal calf serum, HyClone, Logan, UT), according to ATCC's recommendation. The cells will be tested and verified to be free of mycoplasma and used in the log phase of growth. Cells are routinely harvested with 0.125% Trypsin -1.3 mM EDTA (GIBCO).

### d. In vitro transfection

Cells are plated at a density of approximately 5 x 10⁵ cells per 60 mm² in RPMI/10% FBS media and grown in 5% CO₂ at 37°C. Plating densities vary somewhat depending on cell growth rates etc. and are determined empirically.

### e. Recombinant Adenovirus Production

Replication deficient human type 5 Adenovirus (Ad5) carrying the nucleic acid encoding extracellular human MDA-7 (or luciferase gene) linked to an internal CMV-IE promoter and followed by SV40 polyadenylation (pA) signal will be constructed. The adenovirus contruct encoding extracellular MDA-7 is designated Ad-mda7^{TF}, Ad-mda7^{EC}. and Ad-mda7^{TR}, which are used interchangeably to refer to an adenovirus construct encoding a portion of the full-length mda-7 gene sequence. Ad-mda7^{TF} will contain a nucleic acid sequence encoding a truncated transcript that encodes a truncated human MDA-7. An adenovirus (Ad5) construct encoding the full-length mda-7 gene sequence was made as described *infra* and used for some of these experiments.

The Ad-5 vectors harboring the gene cassettes will be co-transfected with plasmid pJM 17 (Graham and Prevec 1992) in 293 cells to rescue recombinant viruses Ad-mda7^{TF}. Plaques will be picked, virus stocks will be grown and their genomes will be confirmed as correct by PCR/restriction analysis and sequencing. Viruses will be propagated in 293 cells and purified by chromatography. Control vectors including Ad-Luc (encoding the firefly luciferase gene), Ad-beta-gal (containing the bacterial beta-gal gene), Ad-GFP (containing the Green Fluorescent Protein) and Ad-CMVpA (containing the expression cassette, but no transgene) have been constructed and purified.

### f. Transduction and Cell Proliferation studies

Cancer or normal cell lines used in this study will be infected with Ad-mda7^{TR} (with either AdCMVpA or AdLuc as controls) in increasing MOIs (multiplicities of infection) or viral particles/cell (0, 100, 250, 500, 1000, 2500, 5000, 10000 vp/cell increasing concentrations). Cells will be plated at 500-2000 cells/well in 96-well format for tritiated thymidine incorporation cell proliferation assay or plated at 10⁵-10⁶ cells/well in a 6 well plate for protein expression or apoptosis assays or plated at 10⁴ cells/well for alamar-blue assay.

For infection Ad-mda7^{TR} or AdLuc (or AdCMVpA) will be used at increasing MOIs (based on viral particles/cell; MOI ranged from 0-10,000 viral particles/cell). For tritiated thymidine/apoptosis and protein expression and alamar assays, cells are analyzed 3 and 5 days post-infection

### g. Tritiated-Thymidine Assay

Growth inhibition of cells after treatment will be measured by analysis of DNA-synthesis. Briefly, for the H³-thymidine incorporation assay cells are plated at 200-5000 cell per well in a 96-well format and grown in DMEM/10% FBS in a 5% CO₂ incubator at 37°C overnight. The next day the media is aspirated and replaced with 50 µl DMEM/10% FBS containing the appropriate adenovirus at the appropriate MOI. Cells will be incubated with infecting media for 1 to 4 hrs and then diluted to 200 µl total volume and grown overnight. Media is replaced with DMEM/10% FBS/mCi H³-thymidine and grown for 16 to 18 hrs. Stock solution of 100uCi/mL of H³-thymidine (Amersham) is prepared by dilution into high glucose DMEM (GIBCO). H³-Thymidine is added to each well at a final concentration of 1 µCi/mL. The reaction is stopped 15 hours later by removal of the supernatant from recipient cells. The cells are harvested by addition of 100x Trypsin/EDTA (GIBCO) to each well for 15 minutes at 37°C. Cells are collected on a filter in the 96-well format using a Packard Filtermate Cell Harvester following the manufacturer's protocol and washed in deionized water and methanol. The filter are dried and analyzed in Matrix 9600 (Packard) and cell proliferation using Viral Particles/cell against Tritiated Thymidine uptake counts are plotted.

### h. Alamar Blue Assay

Growth inhibition of cells also will be measured by alamar blue assay. Briefly, cells are plated at 10⁴ cells/well density in a 96-well plate format. Four days after infection with different MOIs of Ad-mda7^{TR} or control vectors (as previously described), 20 µL of alamar blue dye is added to each well and the plate is incubated at 37°C for 6-8 hours. The plates are then read for optical density on the Dynatech MRX plate reader at wavelength of 595 nm. Revelation 3.2 software program is used to plot MOIs against optical density values at 595 nm.

### i. TUNEL Assay

Cancer cells will be seeded in Lab-Tek chamber slides (Nunc) at density of 10⁴ cells/chamber. Cells are transduced with desired concentration of Ad vectors. At different day points, post-infection, cells are analyzed according to manufacturer's instruction for apoptosis using the Chromogenic TUNEL-peroxidase assay ("In Situ Death Detection Kit, POD", Boehringer Mannheim).

### j. Annexin V Assay

Cancer cells will also be analyzed for Apoptosis, post-Ad-mda7^{TR} treatment, by ApoAlert Annexin V-FITC kit (CLONTECH). After induction of apoptosis in cells, phosphatidylserine (PS), which is predominantly located on the inner leaflet of the plasma membrane, is rapidly translocated to the outer leaflet via a flippase mechanism. In the presence of Ca²⁺, annexin V binds PS with high affinity and FITC conjugated to Annexin help to pinpoint apoptotic cells both via flourescent microscopy and FACs analysis.

### k. DNA staining with Propidium Iodide (PI)

For determining cells at different stages of cell cycle, Ad-mda7^{TR}-infected cancer cells will be prepared as a single cell suspension of 1-2 x 10⁶ cells/mL of PBS. After the cells are fixed with cold 70% ethanol for 2 hours, the cells are centrifuged, and the fixative decanted, and washed 2x with PBS and then stained with propidium iodide working solution, which includes PI at 50 µg/mL and RNAse at 20 µg/mL in PBS. Treated cells are then analyzed by FACS.

### 1. Tumor Xenograft Models

Tumor cells are plated at a density of approximately 20-40% confluency in 150 mm² dishes in RPMI/10% FBS media supplemented with penicillin, streptomycin and fungizone, and grown in 5% CO₂ at 37°C until approximately 80% confluent. Cells are washed twice in PBS, trypsinized, and counted. Cells are diluted to a concentration of 5 x 10⁶ cells/100 µl in PBS. BALB/c nude mice will be injected subcutaneously with 5 x 10⁶ tumor cells in 100 µl of PBS.

### 2. Adenovirus Encoding the Truncated Human MDA-7 Protein (Ad-_{mda7}^{TF}) Produces a Secreted Protein

Crude cell fractionation studies will be done to demonstrate that MDA-7^{TF} is secreted from cells transduced by Ad-mda7^{TF}. MDA-7^{TF} refers specifically to a truncated form of the MDA-7 protein; it includes the secreted form of MDA-7. Cells are plated as described and grown in DMEM/10% FBS in a 5% CO₂ incubator at 37°C overnight. The next day media is removed by aspiration and replace with fresh media containing Ad-mda7^{TF} or Ad-control. Media is aspirated after a 3 hr incubation with adenovirus and replaced with fresh media. Cells are returned to the 5% CO incubator to be grown overnight at 37°C. Cells are harvested the next day and media is harvested by centrifugation. Western blot analysis to detect the MDA-7 protein is performed on cell extracts and total media. This protocol was used with Ad-mda7, and two immunoreactive bands were detected on the western blot using anti-MDA-7 antibody. The bands were approximated to be 23kDa and 18kDa, which correspond with the unprocessed and processed forms of MDA-7 (full-length MDA-7 and the intra-cellularly cleaved form of MDA-7).

### 3. MDA-7 Is Localized to Vesicles and the Cell Surface by Confocal Microscopy

Expression of MDA-7 was also analyzed by FACS and Confocal microscopy. In brief, cancer cells (H460. DLD-1. H1299) were transduced with Ad-mda-7 at MOIs of 1000 and 5000 Vp/cell. 24 hours later the cells were washed with PBS and labeled with anti-mda-7 rabbit polyclonal antibody (1:5000 dilution of 1 mg/ml affinity purified antibody obtained from Corixa Inc.) for 1 hour at 4°C. The first treatment was followed by series of PBS washes and secondary anti-rabbit IgG-FITC (1:1000 dilution, Santa-Cruz Biotechnology), which was incubated with the cells for 1 hour on ice. The cells were washed and fixed with 4% HCHO-PBS and analyzed by FACS and Confocal Microscopy.

### 4. Elevated Expression of MDA-7^{TR} in vitro Induces Cancer Cell-Specific Growth Arrest and Apoptosis

Expression of MDA7^{TR} will inhibit growth and induce apoptosis in cancer cell lines but not in normal cell lines. Ad-mda7^{TR} will be transduced as previously described. Growth inhibition will be evaluated by ³H-thymidine incorporation and alamar blue assays. Cancer cells treated with MDA7^{TF} will show a reduction in the rate of ³H-thymidine incorporation as compared to normal cells transduced with mda7^{TF} and vector controls. Alamar blue assays will demonstrate a reduced optical density in MDA7^{TF}-expressing normal cells and transduced controls as compared to MDA7^{TF}-expressing cancer cells. These assays will be indicative of MDA7^{TF} expression specifically growth arresting cancer cells.

In addition, apoptosis will be evaluated by using TUNEL assay, surface expression of Annexin V, and cell cycle analysis using propidium iodide staining. The TUNEL assay evaluates cellular nuclease activity. Activated nuclease activity, indicative of cells undergoing apoptosis, will produce an intense color reaction in comparison with the background staining associated with non-apoptotic cells. Cancer cell lines expressing MDA-7^{TF} will demonstrate a significant increase in the number TUNEL positive cells as compared to mda-7^{TF}-expressing normal cell lines and control vector transduced cells. Another measure of apoptotic activity is the surface expression of annexin V as detected by surface staining using an annexin V anti-body. Cancer cell lines expressing mda7^{TF} will demonstrate a significant increase in the number of Annexin V positive cells as compared to mda-7^{TF}-expressing normal cell lines and control vector transduced cells, indicating an increased rate of apoptosis in the treated cancer cell lines as compared with normal cells and control treated cancer cells.

### 5. Intratumoral Administration of Ad-mda7^{TF} to H1299 Subcutaneous Tumors Inhibits Tumor Growth

For intratumoral delivery of Ad-mda7^{TF} 3-4 week old female BALB/c nude mice will be subcutaneously injected with 5x10⁶ H1299 cells per animal. Animals will be treated five days post-H1299 injection by intratumoral injection of 100 µl of Ad-mda7^{TF}. Tumors are measured every other day. Treatment groups will be 1) no treatment 2) intratumoral injection every day for 6 days with Ad-mda7^{TF} 3) intratumoral injection every day for 6 days with Ad-control. Tumor size is measured every other day for 16 days. Ad-mda7^{TF} DNA will show a significant inhibition of tumor growth compared to no treatment and DNA alone.

### EXAMPLE 2: MATERIALS AND METHODS

### 1. Cell Lines and Cell Culture

All the tumor cell lines utilized were obtained from American Type Culture Collection (ATCC, Rockville, MD). The cell lines evaluated were: breast (MCF-7, T47D, SKBr3, HBL-100, BT-20, MDA-MB-231, MDA-MB-468, MDA-MB-361), colorectal (DLD-1, SW-620, SW-480, HT-29, HCT-116, LS174T), lung (H1299, H460, A549, H322, H358) and osteosarcoma (SaosLM2) cancer lines. Normal cell lines were obtained from the Clonetics (San Diego) and included HUVEC (human umbelical vein endothelial cells), normal melanocytes, and HMEC (human mammary epithelial cells). MJ90 primary fibroblasts (obtained from Baylor College of Medicine) were also used. The cells were grown in DMEM medium (GIBCO/BRL, Life Technologies, Grand Island, NY) and fetal bovine serum (at 5-10% final concentration, according to suppliers recommendation). The cells were free of mycoplasma and were used in the log phase of growth. Cells were harvested with 0.125% Trypsin -1.3 mM EDTA (GIBCO).

### 2. Recombinant Adenovirus Production

The construction of Ad-mda7 was initiated by using a construct obtained from Dr. Paul Fisher (Columbia University, NY) that has the mda-7 cDNA inserted into a TA cloning vector (Invitrogen Inc., San Diego CA). The mda-7 cDNA, isolated as a Hind III-Not I fragment, was cloned into a shuttle vector (pIN147, obtained from the laboratory of J.A. Roth, M.D.Anderson Cancer Center. Houston, TX) using the Hind III-Not I restriction sites. pIN147 is a shuttle plasmid that is based on a pBR322 backbone and contains the CMV promoter and SV40 poly A elements substituted into the E1 region of Ad 5. pIN147 contains bases #1-456 of the left end of Ad5, the CMV expression cassette and then Ad5 bases #3333-5789. The pIN147-mda-7 cDNA expression construct was named pIN207. Ad-mda7 was constructed by cotransfecting 293 cells with pIN207 and pIN153 using the calcium phosphate kit and protocol provided by GibcoBRL (#123, pp44-46). pIN153 is identical to JM17 obtained from the laboratory of Graham, 1988. (A simple technique for the rescue of early region 1 mutations into infectious human adenovirus type 5, *McGrory et al.,* 1988) and contains most of the Ad5 genome, but lacks the left end. After observing cytopathic effect (CPE), the resulting cell lysate that contained the vector, Ad-mda7, was subjected to two sequential rounds of plaque purification using the following protocol. Briefly, the vector-containing lysate was serially diluted in media (DMEM) in five-fold increments and 400 µl of each dilution was used to infect a confluent layer of 293 cells plated on 6-well plates. The wells were overlayed with 1% agar in media and the resulting plaques were picked one week later. Each of four plaques was resuspended in 500 µl media, vortexed, centrifuged and the supernatant used to infect one well of confluent 293 cells plated on 6-well plates. The cells were allowed to undergo lysis due to Ad vector replication and DNA from each expanded primary plaque was purified using the QIAmp DNA purification kit and protocol provided by Qiagen, Inc. The DNA was examined by PCR analysis, and one plaque was picked to undergo secondary plaque purification, which was performed as above. Plaques were isolated and used to infect 293 cells (as above) in one well of a 6-well plate; this lysate was then used to infect confluent 293 cells plated on a T-75 flask.

Finally, approximately 5x10⁸ cells were infected from the resulting cell lysate Lysate from this sample was then transferred to manufacturing for large-scale amplification and purification. Ad-mda7 (lot #B2119901) and Ad-luc (lot #00697004) were used in the preclinical studies below. Early research studies used Ad-mda7 vector obtained from Dr. Paul Fisher, Columbia University. The Fisher vector uses a similar first generation Ad vector with E1 and E3 deletions. This material was purified and amplified at Introgen Therapeutics Inc and shown to be bioequivalent to the Introgen Ad-mda7 used in all subsequent experiments.

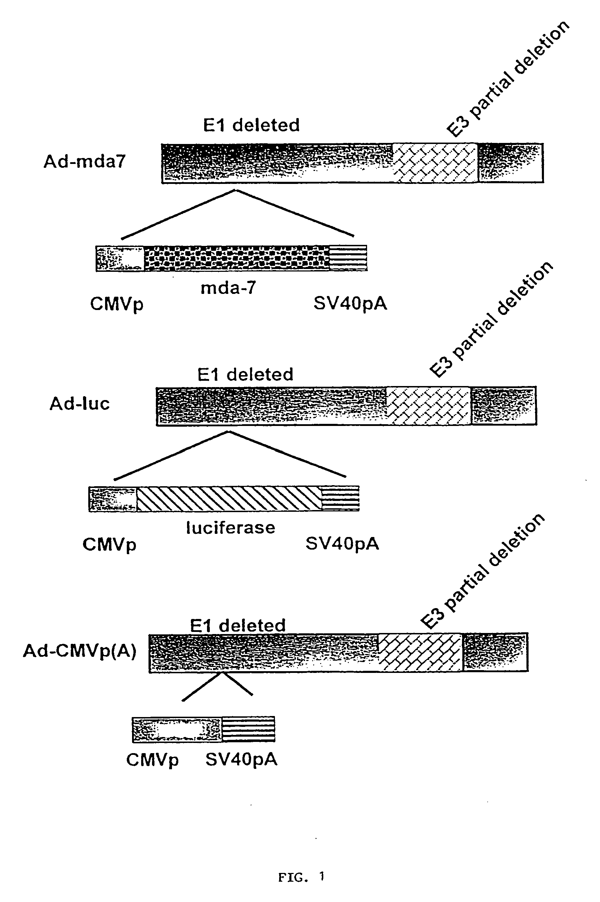

Ad-Luc and Ad-CMVp(A) (Luciferase and empty vector, respectively), were used as control viruses (FIG. 1). For generation of Ad-vectors, Ad5 vectors harboring the gene cassettes were co-transfected with plasmid pJM17 (Graham and Prevec 1992) in 293 cells to rescue recombinant Ad-mda7, Ad-Luc and Ad-CMVp(A) viruses. Plaques were picked, virus stocks were grown, and their genomes were confirmed as correct by PCR/Restriction analysis and DNA sequencing. Viruses were propagated in 293 cells and purified by HPLC.

### 3. MDA-7 Polyclonal Antibodies and Western Blot Analysis

Recombinant MDA-7 protein was produced in *E. coli* and was purified on a nickel NTA agarose column. The material was bound to the nickel resin in a batch mode for 45 minutes and then poured into a column and the eluate was run through the column bed. The material was washed with 10 mM Tris pH 8.0 containing 0.5% chaps and finally eluted off of the column with 10 mM Tris pH 8.0 plus 400 mM imidazole. The eluted MDA-7 was dialyzed against 10 mM Tris pH 8.0. The final product was shown to be a single band with a molecular weight of approx. 23 kDa. The amino terminal protein sequence was shown to be correct and purity was estimated to be greater than 90%.

This material was injected into rabbits using the following protocol: 400 µg MDA-7 protein with IFA and 100 µg of MDP was injected subcutaneously, 3 weeks later 200 ug MDA-7 protein with IFA was injected and 3 weeks after that another 100 µg of MDA-7 protein was injected intravenously. The titer of antiserum was shown to be greater than 1/100,000 based on an ELISA assay.

The MDA-7 protein was coupled via sulfhydryl linkage to a solid support resin. The resin and bound protein was thoroughly washed. This washed material was used to make an MDA-7 column for antibody purification. The rabbit polyclonal sera was diluted 1:1 with 20 mM Tris buffer pH 8.0 and filtered through a 0.2-micron filter before being pumped onto the MDA-7 column. The column was then washed with the same 20 mM Tris buffer pH 8.0 until the absorbance returned to baseline. The antibody was eluted off the column with 0.1 M acetic acid. The eluent containing the antibody was immediately adjusted back to pH 8.0. This affinity-purified antibody was then dialyzed against 10 mM Tris pH 8.0 and concentrated.

Recombinant MDA-7 protein was expressed in *E. coli* and purified using a nickel NTA agarose column. This recombinant MDA-7 protein was used to generate rabbit polyclonal antibodies, which were purified by affinity chromatography. This antibody was used in Western blot analysis at concentrations ranging from 1:1000-1:10,000 dilution (from stock of 1 mg/mL). Cell lysates (10⁵-10⁶ cells were suspended in 500 µL of Laemmli buffer with 5% 2-mercaptoethanol[2ME]) or supernatants (1:1 mixing with Laemmli buffer +2ME), were obtained after cancer cells were treated with Ad-mda7 for desired length/s of time, followed by SDS polyacrylamide gel electrophoresis and western blot analysis using the Super-Signal substrate for horseradish peroxidase (Pierce Inc.). Other monoclonal antibodies used in the study specifically recognized Bax (Santa Cruz Biotechnology) and β-actin (Sigma).

### 4. Transduction and Cell Proliferation Studies

Cancer or normal cell lines used in this study were infected with Ad-mda7 (using Ad-CMVp(A) or Ad-Luc as controls) with increasing MOIs (0 100, 250, 500, 1000, 2500, 5000 and 10.000 viral particles (vp) /cell). Cells were either plated at 500-2000 cells/well in 96-well format for ³H-thymidine incorporation-assay, or plated at 10⁵-10⁶ cells/well in a 6-well plate format for protein expression or apoptosis assays, and plated at 10⁴ cells/well (96-well format) for Alamar-blue assay. For infection, Ad-mda7 or Ad-Luc (or Ad-CMVp(A) were used at increasing MOIs (based on viral particles/cell; MOI ranged from 0-10,000 vp/cell). For ³H-thymidine-incorporation, apoptosis, protein expression and Alamar assays, cells were analyzed 3 and 5 days post-infection

### 5. ³H thymidine Assay

Growth inhibition of cells after treatment was primarily measured by analysis of incorporation of ³H-thymidine into replicating DNA. Briefly, a stock solution of 100 µCi/mL of ³H-thymidine (Amersham) was prepared by dilution into high glucose DMEM (GIBCO). ³H-thymidine was added to each well at a final concentration of 1 µCi/mL. The reaction was stopped 15 hours later by removal of the supernatant from recipient cells. The cells were harvested by addition of 100x trypsin/EDTA (GIBCO) to each well for 15 minutes at 37°C. Cells were collected on a filter using a Packard Filtermate Cell Harvester following the manufacturer's protocol and washed in deionized water and methanol. The filters were dried and analyzed using a Matrix 9600 (Packard).

### 6. Alamar Blue Assay

Growth inhibition of cells was also measured using the Alamar Blue Assay. Cells were plated at 10⁴ cells/well in a 96 well plate format. Four days after infection with different MOIs of Ad-mda7 or control vectors (as mentioned earlier), 20 µL of alamar blue dye was added to each well and the plate was incubated at 37°C for 6-8 hours.

The plates were then processed for optical density absorbance analysis using the Dynatech MRX plate reader at dual wavelengths of 575 and 600nm. Revelation 3.2 software program was used to analyze data.

### 7. TUNEL Assay

Cancer cells were seeded in Lab-Tek chamber slides (Nunc) at density of 10³-10⁴ cells/chamber. Cells were transduced with desired concentration of Ad-vectors. At different time-points after infection, cells were analyzed according to manufacturer's instruction for apoptosis-induction using the Chromogenic TUNEL-POD (Boehringer Mannheim) assay. Cells were analyzed 2-5 days post-infection. The kit utilizes a deoxythymidine transferase (TdT) enzyme to incorporate fluorescein bound-deoxythymidine molecules to fragmented DNA with free hydroxyl groups. After washing with PBS, horseradish peroxidase (POD)-tagged-anti-fluorescein antibody is used as the secondary agent, and samples are exposed to DAB to identify TUNEL positive cells (dark brown staining).

### 8. Annexin V Assay

Cancer cells were also analyzed for apoptosis, using the ApoAlert Annexin V-FITC kit (CLONTECH). Ad-vector-transduced cells (10⁵-10⁶ cells total) were washed extensively in PBS and then incubated with Annexin V-FITC reagent for 30 minutes on ice. Cells are then washed and processed for FACS analysis and fluorescent microscopy.

### 9. Hoechst Protein Staining

Hoechst dye (33258) was a product of ICN Biomedicals (Ohio,USA). Briefly, the cells in chamber slides were fixed (methanol:acetic acid = 3:1) for 5 min, and then fixed again with the same fixative for 10 min. They were air-dried for 30 min, and then placed in 1.0 ml staining solution (0.05 mg/ml Hoechst 33258 in 1 x PBS buffer) for 30 min, followed by washing three times (1 min each) with distilled water. After the washes, the slides were air-dried, and photos were taken under a fluorescence microscope or analysed by confocal microscopy.

### 10. DNA Staining with Propidium Iodide (PI)

Cell-cycle staging was done by the evaluation of DNA content by PI staining. For identifying cells at different stages of cell cycle, vector infected cancer cells were prepared as a single cell suspension of 1-2 x 10⁶ cells/mL of PBS. After the cells were fixed with cold 70% ethanol for 2 hours, the cells were centrifuged, the fixative decanted, and cells washed 2x with PBS and then stained with propidium iodide (PI) at final concentration of 50 µg/mL with RNAse at 20 µg/mL in PBS. Treated cells were then analyzed by FACS analysis.

### J. Surface Expression Studies

Cancer cell lines were treated with increasing MOIs (multiplicities of infection) of Ad-mda7 or Ad-luc as control. Briefly, 1 x 10⁶ cells (in 6-well plate) were infected with Ad vectors and 48 h later the cells were dislodged with 500 µL of Versene. The cells were washed 3x with PBS (3 mL per wash) and treated with 500 µL of 1:2000 diluted rabbit anti-mda7 affinity purified antibody (stock conc. 1 mg/mL) at 4°C for 2h. After this treatment, the cells were washed with PBS (3x) and treated with 1:1000 diluted goat anti-rabbit IgG-FITC for 2h at 4°C. Cells were washed, fixed using 4% formaldehyde in PBS and analyzed by FACS analysis.

The Fluorescent Microscopy and Imaging Core Facility at UT Health Science Center was used for the confocal work described in this report. The following center systems were used: Molecular Dynamics 2001 CSLM (Confocal Scanning Laser Microscope), Applied Precision Deltavision Deconvolution Microscope, Nikon 8000 RSCM (Real time scanning Confocal microscope) and Wallac/Olympus Concord system (real time fluorescence imaging system).

For MDA-7 protein staining, cells were infected with Ad-mda7 (or Ad-luc) at an MOI of 1000 vp/cell. After 48 hours, the cells were washed 3x with PBS (3 mL per wash) and treated with 500 µL of 1:2000 diluted rabbit anti-Mda-7 affinity purified antibody (stock conc. 1mg/mL) at 4°C for 2h. After the treatment, the cells were washed with PBS (3x) and treated with 1:1000 diluted goat anti-rabbit IgG-rhodamine. The cells were also stained with Annexin V and Hoechst stain. Cells were fixed post-staining using 4% formaldehyde in PBS.

For intracellular [Ca²⁺] characterization, Ad-mda7 transduced cells were loaded for 15min in the dark at 37°C with the calcium probe FLU03 at final concentration of 2 µM *(*Minta et al. 1989, Molecular Probes, Eugene, OR), then visualized with a Molecular Dynamics scanning laser confocal microscope at wavelength of 488 nm. Areas and volumes of the cells were determined using Image Space software (Molecular Dynamics, Sunnyvale, CA), following optical stack sectioning of the cells. Fast scan image captures were made to visualize calcium waves passing through the Ad-mda7 transduced cancer cells, and these images were then compiled and sequenced.

Mitochondrial content was determined using similar protocols as above (Ca²⁺ experiments), except the probe used was MitoTrack (Molecular Probes). Loading parameters and probe concentration were the same as FLUO 3, with the scans performed at a wavelength setting of 595 nm (Marin et al. 1996).

### K. Glycosylation Analyses

Supernatant was treated with the following three enzymes either individually or in different combinations. The enzymes used were sialidase (neuraminidase), endoglycosidase-H and endoglycosidase-F (all obtained from Sigma). The buffer conditions were 100 mM Tris, pH 8.2. For every microgram of total supernatant protein used, approximately 0.1 units of enzyme/s were used. The enzymes used were Sialidase (Neuraminidase), Endoglycosidase-H and N-glycosidase-F (all obtained from Sigma). The buffer conditions were 100 mM Tris, pH 8.2. The reaction was carried out at 37°C for one hour and then the samples were run on SDS-PAGE and analyzed by Western blot using the specific anti-MDA7 rabbit polyclonal antibody.

### EXAMPLE 3: AD-MDA7 KILLS CANCER CELLS AND INDUCES APOPTOSIS

### 1. Breast Cancer Cells

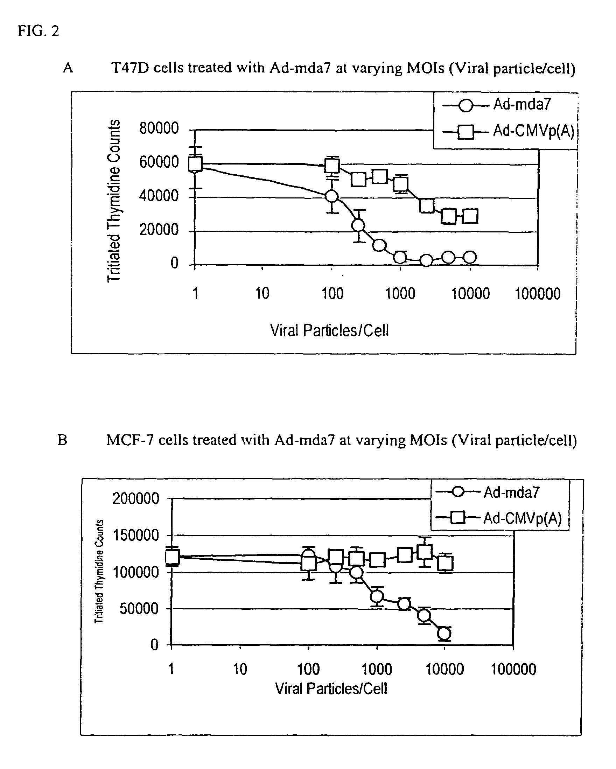

A series of breast cancer cell lines (T47D, MCF-7, BT-20, MDA-MB-361, SKBr3, MDA-MB-231, MDA-MB-468) were transduced with Ad-mda7 (or Ad-CMVp(A) or Ad-luc as control vectors). The cell lines were strongly growth-inhibited by Ad-mda7 transduction. The two cell lines that demonstrated the highest sensitivity to Admda7 were T47D (p53 mutated) and MCF-7 (p53 wild-type) (FIG. 2A and 2B), as determined by ³H-thymidine incorporation assay. Cancer cells were analyzed 3-6 days after Ad-mda7 transduction. See TABLE 7 below.

**TABLE 7: Summary of breast cancer lines used for Ad-mda7 studies.**

| **Cell Line** | **Tumor type** | **p53 status** | **Source** |
|---|---|---|---|
| ***Breast Cancer*** | | | |
| (1) T47D | ductal carcinoma | L194F | ATCC |
| (2) MCF-7 | carcinoma | wt | ATCC |
| (3) MDA-MB-361 | adenocarcinoma | wt | ATCC |
| (4) MDA-MB-231 | adenocarcinoma | R280K | ATCC |
| (5) MDA-MB-468 | adenocarcinoma | R273H | ATCC |
| (6) SKBr-3 | adenocarcinoma | Mut | ATCC |
| (7) BT-20 | carcinoma | Mut | ATCC |
| - | | | |

| ***Normal*** | | | |
|---|---|---|---|
| (1) MJ90 | fibroblast | wt | Smith lab |
| (2) HUVECs | endothelium | wt | Clonetics |
| (3) HMECs | mamm. epithelium | wt | Clonetics |

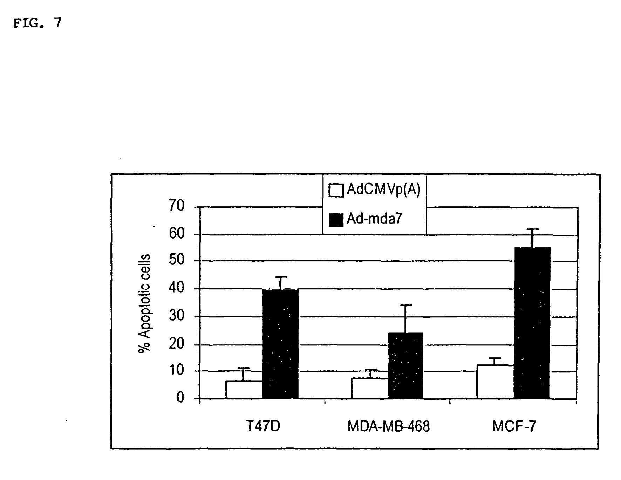

FIG. 7 illustrates the high levels of apoptosis (as measured by Annexin V staining) induced in breast cancer cell lines by Ad-mda-7. Annexin V staining identifies cells in early and mid-stages of apoptosis, whereas the TUNEL assay detects DNA cleavage products, one of the final stages of apoptosis. TUNEL assays performed on MCF-7 cells infected with Ad-mda7 confirmed that these cells are killed via apoptotic pathways. Ad-CMVp(A) or Ad-luc control vectors were ineffective at inducing apoptosis.

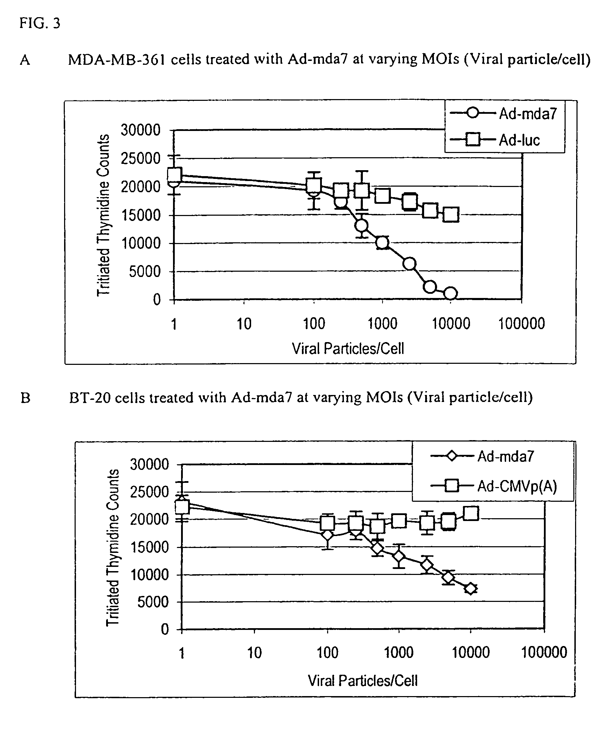

The two cell lines that demonstrated the highest sensitivity to Ad-mda7 were T47D (p53 mutant) and MCF-7 (p53 wild-type) (FIG. 2A and 2B). The Ad-mda7 concentration needed to inhibit growth by 50% (IC₅₀) of the T47D or MCF-7 cells averaged 500 and 1500 vp/cell, respectively (TABLE 8). Also included in FIG. 3 (Panels A and B) are representative experiments using MDA-MB-361 and BT-20 cells. These two cell lines also showed marked sensitivity to Ad-mda7 infection. Table 8 summarizes the responsiveness of breast cancer cells to Ad-mda7 infection (as determined by a comparison of IC₅₀ values for Ad-mda7 and control Ad vector). Also included in Table 8 are the IC₅₀ values in normal cell lines.

**Table 8: Summary of IC₅₀ values of Ad-mda7 in Breast Cancer and Normal lines**

| **Cell Line** | **Tumor type** | **IC₅₀ range** | |
|---|---|---|---|
| | | **Admda-7** | **Control*** |
| ***Breast Cancer*** | | | |
| (1) T47D | ductal carcinoma | 150-500 | > 10,000 |
| (2) MCF-7 | carcinoma | 1200-4000 | > 10,000 |
| (3) MDA-MB-361 | adenocarcinoma | ~1500 | >10.000 |
| (4) MDA-MB-231 | adenocarcinoma | ~3000 | > 10,000 |
| (5) MDA-MB-468 | adenocarcinoma | > 10,000 | > 10,000 |
| (6) SKBr-3 | adenocarcinoma | ~5000 | > 10,000 |
| (7) BT-20 | carcinoma | ~2500 | > 10,000 |
| - | | | |

| ***Normal*** | | | |
|---|---|---|---|
| (8) MJ90 | fibroblasts | > 10,000 | > 10,000 |
| (9) HUVECs | endothelium | >10,000 | >10,000 |
| (10) HMECs | mamm. epithelium | >10,000 | >10,000 |

| | | | |
|---|---|---|---|
| * The control vectors used in these experiments were either Ad-CMVp(A) or Ad-luc. | | | |

### 2. AD-MDA7 KILLS LUNG CANCER CELLS AND INDUCES APOPTOSIS

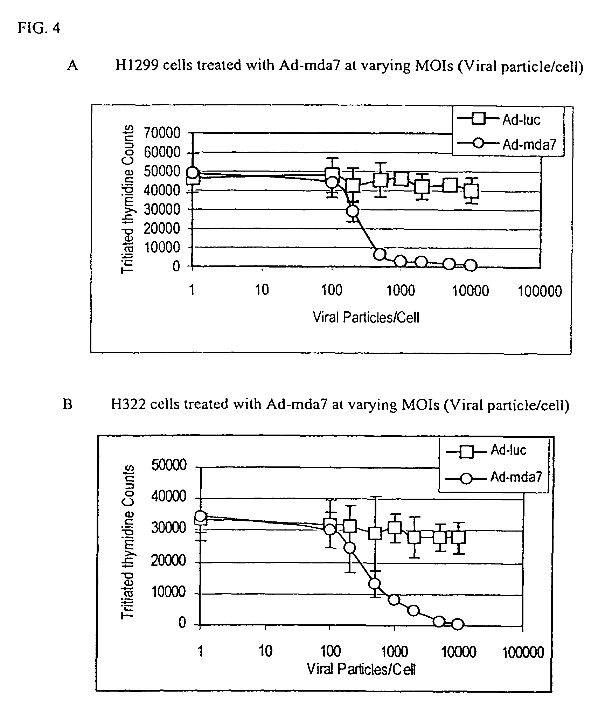

Six lung cancer lines (H1299, H460, A549, H322, H358 and SaosLM2) were infected with Ad-mda7. All of these demonstrated effective killing by Ad-mda7 transduction. The H1299, and H322 cell lines were the most sensitive to Ad-mda7 killing (see FIG. 4A and B). The IC₅₀ in these lines ranged from 600 vp/cell to 2000 vp/cell as determined by ³H-thymidine incorporation assay.

### 3. AD-MDA7 KILLS COLORECTAL CANCER CELLS AND INDUCES APOPTOSIS

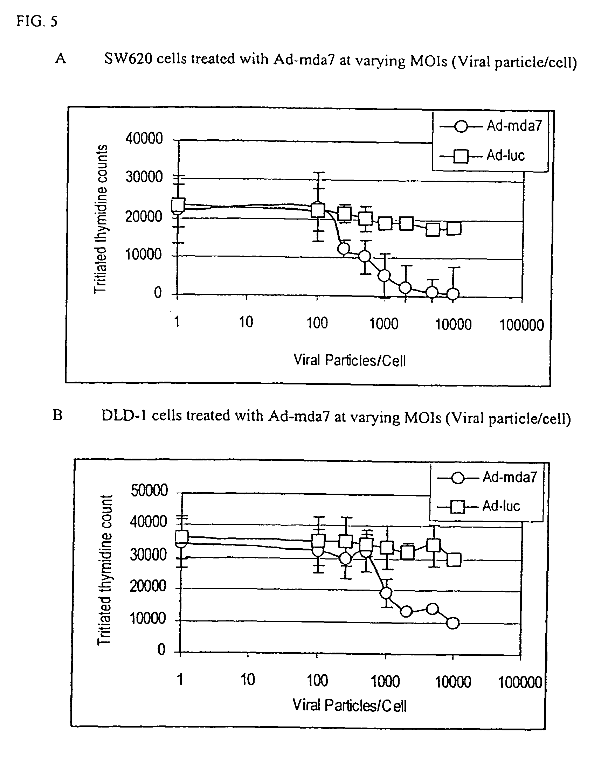

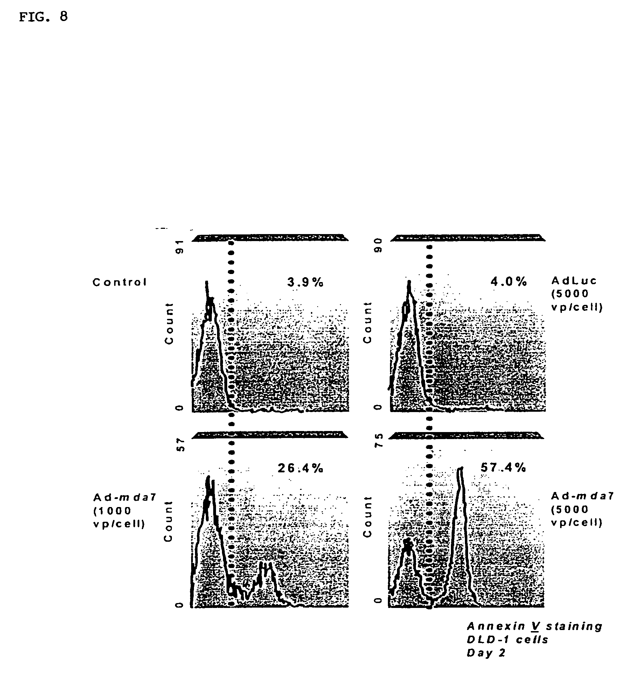

Six colorectal cancer lines (DLD-1, SW-620, SW-480, HT-29, HCT-116, LS174T) were infected with Ad-mda7. All of these cell lines were effectively growth inhibited by Ad-mda7 transduction, with SW620, DLD-1 and SW-480 being the most sensitive. SW620 cells treated with Ad-mda7 at variying MOIs is shown in FIG. 5A. while DLD-1 cells are shown in FIG. 5B. Cell proliferation, as determined by ³H-thymidine incorporation assay, demonstrated an IC₅₀ that averaged 1000 vp/cell in the more sensitive cell lines to 2000 vp/cell in the other less-sensitive cell lines. The DLD-1 cell line was infected with Ad-mda7 at 1000 and 5000 vp/cell, using uninfected cells and Ad-Luc as controls. Forty-eight hours later the transduced cells were analyzed for apoptosis using Annexin V staining in conjunction with FACS analysis. Neither the uninfected or AdLuc-infected (5000 vp/cell) cells showed signs of apoptosis, whereas Ad-mda7 infected cells exhibited approximately 26% apoptotic cells at 1000 vp/cell and 58% apoptotic cells at 5000 vp/cell (FIG. 8).

### 4. AD-MDA7 INFECTION IN NORMAL CELLS

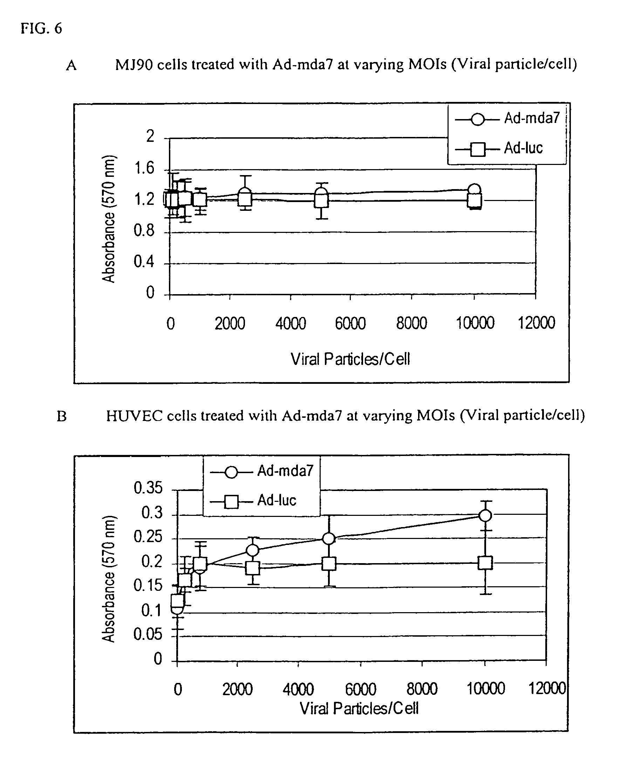

Three normal human cell lines (MJ90 fibroblasts, HUVEC endothelial cells and human mammary epithelial cells) showed no growth inhibition when infected with Ad-mda7. The primary fibroblast cell line MJ90 showed overlapping growth curves when treated with Ad-mda7 or Ad-luc control vector (FIG. 6A). HUVEC and human mammary epithelium cells showed similar results (FIG. 6B).

### 5. PROTEIN ANALYSES

Cell lysates obtained from Ad-mda7 transduced cancer cell lines were size fractionated by SDS-PAGE followed by western-blot analysis using a rabbit anti-MDA7 antibody. The migration of the MDA-7 protein was consistent with an approximate size of 23 kD, however, an additional band at 17 kD was also observed. A Western blot analysis of H1299 (lung cancer) and DLD-1 (colorectal cancer) cell lines was performed after Ad-mda7 and Ad-luc infection. Two bands at approximately 23 and 17 kD were observed. Similar molecular weight size bands were also seen in breast cancer lines infected with Ad-mda7. During the first 48 hours post-infection the 17 kD band was the major species observed in DLD-1 cells. At 72 and 96 hours post infection, the intensity of the 23 kD band decreased with time and other smaller degradation products were seen. In H1299 cells, both bands had similar intensities. The blots were also probed for β-actin, and at 72 and 96 hours post-infection, actin was substantially degraded (data not shown), consistent with the rapid apoptotic death of cells.

As seen in these protein expression studies, lysates from Ad-mda7 infected cells show a 23kD/17 kDa doublet, suggesting that MDA-7 is processed intracellularly. Previous studies by Su *et al.,* 1998, indicated that in human melanoma cells induced with interferon β and mezerin, the 23 D MDA-7 protein translocated from the cytosol to the nucleus. On the basis of primary protein sequence analysis, MDA-7 does not possess any consensus nuclear localization motifs, which may suggest MDA-7 protein associating with a cytoplasmic chaperone (such as HMC) (*Jiang et al.,* 1995*,* 1996). It was proposed that this association may facilitate the translocation of mda-7 into the nucleus.

### 6. APOPTOSIS STUDIES

Annexin V staining identifies cells in early and mid-stages of apoptosis, whereas the TUNEL assay detects DNA cleavage products, one of the final stages of apoptosis. FIG. 7 illustrates the high levels of apoptosis (as measured by Annexin V staining) induced in breast cancer cell lines by Ad-mda7. TUNEL assays were performed on MCF-7 cells infected with Ad-mda7, thus confirming that these cells are killed via apoptotic pathways. Ad-CMVp(A) or Ad-luc control vectors were ineffective at inducing apoptosis.

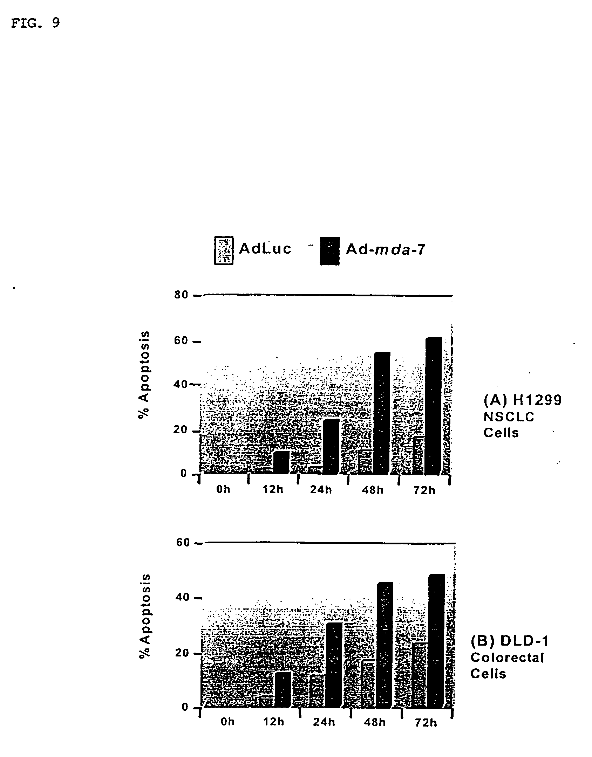

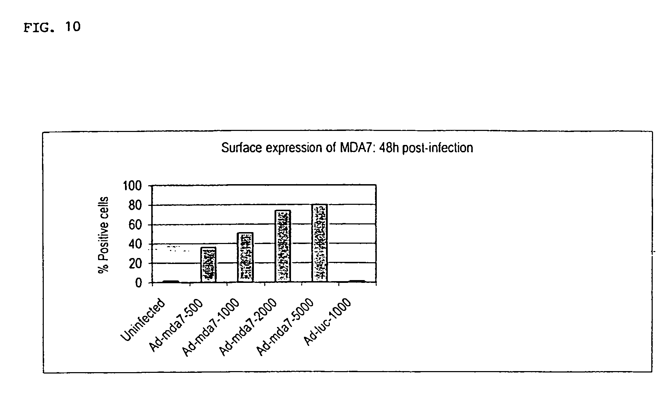

Further examples of Ad-mda7-induced apoptosis are shown in Figures 10 and 11. The DLD-1 cell line was infected with Ad-mda7 at 1000 and 5000 vp/cell, using uninfected cells and Ad-Luc as controls. Forty-eight hours later the transduced cells were analyzed for apoptosis using Annexin V staining in conjunction with FACS analysis (FIG. 8). Neither the uninfected or Ad-Luc infected (5000 vp/cell) cells showed signs of apoptosis, whereas Ad-mda7 infected cells exhibited approximately 26% apoptotic cells at 1000 vp/cell and 58% apoptotic cells at 5000 vp/cell. Ad-mda7 caused rapid induction of apoptosis (FIG. 9). Two cell lines representing NSCLC and colorectal cancer are shown. Substantial levels of apoptosis were evident as soon as 12 hours post-infection with Ad-mda7, and increased over the next few days. The demonstration of apoptosis as soon as 12 hr post-infection is notable as immunoreactive MDA-7 protein is just detectable at 12 hr and, generally, does not peak until 24-48 hr post-infection. Ad-p53 can also cause rapid induction of apoptosis, however, other tumor suppressors, such as p16 or PTEN tend to cause apoptosis only after 2-3 days post infection with the Ad expression vector.

### 7. AD-MDA7 INCREASES BAX PROTEIN LEVELS IN LUNG, BREAST AND COLORECTAL CANCER LINES

Regulation of programmed cell death relies on the interaction between signaling pathways that either promote or inhibit apoptosis (Reed 1997; White 1996). The bcl-2 family members (bcl-2, bcl-w, bax, bad, bak, bcl-xs) play an important role in apoptotic signaling (Sedlak *et* al.1995; *Reed et* al.1996). Using Western blot analysis in conjunction with an anti-bax antibody it was determined that Ad-mda7 infection upregulated the BAX protein in T47D, DLD-1, A549 and H460 cells. Western blot analysis of lysates prepared 24 hours after infection with 30 to 150 pfu/cell of Ad-mda7 demonstrated increased expression of BAX in all cell lines tested. For example, upregulation of BAX in Ad-mda7 infected T47D cancer cell line was observed by Western blot analysis. Cells were infected with Ad-mda7 and analyzed for MDA-7 and BAX protein expression. Ad-mda7 increased BAX expression in T47D, as was observed with the other cell lines.

### 8. ENDOGENOUS EXPRESSION OF MDA-7 IN CANCER AND NORMAL CELLS

Of the more than 50 tumor cell lines evaluated for endogenous Mda-7 protein expression, only two, DLD-1 (colorectal) and LnCap (prostate) were positive. Studies are underway to look at mda-7 mRNA in the various cancer lines. Table 9 is a list of some of the cancer lines used in the Ad-mda7 studies and their endogenous MDA-7 status. There was no correlation between the anti-tumor activity of Ad-mda7 and MDA-7 endogenous expression based on Western blot analysis (TABLE 9).

| **TABLE 9** | | |
|---|---|---|
| **Cell Type** | **Endogenous MDA-7 protein** | **Ad-mda7 killing** |
| (A)Normal lines | | |
| (1) MJ90 | -- | -- |
| (2) HUVEC | -- | -- |
| (3) HMEC | -- | -- |

| (B) Breast cancer lines | | |
|---|---|---|
| (1) T47D | -- | ++++ |
| (2) MCF-7 | -- | +++ |
| (3) MDA-MB231 | -- | ++ |
| (4) MDA-MB468 | -- | ++ |

| (C) Lung cancer lines | | |
|---|---|---|
| (1) H1299 | -- | ++++ |
| (2) A549 | -- | ++ |
| (3) H460 | -- | ++ |

| (D) Colorectal cancer lines | | |
|---|---|---|
| (1) DLD-1 | ++ | +++ |
| (2) SW620 | -- | +++ |
| (3) HCT 116 | -- | ++ |
| (4) HT29 | -- | ++ |

| (E) Prostate cancer lines | | |
|---|---|---|
| (1) LnCap | ++ | +++ |
| (2) Du145 | -- | ++ |

| | | |
|---|---|---|
| Note: -- denotes undetectable endogenous protein/no response to Ad-mda7 infection; ++ denotes presence of endogenous mda7 protein or effective responsiveness to Ad-mda7. | | |

### 9. AD-MDA7 FUNCTIONS INDEPENDENTLY OF ENDOGENOUS P53, RB, RAS, AND P16 STATUS

Table 10 presents the status of different tumor suppressor/oncogene/cell cycle regulating genes and their response to Ad-mda7 infection in different cell lines used in this study. The growth-inhibitory action of MDA-7 was observed in a wide variety of cancer cell lines, independent of their p53, RB, p16, and Ras status. Although, Bax expression is positively regulated by wild-type p53 (Han *et al.,* 1996), the ability of MDA-7 to induce BAX appears to be independent of p53 since BAX up-regulation is observed in p53-mutant (DLD-1, T47D) and p53-wild-type (H460). It is interesting to note that MDA-7 was able to effectively induce apoptosis in the MCF-7 breast cancer cells that are devoid of caspase 3, one of the several caspases involved in the downstream apoptotic events.

| **TABLE 10** | | | | |
|---|---|---|---|---|
| **Cell Type p16** | **Ad-mda7 effect** | **p53** | **RB** | **ras** |
| (A)Normal lines | | | | |
| (1) Melanocytes | ND | wt | wt | wt |
| wt | | | | |
| (2) MJ90 fibroblasts | -- | wt | wt | wt |
| wt | | | | |
| (3) HUVEC | -- | wt | wt | wt |
| wt | | | | |
| (4) HMEC | -- | wt | wt | wt |
| wt | | | | |

| (B) Cancer Lines | | | | |
|---|---|---|---|---|
| (1) T47D | ++++ | mut | -- | wt |
| -- | | | | |
| (2) MCF-7 | +++ | wt | -- | wt |
| -- | | | | |
| (3) H1299 | ++++ | null | wt | mut |
| -- | | | | |
| (4) Saos-LM2 | ++ | del | -- | wt |
| -- | | | | |
| del | | | | |
| (5) A549 | ++ | wt | -- | mut |
| -- | | | | |
| (6) H460 | ++ | wt | wt | mut |
| del | | | | |
| (7) SW620 | +++ | mut | -- | mut |
| -- | | | | |
| (8) HCTI 16 | ++ | wt | -- | mut |
| -- | | | | |
| Note: ND, Not determined; mut, mutation; del, deletion; wt, wild-type | | | | |

### EXAMPLE 4: MDA-7 CELLULAR LOCALIZATION STUDIES

### 1. SURFACE EXPRESSION STUDIES

The H460 NSCLC cell line was treated with increasing MOIs of Ad-mda7 or Ad-luc as control, and 48 h later, the cells were stained with the polyclonal anti-MDA-7 antibody and analyzed by FACS analysis (FIG. 10). A high level of staining was observed in the Ad-mda7 treated cells only. The staining was dose-dependent and approximately 50% of cells were MDA-7 positive at 1000 vp/ cell. This result indicated that Ad-mda7 treatment of H460 cells resulted in high levels of protein production (verified by Western blot analysis) and that the protein appeared to on the cell surface.

### B. Confocal Microscopy Studies

To confirm and extend the results shown in FIG. 10, confocal microscopic analyses were performed on various cell lines (H460, H1299, T47D and DLD-1 cells) to determine sub-cellular distribution MDA-7 protein after Ad-mda7 treatment. Background staining in untreated or Ad-luc-treated cells was low and diffuse. The background is believed to be due to the anti-MDA-7 reagent being a polyclonal antiserum. However, highly specific staining was observed when cells were treated with Ad-mda7. At low MOIs, distinct membrane staining was observed with punctate staining in the cytoplasm. At higher MOIs, the punctate staining and membrane staining were reproduced and more intense. The pattern of staining was suggestive of a secreted protein, with the punctate staining representing protein trafficking and release at the plasma membrane. Similar observations were observed in the other cell lines

In additional confocal microscopy experiments, cancer cell lines were treated with Ad-mda7 and analyzed for apoptosis (Annexin V staining), DNA content (Hoechst), Ca2+ influx/eflux (Fluo 3, Molecular Probes) and mitochondrial integrity (MitoTrack, Molecular Probes). The protocols used were those established in the Confocal Microscope Facility, UTHSC, Houston, TX.

Confocal microscopic studies of H460 and MCF-7 cells were done. They show a composite of individual microscopic fields: (1) denotes surface expression of MDA-7 (red surface and punctate staining), (2) showing apoptosing cells (polarized green staining), (3) Hoechst staining to identify nuclei (blue) and (4) composite of (1) (2) and (3).

Calcium and mitochondrial staining was done in Ad-mda7- or Ad-luc control-transduced cells. Cells were plated on laminin-coated cover-slips and treated with FLUO-3 (for Ca²⁺) or with Mitotracker (for mitochondria). The control Ad-luc treated cells show a well distributed intracellular calcium content (green fluorescence) and displayed good mitochondrial integrity (red staining). However, on Ad-mda7 treatment, intracellular Ca²⁺ levels are disrupted and the mitochondrial integrity is disrupted.

### EXAMPLE 5: SECRETED MDA-7 PROTEIN

### 1. Secretion of MDA-7

H1299 cells were infected with Ad-mda7 (MOI of 1000 vp/cell) for 6 hours, washed with fresh media and incubated at 37°C in fresh DMEM media. Twenty-four hours later, the cell lysate and the growth media were analyzed for MDA-7 protein expression using Western blot. Ad-mda7 transduced cells showed a specific 40 kD protein produced in growth media, which was absent in untransduced or Ad-luc transduced cells that only showed 19 kD and 23 kD bands. A dose-dependent increase in the intra-cellular MDA-7 and the extra-cellular MDA-7 protein was observed. As a control, the blot in Panel B was probed with an anti-actin antibody. As predicted, the cell lysates showed an actin signal at approx. 40 kD, whereas the cell supernatants did not show any actin signal. This suggests that the MDA-7 protein signal observed in the supernatants is due to active release/ secretion of MDA-7 and is not due to release from dying cells.

### 2. Glycosylation of Secreted MDA-7 Protein

The supernatant from Ad-mda7 transduced H1299 cells was a good source of obtaining the secreted MDA-7 protein. The supernatant was further evaluated for protein glycosylation. Supernantant was treated with the following three enzymes either individually or in different combinations. The enzymes used were sialidase (neuraminidase), endoglycosidase-H and endoglycosidase-F (all obtained from Sigma). The samples were analyzed by Western blot using the specific anti-MDA-7 rabbit polyclonal antibody.

Endoglycosidase treatment suggests that soluble MDA-7 protein is glycosylated. Using various glycosidases, especially Endo F, a lower molecular weight band is also observed (which is approximately the same size as the MDA-7 protein band observed in cell lysate.

### 3. Inhibition of Glycosylation and Secretion of MDA-7 Protein

Two antibiotics, Tunicamycin and Brefeldin A, have been used to provide a more detailed characterization of the secretion of soluble MDA-7. N-linked glycosylation plays an important role in a protein's ultimate processing, whether it is sorted to a lysosomal pathway, or translocated to the cell surface or secreted. Using Tunicamycin, the N-linked glycosylation process in the golgi apparatus can be inhibited, thus inhibiting protein secretion or other sugar-dependent sorting processes. Brefeldin A is a fungal metabolite (macrocyclic lactone) which exhibits a wide variety of antibiotic activities. Brefeldin A reversibly inhibits the intracellular translocation of proteins (during transport of protein to the cell surface for secretion or expression. Both Tunicamycin and Brefeldin A effectively inhibit the secretion of soluble MDA-7 protein. Therefore, intracellular processing and glycosylation appear to be required for MDA-7 secretion.

### 4. Secreted MDA-7 Protein Induces Killing in Cancer Cells

The secreted form of MDA-7 (sMDA-7) was produced using various cell lines and evaluated for anti-tumor activity. A representative experiment is shown in FIG. 11. Soluble MDA-7 was analyzed for its anti-proliferative effects on H1299 cells. Briefly, H1299 cells were plated at cell density of 10³ cells/chamber in Nunc chamber slides. 24 hours later, the cells were challenged with supernatants obtained from H1299 cells transduced with either Ad-mda-7 or Ad-luc (at 1000 vp/cell infection). Ad-mda7 and Ad-luc viruses were also used as additional controls. The soluble protein supernatants (500 uL total volume, different dilutions) were applied to naïve H1299 cells and 24 hours later an additional 0.5mL of 10% FBS in DMEM was added. After 24 and 48 hours, the cells were microscopically examined for viability using the trypan blue exclusion staining. The soluble MDA-7 protein showed H1299 killing after 48 hours; however, Ad-luc supernatants had little effect (FIG. 11A).

Various dilutions of soluble MDA-7 supernatant were also analyzed for H1299 killing using the Trypan blue exclusion assay. A concentration-dependent bystander killing effect of soluble MDA-7 was observed (FIG. 11B).

### EXAMPLE 6: COMBINATION STUDIES OF AD-MDA7 IN BREAST CANCER LINES

### 1. Combination with Tamoxifen

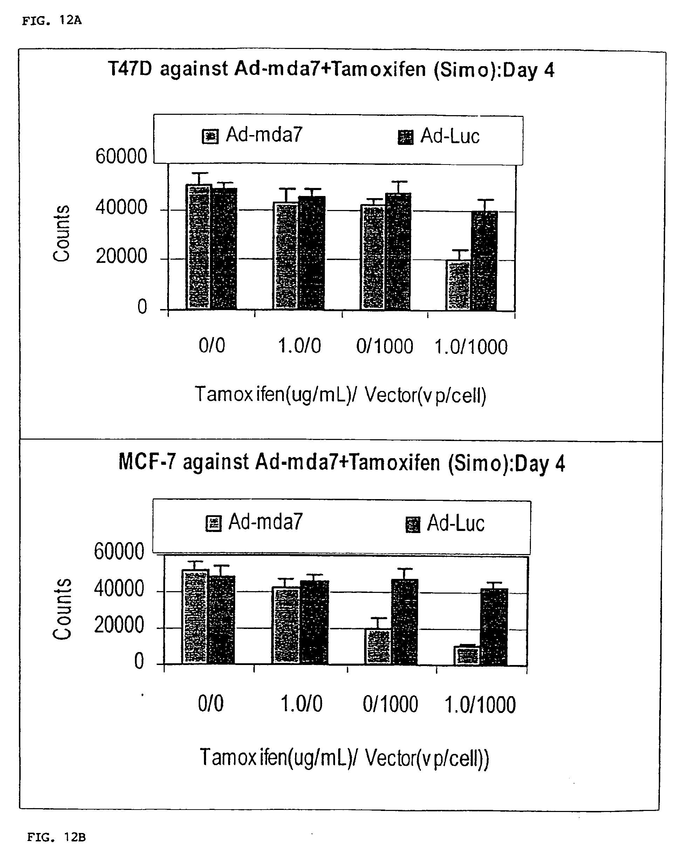

Ad-mda7 has been combined with tamoxifen and evaluated for anti-tumor effects in breast cancer cell lines (FIG. 12). The graphs demonstrate that combining these two agents provides superior anti-tumor activity compared to either agent alone. The effect of tamoxifen on T47D cells is shown (FIG. 12A) and on MCF-7 cells (FIG. 12B). Cells were plated and four days after treatment, a tritiated thymidine assay was performed to measure DNA replication. Cells were treated with 0/0 (no drug and no vector) or varying doses of tamoxifen or vectors (Ad-luc or Ad-mda7). In T47D cells, tamoxifen or Ad-mda7 had minimal effect on DNA replication. However, when the tamoxifen and Ad-mda7 were combined, a supra-additive effect was observed. In MCF-7 cells, tamoxifen had little effect at Ing/ml dose. Ad-mda7 reduced signal compared to Ad-luc. However when tamoxifen was combined with Ad-mda7, a supra-additive effect was observed, again demonstrating the enhanced effects of combining a chemotherapeutic agent with Ad-mda7.

### 2. Combination with Adriamycin

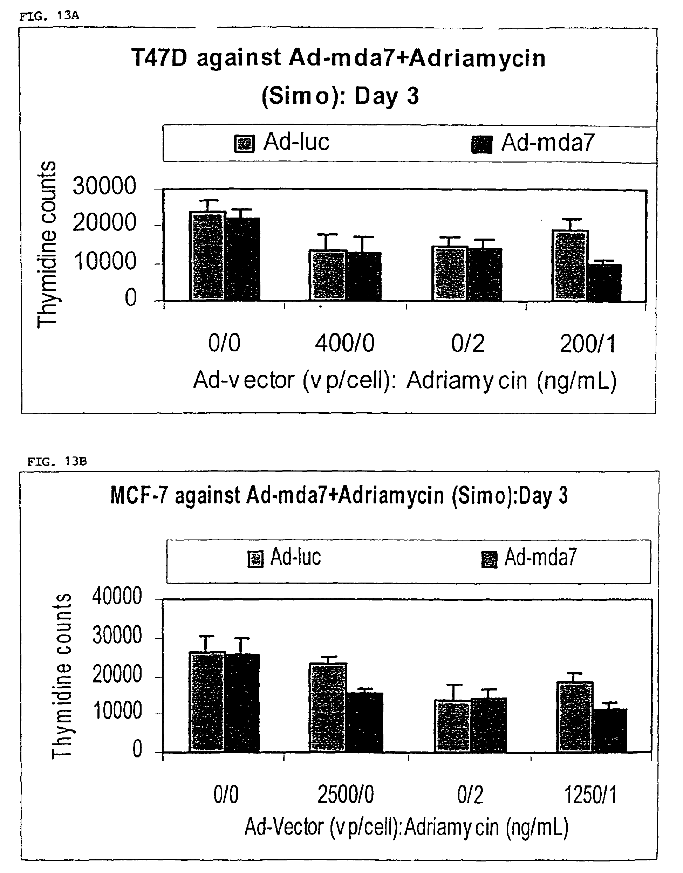

Ad-mda7 has been combined with adriamycin and evaluated for anti-tumor effects in breast cancer cell lines (FIG. 13). The graphs demonstrate that combining these two agents provides superior anti-tumor activity compared to either agent alone.

### EXAMPLE 7: ACTIVATION OF CASPASE CASCADE BY AD-MDA7

### 1. Material and Methods

### a. Cell Culture

Human non-small cell lung carcinoma cells A549, H460, H1299, human prostate cancer cells DU145, and human breast cancer cells MCF-7 were obtained from the American Type Culture Collection (ATCC, Bethesda, MD). All cells were maintained in DMEM medium containing 10% of Fetal Bovine Serum, antibiotics and L-glutamine. Normal human bronchial epithelium cells (NHBE cells) were obtained from Clonetics Inc (Clonetics Inc., Walkersville, MD) and maintained according to the manufacturer's instructions.

The cells were verified to be free of mycoplasma and used in the log phase of growth. Cells were routinely harvested with 0.125% Trypsin -1.3 mM EDTA (GIBCO).

### b. Construction of Recombinant Adenoviral Vector

Same as described above.

### c. Determination of Cell Growth Rate

Cancer or normal cell lines used in this study were plated in 12-well dishes with 2 x 10⁴ cells in each well. Cells were infected with Ad-mda7, with Ad-Luc controls (5000 viral particles/cell), or with PBS as an additional control. Cells were harvested by trypsinization, diluted with trypan blue (GIBCO) and the numbers of viable cells were counted on a hemocytometer. In addition, inhibition of cell growth was assayed by XTT assay as per the manufacturer's guidelines (Cell Proliferation Detection Kit II, Roche) or by H³-thymidine assay.

### d. Cell Cycle Analysis

Fluorescence-activated cell sorter analysis was performed as follows: cells (5x10⁵/plate) were seeded on 10cm plates and infected with PBS, Ad-mda7 or Ad-Luc at 5000 vp/cell. Cells were harvested by trypsinization at designated times (24, 48, 72 hrs after infection) and washed twice with PBS. Cells were fixed with 70 % ethanol, washed with PBS twice and resusupended with 500 µl of PI solution (5 µg/ml PI and 10µg/ml RNase). Cells were analyzed using a FASCscan analyzer.

### e. Detection of Apoptosis

Tumor cells were seeded in chamber slides (Falcon) at a density of 1 x 10⁵ cells/chamber. Cells were transduced with Ad-mda7 or Ad-Luc vectors. At different days post-infection, cells were analyzed for apoptosis by Hoechst 33342 staining (Boehringer Mannheim) and terminal deoxynucleotidyl transferase-mediated biotinylated UTP nick end labeling (TUNEL) staining with Terminal Transferase (Boehringer Mannheim).

### f. Immunohistochemical Staining

Immunohistochemical staining was carried out on virus infected cells to determine MDA-7 protein expression. Briefly, cells (H1299, A549, H460, and NHBE) were plated at a density of 1x10⁵ in chamber slides (Falcon) and infected with Ad-mda7 or Ad-Luc (5000 viral particles/cell). 48 hrs later, cells were washed with PBS and fixed in 4% formalin solution for two minutes. After blocking of endogenous peroxidase activity with 0.3% H₂O₂ in methanol for 30 minutes, cells were incubated with normal goat serum for 30 minutes at room temperature. Following incubation, slides were treated with rabbit polyclonal anti-MDA-7 antibody (1: 5000 dilution) for 60 minutes. After 30 minutes incubation with anti-rabbit secondary antibody (provided with ABC kit, Vector) expression of MDA-7 in cells was detected with DAB by enhancement with avidin-biotin reaction ABC kit. The slides were counterstained with hematoxylin and then mounted with Aqua-mount (Lerner Labs, Pittsburgh, PA). Negative controls included cells uninfected but subjected through all staining proceeded.

### g. Western Blotting Analysis

Cells were harvested by trypsinization, washed with PBS and resuspended in 100µl of lysis buffer (62.5mM Tris-Hcl, 2% SDS, 10% glycerol, 4M Urea). Cell extracts were homogenized with sonicator for 30 sec and after an hour incubation on ice, cell extracts were spun for 5min at 14000 rpm at 4 °C. Cell extracts were collected and stored in -70°C. Protein concentrations of all extracts were determined using the Bio-Rad protein determination kit (Bio-Rad). Each of 50pg protein samples were diluted into 20µl with lysis buffer and 5% of 2-Mercaptoethanol (Bio-Rad) and heated in a water bath at 95°C for 5min. Then protein extracts were separated on a 10% SDS-PAGE gel in a vertical slab gel electrophoresis cell (Bio-Rad). Proteins were transferred from gel to nitrocellulose membrane (Hybond-ECL membranes, Amarsham International. Little Chalfont, England). Proteins were blocked in a blocking solution (5% dry milk and 0.3% Tween 20 in PBS) for one hour at room temperature. Membranes were incubated with primary antibody and then horse raddish peroxidase labeled secondary antibodies followed by application of Enhanced Chemiluminescence Western Blotting Detection System (Amersham) for 30 seconds. Proteins were visualized on Amersham Hyperfilm enhanced chemiluminescence film using exposure time varying 30 seconds to 30minutes.

### 2. Inhibition of Cell Proliferation by Overexpression of MDA-7

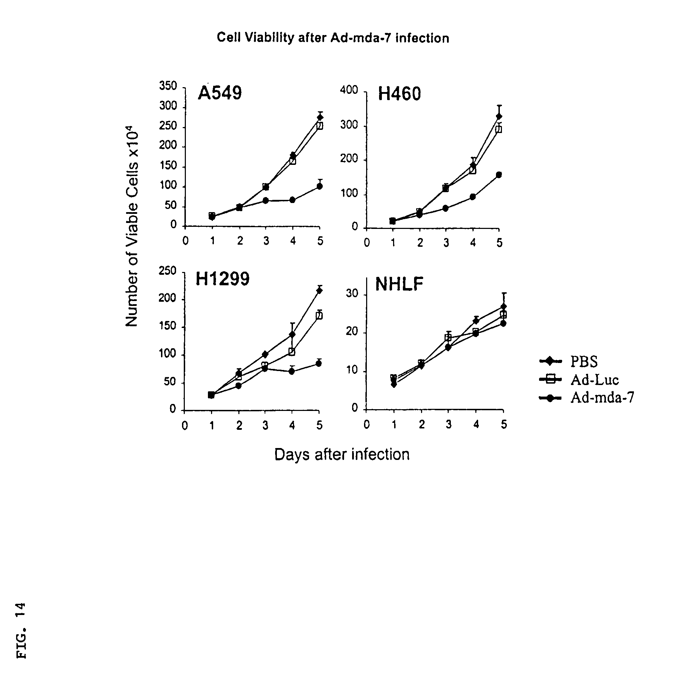

To detect MDA-7 expression in cells, A549, H1299, H460, and NHBE cells were infected with 5000vp/cell of Ad-mda7. Forty-eight hours later cells were fixed and stained with anti-MDA-7 antibody. Uninfected cells were stained with the same antibody as controls. High level of MDA-7 expression was observed in cytoplasm of cells, while no stained cells were seen in uninfected controls (FIG. 14).

A549, H1299, H460, and NHBE cells were prepared in 12 well plates and treated with Ad-mda7. Ad-Luc, or PBS. The numbers of viable cells were counted from day 1 to day 5 after treatment. Infection with Ad-mda7 significantly suppressed cell proliferation in all the tumor cell lines as compare to PBS or Ad-Luc controls (Fig 23).

Cell cycle analysis using PI staining showed a G2/M cell cycle arrest in Ad-mda7-infected A549 and H1299 cells. In contrast, PBS and Ad-Luc infection did not affect the cell cycle (FIG. 14).

Following Ad-mda7 infection, morphological changeswere observed in tumor cells. These changes, such as flattenning and enlargement were observed in all of infected cell lines. Apoptotic morphological changes were visualized using Hoechst 33342. 72 hours after infection of Ad-mda7 or Ad-Luc, nuclear condensation and fragmentation were observed in Ad-mda7 infected A549, H1299, and H460 cells, while apoptotic alterations were not seen in NHBE cells. TUNEL staining demonstrated many positive cells in Ad-mda7 infected A549 cells, while very few positive cells were seen in NHBE cells. TUNEL positive cells were also very rare in Ad-luc treated samples.

These results showed significant supression of cell proliferation with concomitant G2/M cell cycle arrest and induction of apoptosis in lung cancer cell lines. In contrast, in NHBE cells overexpression of MDA-7 resulted in minimal suppression of cell proliferation, but did not induce apoptosis.

### 3. Upregulation of p53 and Bax in Cells with Wild type p53

Cells were infected with Ad-mda7 and Ad-Luc, and cell extracts were harvested at 24, 48, and 72 hours after infection for Western blot analysis. Cell extracts from untreated cell were harvested as a control. MDA-7 protein expression was detected in all of the Ad-mda7-infected cancer cell lines. Untreated controls and Ad-Luc-infected cells did not show any expression of MDA-7 protein. Upregulation of p53 protein was seen in p53 wild type A549 and H460 cells after Ad-mda7 infection. As predicted, no expression or modulation of p53 was seen in p53-deleted H1299 cells. An increase in BAX protein levels was demonstrated in A549 and H460 cells (p53 wild-type), while no change was observed in H1299 (p53-null) cells. The expression level of Bcl-2 was not changed in all of the three cell lines analyzed. In the Bax-deficient, human prostate cancer cell line DU 145, p53 expression levels were not changed and BAX was not detected. However, DU-145 cells were sensitive to Ad-mda7 infection and displayed growth arrest and apoptosis. p53 and bax are up-regulated by Ad-mda7 in p53 wild type tumor cells. In addition, caspases 3 and 9 and PARP are activated by Ad-mda7. Normal cells do not exhibit alterations in apoptotic mediators.

### 4. Activation of Caspase Cascade and Cleavage of PARP

Western blots demonstrated activation of the caspase cascade by Ad-mda7 infection (FIG. 14B). The proforms of caspase-9 and caspase-3 were cleaved and converted to the activated/ cleaved forms 48 hrs after Ad-mda7 infection in A549 and H460 cells and after 72hrs in H1299 cells. Cleavage of caspase-8 was demonstrated after 48 hrs of Ad-mda7 infection in A549 and H460 cells. Poly (ADP-ribose) polymerase (PARP) was cleaved in A549 and H460 cells after 48 hrs in H1299 cells. In Bax-deficient DU145 cells, caspase-9 and caspase-3 were cleaved after 72 hrs of Ad-mda7 infection.

### EXAMPLE 8: IN VIVO EFFECTS OF AD-MDA7

### 1. Materials and Methods

### A. Cell culture

Human non-small cell lung carcinoma cells A549 and H1299 were obtained from the American Type Culture Collection (ATCC, Bethesda, MD). All cells were maintained in RPMI1640 medium containing 10% of Fetal Bovine Serum, antibiotics and L-glutamine. Prior to start of the experiments, the cells were verified to be free of mycoplasma and used in the log phase of growth. Cells were routinely harvested with 0.125% Trypsin -1.3 mM EDTA (GIBCO).

### B. Construction of recombinant adenoviral vector

Replication-deficient human type 5 Adenoviral vectors (Ad5) carrying the mda-7 or Luc genes linked to an internal CMV-IE promoter and followed by SV40 polyadenylation (pA) signal have been constructed and will be referred to as Ad-mda7 and Ad-luc, respectively. Viruses were propagated in 293 cells and purified by chromatography.

### C. Apoptotic cell staining

Sections were stained for apoptotic cell death using the terminal deoxynucleotide transferase (Tdt) (Boehringer Mannheim) kit and counterstained with methylene blue or methlene green as described (Fujiwara *et al.,* 1994).

### D. Western blotting analysis

Western blotting was performed as described above. Cells were harvested by trypsinization, washed with PBS and resuspended in 100 µl of lysis buffer (62.5 mM Tris-Hcl, 2% SDS, 10% glycerol, 4 M Urea). Cell extracts were homogenized with sonicator for 30sec and after an hour incubation on ice, cell extracts were spun for 5min at 14000 rpm at 4 °C. Cell extracts were collected and stored in -70°C. Protein concentrations of all extracts were determined using the Bio-Rad protein determination kit (Bio-Rad). Each of 50 µg protein samples were diluted into 20 µl with lysis buffer and 5% of 2-Mercapto Ethanol (Bio-Rad), and heated in a water bath at 95°C for 5min. Then protein extracts were separated on a 10% SDS-PAGE gel in a dual vertical slab gel electrophoresis cell (Bio-Rad).

Proteins were transferred from gel to nitrocellulose membrane (Hybond-ECL membranes). Proteins were blocked in a blocking solution (5% dry milk and 0.3% Tween 20 in PBS) for 1 hour at room temperature. Then membranes were incubated with primary antibody. Horse raddish peroxidase labeled secondary antibodies were applied and Enhanced chemiluminescence Western Blotting detection system (Amersham) was applied for 30 second and proteins were then visualizen on Amersham Hyperfilm enhanced chemiluminescence film using exposure time varying 30sec to 30min.

### E. Evaluation of tumor growth and treatments in vivo

Prior to the start of all experiments involving subcutaneous tumor growth and treatments, *nu*/*nu* mice were irradiated (3.5 Gy) using a cesium source to enhance tumor uptake. In all the experiments, 5x10⁶ tumor cells (H1299, A549) suspended in 100 µl sterile phosphate buffered saline (PBS) were injected into the right dorsal flank. When the tumor had reached a size of 50-100mm³, animals were randomized into three groups (n = 8 animals/group) and treatment initiated as follows. Group 1 received no treatment, Group 2 received Ad-Luc (5x109 vp / dose) and Group 3 received Ad-mda-7 (5x109 vp / dose) every alternate day for a total of three doses. Intratumoral injections were performed under anesthesia using methoxyflurane (Schering Plough, Kenilworth, NJ) as per institutional guidelines. Tumor measurements were recorded every other day without knowledge of the treatment groups, and the volume was calculated using the formula V (mm3) = a x b² /2, where "a" is the largest dimension and "b" is the perpendicular diameter. Antitumor efficacy data are presented as cumulative tumor volumes for all animals in each group to account for both size and number of tumors.

### F. Immunohistochemical analysis

Tumors established subcutaneously in nude mice were obtained and fixed in 10% buffered formalin, paraffin embedded and cut as 4 µm thick sections. Sections were stained for mda-7 gene expression. Briefly, tissue sections were treated with 0.3% H₂O₂ in methanol for 30 minutes to block endogenous peroxidase activity and were subsequently incubated with normal goat serum for 30 minutes at room temperature. Following incubation, slides were treated with rabbit polyclonal anti-MDA-7 antibody (1: 5000 dilution) for 60 minutes. After 30 minutes incubation with anti-rabbit secondary antibody (provided with ABC kit, Vector) protein expression of MDA-7 in tissues were detected with DAB by enhancement with avidin-biotin reaction ABC kit. The slides will be counterstained with hematoxylin and then mounted with Aqua-mount (Lerner Labs., Pittsburgh, PA). The number of tumor cells staining positive for MDA-7 were analyzed under bright field microscopy and quantitated in a blind fashion using image analysis and statpro software. A total of at least five fields per specimen were analyzed.

### G. TUNEL staining

Tissue sections obtained from subcutaneous tumors were stained for apoptotic cell death using the terminal deoxynucleotide transferase kit (Tdt) (Boehringer Mannheim). In all the staining procedures, appropriate negative controls were included. The number of tumor cells staining TUNEL positive were analyzed under bright field microscopy and quantitated in a blind fashion using image analysis and statpro software. A total of at least five fields per specimen were analyzed.

### H. Statistical analysis

The statistical significance of the experimental results was calculated using Student's *t*-test for tumor measurements.

### 2. In vivo suppression of local tumor growth by Ad-mda7

The therapeutic effect of the mda-7 gene on H1299 and A549 subcutaneous tumors was evaluated in nude mice. Mice bearing each tumor cell type (H1299 and A549) were divided into three groups, one receiving no treatment, one treatment with Ad-Luc, and one treatment with the Ad-mda-7 daily for a total of three doses (5 x 10⁹ viral particles/dose). A significant growth inhibition of H1299 tumors and A549 tumors was observed in mice treated with the Ad-mda-7 compared with the tumor growth in the two control groups for each tumor type.

Further evidence that the observed therapeutic effect was due to mda-7 gene expression was obtained by removing subcutaneous tumors 48 hours after injection and analyzing them by immunohistochemistry. mda-7 gene expression was observed in tumor cells in animals receiving the Ad-mda7, as compared to no positive staining in control tumors that were either not treated or treated with Ad-Luc.

MDA-7 gene expression *in situ* results in apoptotic cell death through caspase-3 and Apo2/TRAIL activation. To understand the mechanism of tumor inhibition mediated by mda-7, subcutaneous tumors harvested at 48 hours following the last treatment were analyzed for apoptotic tumor cell death by TUNEL staining. Tumors from control mice that were either untreated or treated with Ad-Luc showed minimal apoptotic cell death while tumors from animals treated with Ad-mda-7 demonstrated extensive apoptosis.

Since apoptosis is mediated by activation of caspases, tumor tissues were examined for caspase-3, a downstream caspase. Activated form of caspase-3 was observed in tissues treated with Ad-mda-7 while no caspase-3 activation was observed in the tissues from control mice. Similarly, activation of Apo2/TRAIL was observed in tumors expressing mda-7. In contrast, TRAIL expression was not observed in tumors that were not treated or treated with Ad-luc.

### 3. MDA-7 Expression Results in Upregulation of Costimulatory Molecules

The ability of dying tumor *cells in situ* to activate costimulatory molecules, B7 and ICAM, was investigated. Subcutaneous tumors injected with Ad-MDA7 or Ad-Luc were harvested 48 hrs following the last dose and analyzed by immunohistochemistry. Expression of B7 (7.1 and 7.2) and ICAM was observed in tumors expressing MDA-7 while no expression was observed in tumors treated with Ad-Luc.

### 4. Expression of MDA-7 in in situ Tumor Inhibits Angiogenesis

To further determine the tumor suppressive effects of mda-7, subcutaneous tumors were analyzed for CD31 expression, a marker frequently used to identify angiogenesis in tumors. Subcutaneous tumors treated with Ad-mda-7 demonstrated fewer numbers of blood vessels when compared to tumors treated with Ad-luc or no treatment groups.

### EXAMPLE 9: EFFICACY OF AD-MDA7 TO PREVENT METASTATIC SPREAD OF TUMOR

Experiments have demonstrated that Ad-mda7 can inhibit metastatic spread of lung cancer tumors *in vivo.* Further experiments will be performed using melanoma cell lines to evaluate the ability of MDA-7 to prevent the metastatic spread of melanoma tumors. Techniques and protocols discussed previously will be employed.

Human melanoma xenografts will be established by subcutaneous injection of human melanoma cells (1 x 10⁶ cells) into the flanks of nude mice. TXM-1 or TXM-18 cells may be used. Once the tumor reaches 5 mm mean diameter, increasing doses of Ad-mda7 or control Ad-luc will be injected into the tumors. Doses of 3 x 10⁷ to 3 x 10⁹ pfu will be tried. Adenoviral vector will be delivered in three injections of approximately 33 ml, total 100 ml, intralesionally. Each injection will be orthogonally oriented to the preceding injection to ensure efficient tumor coverage. After establishment of the appropriate dose, tumor xenografts will be treated with a single 100 ml dose or multiple fractional doses equaling 100 ml over a three day time period to assess the effectiveness of the described administration regimens. Following these studies, a comparison between single dose administration versus multiple dose administration will be performed, with a dose being defined as 100 ml injection of the previously optimized concentrations in pfus. Efficacy studies will consist of the treatment of tumor xenografts following the established adenoviral concentrations and treatment regimen for 3 to 5 days. Efficacy will be assessed by the reduction in tumor size. Tumor size will be determined by the direct measurement of tumor diameters.

Ad-mda7 treated tumors will be evaluated for expression of MDA-7 protein and apoptosis induction. Immunohistochemical detection of MDA-7 and TUNEL assay detection of apoptosis will be used to evaluate the efficacy of Ad-mda7 treatment at the cellular level. An MDA-7 antibody that specifically recognizes MDA-7 protein will be employed for immunohistochemistry procedures. Endothelial cells in the melanoma xenografts will be detected with antibodies directed against mouse CD-31. Areas of the tumor sections with high numbers of capiallaries and small venules will be found by scanning the sections at low power (x40 and x100). In these areas individual vessels will be counted in x200 magnification fields, and average scores recorded for the treated and untreated tumor samples. This method has been used to compare blood distribution and density in human xenografts in nude mice (Yoneda *et al.,* 1998).

### EXAMPLE 10: MODULATION OF GROWTH FACTORS DURING ECTOPIC EXPRESSION OF MDA-7

Because it has been hypothesized that MDA-7 has an autocrine/paracrine activity, the effect of Ad-mda7 on melanoma cells will be evaluated with respect to the secretion of factors involved in the progression of melanoma. ELISA assays will be used to address the release of these soluble mediators, such as different types of TGF-β1, IL-8, IL-10, and bFGF. Melanoma cells lines and normal cells will be treated with Ad-mda7, Ad-luc, or diluent control and then monitored for modulation of growth factor levels in culture supernatant after 24-48 hours. Immunoblotting on the lysates may also be performed at various times post-treatment.

### EXAMPLE 11: AD-MDA7 ENHANCES ACTIVITY OF HERCEPTIN

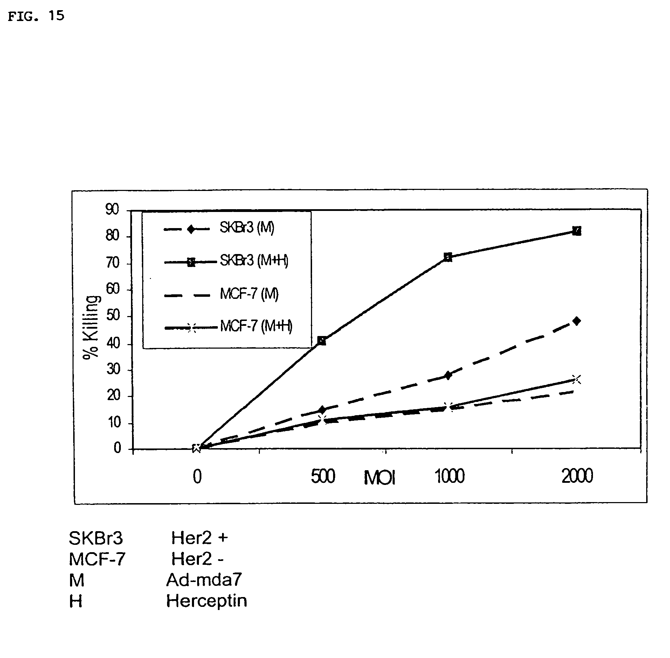

The breast cancer SkBr3 (Her2+) and MCF-7 (Her2-) cell lines were both obtained from ATCC. Cells were plated at a density of 1000 cells/well in Nunc 2-chamber slides and propagated in DMEM medium with 10% FBS. The following day, the cells were left untreated or treated with Ad-mda7 at (increasing MOIs: 0, 500, 1000 and 2000 vp/cell) without (M series) or with Herceptin (M+H series) at a final concentration of I µg/mL. The cells were washed after 3 hours and growth media (with or without Herceptin, as indicated) was replaced. Three days later viable cells were counted using the trypan blue exclusion assay (average of 3-4 fields) and plotted as shown in FIG. 15. Herceptin alone yields approximately 12% dead cells in both cell lines. However, Ad-mda7 appeared to enhance the killing effect of Herceptin in breast cancer cell lines.

### REFERENCES

The following references, to the extent that they provide exemplary procedural or other details supplementary to those set forth herein, are specifically incorporated herein by reference.
U.S. Patent 4,682,195, issued July 21, 1987.
U.S. Patent 4,683,202, issued July 28, 1987.
U.S. Patent 4.797,368, issued Jan. 10, 1989.
U.S. Patent 5,139,941, issued Aug. 18, 1992.
U.S. Patent 5,399,363, issued March 21, 1995.
U.S. Patent 5,466,468, issued Nov. 14, 1995.
U.S. Patent 5,543,158, issued Aug. 6, 1996.
U.S. Patent 5,633,016, issued May 27, 1997.
U.S. Patent 5,641,515, issued June 24, 1997.
U.S. Patent 5,645,897, issued July 8, 1997.
U.S. Patent 5,705,629, issued Jan. 6, 1998.
U.S. Patent 5,739,169, issued April 14, 1998.
U.S. Patent 5,798,339, issued Aug. 25, 1998.
U.S. Patent 5,824,311, issued Oct. 20, 1998.
U.S. Patent 5,824,348, issued Oct. 20, 1998.
U.S. Patent 5,830,880, issued Nov. 3, 1998.
U.S. Patent 5,846,225, issued Dec. 8, 1998.
U.S. Patent 5,846,233, issued Dec. 8, 1998.
U.S. Patent 5,846,945, issued Dec. 8, 1998.
U.S. Patent. 5.801,005, issued Sept. 1, 1998.
EP 266,032
WO 9828425
WO 9807408
WO 9511986

Aksentijevich et al. Human Gene Ther. 7:1111, 1996.
Arap et al., Cancer Res., 55:1351-1354, 1995.
Angel et al., Cell, 49:729, 1987b.
Angel et al., Mol. Cell. Biol., 7:2256, 1987a.
Atchison and Perry, Cell, 46:253, 1986.
Atchison and Perry, Cell, 48:121, 1987.
Austin-Ward and Villaseca, Rev. Med Chil., 126:838-45, 1998.
Baichwal and Sugden, In: Gene Transfer, Kucherlapati R, ed., New York, Plenum Press, pp. 117-148, 1986.
Baichwal et al., "Vectors for gene transfer derived from animal DNA viruses: Transient and stable expression of transferred genes," In: Gene transfer, Kucherlapati R, ed., New York: Plenum Press, pp. 117-148, 1986.
Bajorin et al., Proc. Annu. Meet. Am. Soc. Clin. Oncol., 7:A967, 1988.
Bakhshi et al., Cell, 41:899-906, 1985.
Banerji et al., Cell, 35:729, 1983.
Bedzyk et al., J. Biol. Chem., 265:18615, 1990
Benvenisty and Neshif, Proc. Nat. Acad. Sci. USA, 83:9551-9555, 1986.
Berkhout et al., Cell, 59:273, 1989.
Blanar et al., EMBO J., 8:1139, 1989.
Bodine and Ley, EMBD J., 6:2997, 1987.
Bonavida et al., Int J Oncol, 15:793-802, 1999.
Bonavida et al., Proc Nat'l Acad Sci USA. 97:1754-9, 2000.
Boshart et al., Cell, 41:521, 1985.
Bosze et al., EMBOJ., 5:1615, 1986.
Braddock et al., Cell, 58:269. 1989.
Brizel, Semin. Radiat. Oncol., 8(4 Suppl. 1):17-20, 1998.
Bukowski et al., Clin. Cancer Res., 4(10):2337-47, 1998.
Bulla and Siddiqui, J. Virol., 62:1437, 1986.
Caldas et al., Nat. Genet., 8:27-32, 1994.
Caley et al., J Virol., 71(4):3031-8, 1997.
Campbell and Villarreal, Mol. Cell. Biol., 8:1993, 1988.
Campere and Tilghman, Genes and Dev., 3:537, 1989.
Campo et al., Nature, 303:77, 1983.
Celander and Haseltine, J Virology, 61:269, 1987.
Celander et al., J. Virology, 62:1314, 1988.
Chandler et al., Cell, 33:489, 1983.
Chang et al., Hepatology, 14:134A, 1991.
Chang et al., Mol. Cell. Biol., 9:2153, 1989.
Chatterjee et al., Proc. Nat'l Acad. Sci. USA., 86:9114. 1989.
Chaudhary et al., Proc. Nat'l Acad. Sci., 87:9491, 1990
Chen and Okayama, Mol. Cell Biol., 7:2745-2752, 1987.
Chen and Okayama, Mol. Cell Biol., 7:2745-2752, 1987.
Cheng et al., Cancer Res., 54:5547-5551, 1994.
Choi et al., Cell, 53:519, 1988.
Christodoulides et al., Microbiology, 144(Pt 11):3027-37, 1998.
Clark et al., Human Gene Therapy, 6:1329-1341, 1995.
Cleary et al., J. Exp. Med., 164:315-20, 1986.
Cleary and Sklar, Proc. Nat'l. Acad. Sci. USA, 82:7439-43, 1985.
Coffin, "Retroviridae and their replication," In: Virology, Fields et al. (eds.), New York: Raven Press, pp. 1437-1500, 1990.
Cohen, Cancer Biother. Radiopharm., 14(1):1-4, 1999.
Cohen et al., Proc. Nat'l Acad. Sci. USA 75:472-476, 1978.
Costa et al., Mol. Cell. Biol., 8:81, 1988.
Couch et al., Am. Rev. Resp. Dis., 88:394-403, 1963.
Coupar et al., Gene, 68:1-10, 1988.
Coupar et al., Gene, 68:1-10, 1988.
Cripe et al., EMBO J., 6:3745, 1987.
Culotta and Hamer, Mol. Cell. Biol., 9:1376, 1989.
Culver et al., Science, 256:1550-1552, 1992.
Curran, Semin. Radial. Oncol., 8(4 Suppl. 1):2-4, 1998.
Dandolo et al., J Virology, 47:55, 1983.
Davidson et al., J Immunother., 21:389-98, 1998.
Davis et al., J. Virol. 70:3781-3787, 1996.
De Villiers et al., Nature, 312:242, 1984.
Deschamps et al., Science, 230:1174, 1985.
Dillman, Cancer Biother. Radiopharm., 14(1):5-10, 1999.
Dragovich et al., Oncogene, 17:3207-3213, 1998
Dubensky et al., Proc. Nat'l Acad Sci. USA, 81:7529-7533, 1984.
Edbrooke et al., Mol. Cell. Biol., 9:1908, 1989.
Edlund et al., Science, 230:912, 1985.
el-Kareh, Secomb, Crit. Rev. Biomed. Eng., 25:503-71, 1997.
Erlandsson, Cancer Genet. Cytogenet, 104:1-18, 1998.
Fechheimer et al., Proc. Nat'l Acad. Sci. USA, 84:8463-8467, 1987.
Felgner et al. Proc. Nat'l Acad. Sci. USA 84:7413, 1987.
Feng and Holland, Nature, 334:6178, 1988.
Ferkol et al., FASEB J., 7:1081-1091, 1993.
Firak and Subramanian, Mol. Cell. BioL, 6:3667, 1986.
Flotte et al.. Proc. Nat'l Acad. Sci. USA, 90:10613-10617, 1993.
Flotte et al., Gene Therapy, 2:29-37, 1995.
Flotte et al., Am. J. Respir. Cell Mol. Biol., 7:349-356, 1992.
Forster and Symons, Cell, 49:211-220, 1987.
Fraley et al., Proc. Nat'l Acad. Sci. USA, 76:3348-3352, 1979.
Friedmann, Science, 244:1275-1281, 1989.
Froehler et al., Nucleic Acids Res., 14:5399-407, 1986.
Frohman, In: PCR Protocols: A Guide To Methods And Applications, Academic Press, N.Y., 1990.
Fujita et al., Cell, 49:357, 1987.
Fulci et al.. Brain Pathol., 8(4):599-613, 1998.
Gabizon et al., Cancer Res., 50:6371-8, 1990.
Gerlach et al., Nature (London), 328:802-805, 1987.
Gertig and Hunter, Semin. Cancer Biol., 8(4):285-298, 1997.
Ghosh and Bachhawat, "Targeting of liposomes to hepatocytes," In: Liver Diseases, Targeted Diagnosis and Therapy Using Specific Receptors and Ligands," Wu G. and C. Wu ed. New York: Marcel Dekker, pp. 87-104, 1991.
Ghosh and Bachhawat, In: Liver diseases, targeted diagnosis and therapy using specific receptors and ligands, (Wu G, Wu C ed.), New York: Marcel Dekker, pp. 87-104, 1991.
Ghosh-Choudhury et al., EMBO J., 6:1733-1739, 1987.
Gilles et al., Cell, 33:717, 1983.
Gliniak et al., Cancer Res. 59:6153-8, 1999.
Gloss et al., EMBO J., 6:3735, 1987.
Godbout et al., Mol. Cell. Biol., 8:1169, 1988.
Gomez-Foix et al., J Biol. Chem., 267:25129-25134, 1992.
Goodbourn and Maniatis, Proc. Nat 'l Acad. Sci. USA, 85:1447, 1988.
Gopal, Mol. Cell Biol., 5:1188-1190, 1985.
Graham and Prevec, "Adenovirus-based expression vectors and recombinant vaccines," Biotechnology, 20:363-390, 1992.
Graham and Prevec, "Manipulation of adenovirus vector," In: Methods in Molecular Biology: Gene Transfer and Expression Protocol, E.J. Murray (ed.), Clifton, NJ: Humana Press, 7:109-128, 1991.
Graham and Van Der Eb, Virology, 52:456-467, 1973.
Graham et al., J Gen. Virol., 36:59-72, 1977.
Graham, Virology, 163(2):614-7, 1988.
Greene et al., Immunology Today, 10:272, 1989.
Grosschedl and Baltimore, Cell, 41:885, 1985.
Grunhaus and Horwitz, Seminar in Virology, 3:237-252, 1992.
Han et al., Genes Dev., 10(4):461-77, 1996.
Hanibuchi et al., Int. J. Cancer, 78:480-5, 1998.
Harland and Weintraub, J Cell Biol., 101:1094-1099, 1985.
Hartmann et al., Br. J Cancer, 73(8):896-901, 1996.
Hartmann et al., Int. J. Cancer, 67(3):313-317, 1996.
Haslinger and Karin, Proc. Nat 'l Acad. Sci. USA., 82:8572, 1985.
Hauber and Cullen, J. Virology, 62:673, 1988.
Heller et al., Cancer 77:964-71, 1996.
Hellstrand et al., Acta. Oncol., 37:347-53, 1998.
Hen et al., Nature, 321:249, 1986.
Hensel et al., Lymphokine Res., 8:347, 1989.
Hermonat and Muzyczka, Proc. Nat'l. Acad Sci. USA, 81:6466-6470, 1984.
Herr and Clarke, Cell, 45:461, 1986.
Hersdorffer et al., DNA Cell Biol., 9:713-723, 1990.
Herz and Gerard, Proc. Nat'l Acad Sci. USA 90:2812-2816, 1993.
Hirochika et al., J. Virol., 61:2599, 1987.
Hirsch et al., Mol. Cell. Biol., 10:1959, 1990.
Ho et al., Cancer, 83:1894-907, 1998.
Hofmann et al., Crit Rev Ther Drug Carrier Syst. 16:523-69, 1999.
Holbrook et al., Virology, 157:211, 1987.
Hollstein et al., Science 253:49-53, 1991.
Horlick and Benfield. Mol. Cell. Biol., 9:2396, 1989.
Horwich et al., J. Virol., 64:642-650, 1990.
Huang et al., Cell, 27:245, 1981.
Hui and Hashimoto, Infect. Immun., 66:5329-36, 1998.
Hussussian et al., Nature Genetics, 15-21, 1994.
Hwang et al., Mol. Cell. Biol., 10:585, 1990.
Imagawa et al., Cell, 51:251, 1987.
Imbra and Karin, Nature, 323:555, 1986.
Imler et al., Mol. Cell. Biol., 7:2558, 1987.
Imperiale and Nevins, Mol. Cell. Biol., 4:875, 1984.
Irie & Morton, Proc. Nat'l Acad. Sci. USA 83:8694-8698, 1986
Irie et al., "Melanoma gangliosides and human monoclonal antibody," In: Human Tumor Antigens and Specific Tumor Therapy, Metzgar & Mitchell (eds.), Alan R. Liss, Inc., New York, pp. 115-126, 1989.
Jakobovits et al., Mol. Cell. Biol., 8:2555, 1988.
Jameel and Siddiqui, Mol. Cell. Biol., 6:710, 1986.
Jaynes et al., Mol. Cell. Biol., 8:62, 1988.
Jiang et al., Oncogene, 11:2477-2486, 1995.
Jiang et al., Proc. Natl Acad. Sci. USA, 93:9160-9165, 1996.
Johnson and Hamdy, Oncol. Rep., 5:553-557, 1998.
Johnson et al., Mol. Cell. Biol., 9:3393, 1989.
Jones and Shenk, Cell, 13:181-188, 1978.
Joyce, Nature, 338:217-244, 1989.
Ju et al., Gene Ther., 7:329-38, 2000.
Kadesch and Berg, Mol. Cell. Biol., 6:2593, 1986.
Kamb et al., Nature Genetics, 8:22-26, 1994.
Kamb et al., Science, 2674:436-440, 1994.
Kaneda et al., Science, 243:375-378, 1989.
Kaneda et al., Science, 243:375-378, 1989.
Kaplitt et al., Nature Genetics, 8:148-154, 1994.
Karin et al., Mol. Cell. Biol., 7:606, 1987.
Karlsson et al., EMBO J., 5:2377-2385, 1986.
Katinka et al., Cell, 20:393, 1980.
Katinka et al., Nature, 290:720, 1981.
Kato et al., J. Biol. Chem., 266:3361-3364, 1991.
Kawamoto et al., Mol. Cell. Biol., 8:267, 1988.
Keane et al., Cancer Res. 59:734-41, 1999.
Kerr et al., Br. J. Cancer, 26:239-57, 1972.
Kiledjian et al., Mol. Cell. Biol., 8:145, 1988.
Klamut et al., Mol. Cell. Biol., 10:193, 1990.
Klein et al., Nature, 327:70-73, 1987.
Klein et al., Nature, 327:70-73, 1987.
Koch et al., Mol. Cell. Biol., 9:303, 1989.
Kolmel, J. Neurooncol., 38:121-125, 1998.
Kotin et al., Proc. Nat'l Acad. Sci. USA, 87:2211-2215, 1990.
Kriegler and Botchan, In: Eukaryotic Viral Vectors, Y. Gluzman, ed., Cold Spring Harbor: Cold Spring Harbor Laboratory, NY, 1982.
Kriegler and Botchan, Mol. Cell. Biol., 3:325, 1983.
Kriegler et al., Cell, 38:483. 1984a.
Kriegler et al., Cell, 53:45, 1988.
Kriegler et al., In: Cancer Cells 2/Oncogenes and Viral Genes, Van de Woude et al. eds, Cold Spring Harbor, Cold Spring Harbor Laboratory, 1984b.
Kriegler et al., In: Gene Expression, D. Hamer and M. Rosenberg, eds., New York: Alan R. Liss, 1983.
Kuhl et al., Cell, 50:1057, 1987.
Kunz et al., Nucl. Acids Res., 17:1121, 1989.
LaFace et al., Virology, 162:483-486, 1988.
Larsen et al., Proc. Nat'l Acad. Sci. USA., 83:8283, 1986.
Laspia et al., Cell, 59:283, 1989.
Latimer et al., Mol. Cell. Biol., 10:760, 1990.
Laughlin et al., J. Virol., 60:515-524, 1986.
Le Gal La Salle et al., Science, 259:988-990, 1993.
Lebkowski et al., Mol. Cell. Biol., 8:3988-3996, 1988.
Lee et al., Mol. Endocrinol., 2:404-411, 1988.
Lee et al., Nature, 294:228, 1981.
Levine et al., Nature, 351:453-456, 1991.
Levinson et al., Nature, 295:79, 1982.
Levrero et al., Gene, 101:195-202, 1991.
Liebermann et al., Int. J. Oncol., 12:685-700, 1998.
Lin et al., Mol. Cell. Biol., 10:850, 1990.
Liu et al. J. Biol. Chem. 270:24864, 1995.
Luo et al., Blood, 82 (Supp.):1,303A, 1994.
Luria et al., EMBO J., 6:3307, 1987.
Lusky and Botchan, Proc. Nat'l Acad. Sci. USA., 83:3609, 1986.
Macejak and Sarnow, Nature, 353:90-94, 1991.
Magi-Galluzzi et al., Anal. Quant. Cytol. Histol., 20:343-350, 1998.
Majors and Varmus, Proc. Nat'l Acad. Sci. USA., 80:5866, 1983.
Mangray and King, Front Biosci., 3:D1148-1160, 1998.
Mann et al., Cell, 33:153-159, 1983.
Marin, et al., Oncogene, 12(11):2259-66, 1996.
Markowitz et al., J. Virol., 62:1120-1124, 1988.
Marsters SA et al., Recent Prog Horm Res 54:225-34, 1999.
Mayer, Cancer Metastasis Rev., 17:211-8, 1998.
McCarty et al., J Virol., 65:2936-2945, 1991.
McGrory et al., Virology, 163:614-7, 1988.
McLaughlin et al., J. Virol., 62:1963-1973, 1988.
McNeall et al., Gene, 76:81, 1989.
Michel and Westhof, J Mol. Biol., 216:585-610, 1990.
Miksicek et al., Cell, 46:203, 1986.
Minta, Kao, and Tsien, J Biol Chem. 264:8171-8, 1989.
Mitchell et al., Ann. N. Y Acad. Sci., 690:153-166, 1993.
Mitchell et al., J. Clin. Oncol,. 8:856-859, 1990.
Miyashita and Reed, Cell 80:293-299, 1995.
Mordacq and Linzer, Genes and Dev., 3:760, 1989.
Moreau et al., Nucl. Acids Res., 9:6047, 1981.
Mori et al., Cancer Res., 54:3396-3397, 1994.
Morton D. L., and Ravindranath, M. H. Current concepts concerning melanoma vaccines. In Tumor Immunology, Dalgleish AG (ed.), London: Cambridge University Press, 1-55, 1996.
Morton et al., Ann. Surg. 216: 463-482, 1992.
Mougin et al., Ann. Biol. Clin. (Paris), 56:21-28, 1998.
Muesing et al., Cell, 48:691, 1987.
Mumby and Walter, Cell Regul., 2:589-598, 1991.
Muzyczka, Curr. Top. Microbiol. Immunol., 158:97-129, 1992.
Natoli et al., Biochem. Pharmacol., 56(8):915-920, 1998.
Ng et al., Nuc. Acids Res., 17:601, 1989.
Nicolas and Rubenstein, In: Vectors: A survey of molecular cloning vectors and their uses, Rodriguez and Denhardt (eds.), Stoneham: Butterworth, pp. 493-513, 1988.
Nicolau and Sene, Biochim. Biophys. Acta, 721:185-190, 1982.
Nicolau et al., Methods Enzymol., 149:157-176, 1987.
Nobri et al., Nature, 368:753-756, 1995.
Ochi et al., Am. J. Gastroenterol., 93:1366-1368, 1998.
Ohara, Gan. To. Kagaku. Ryoho., 25:823-8, 1998.
Ohi et al., Gene, 89L:279-282, 1990.
Okamoto et al., Proc. Nat'l Acad. Sci. USA, 91:11045-11049, 1994.
Ondek et al., EMBO J.. 6:1017, 1987.
Orlow et al., Cancer Res., 54:2848-2851, 1994.
Omitz et al., Mol. Cell. Biol., 7:3466, 1987.
Palmiter et al., Nature, 300:611, 1982.
Paskind et al., Virology, 67:242-248, 1975.
Paskind et al., Virology, 67:242-248, 1975.
Pech et al., Mol. Cell. Biol., 9:396, 1989.
Pelletier and Sonenberg, Nature, 334:320-325, 1988.
Perales et al., Proc. Nat'l Acad. Sci. 91:4086-4090, 1994.
Perez-Stable and Constantini, Mol. Cell. Biol., 10:1116, 1990.
Philip et al. J. Biol. Chem., 268:16087, 1993.
Picard and Schaffner, Nature, 307:83, 1984.
Pietras et al., Oncogene, 17:2235-49, 1998.
Pinkert et al., Genes and Dev., 1:268, 1987.
Ponta et al., Proc. Nat 'l Acad. Sci. USA. , 82:1020, 1985.
Porton et al., Mol. Cell. Biol., 10:1076, 1990.
Potter et al., Proc. Nat'l Acad. Sci. USA, 81:7161-7165, 1984.
Remington's Pharmaceutical Sciences, 15th ed., pages 1035-1038 and 1570-1580, Mack Publishing Company, Easton, Pa, 1980.
Qin et al., Proc. Nat'l Acad Sci. USA, 95(24):1411-6, 1998.
Queen and Baltimore, Cell, 35:741, 1983.
Quinn et al., Mol. Cell. Biol., 9:4713, 1989.
Racher et al.. Biotechnology Techniques, 9:169-174, 1995.
Ragot el al., Nature, 361:647-650, 1993.
Ravindranath, M.H. and Morton, D.L. Intern. Rev. lmmunol. 7: 303-329, 1991.
Redondo et al., Science, 247:1225, 1990.
Reed, Semin Hematol., 34(4 Suppl. 5):9-19, 1997.
Reed et al., Adv. Exp. Med. Biol., 406:99-112, 1996.
Reinhold-Hurek and Shub, Nature, 357:173-176, 1992.
Reisman and Rotter, Mol. Cell. Biol., 9:3571, 1989.
Renan, Radiother. Oncol., 19:197-218, 1990.
Resendez Jr. et al., Mol. Cell. Biol., 8:4579, 1988.
Rich et al., Hum. Gene Ther., 4:461-476, 1993.
Ridgeway, In: Vectors: A survey of molecular cloning vectors and their uses, Rodriguez RL. Denhardt DT, ed., Stoneham:Butterworth, pp. 467-492. 1988.
Rippe et al., Mol. Cell Biol., 10:689-695, 1990.
Rittling et al., Nucl. Acids Res., 17:1619, 1989.
Rosen et al., Cell, 41:813, 1988.
Rosenberg et al., Ann. Surg., 210:474, 1989.
Rosenberg et al., N. Engl. J. Med., 319:1676, 1988.
Rosenfeld et al., Science, 252:431-434, 1991.
Rosenfeld et al., Cell, 68:143-155, 1992.
Rosse et al., Nature 391:496-499, 1998.
Roux et al., Proc. Nat'l Acad. Sci. USA. 86:9079-9083, 1989.
Rovere et al., J Leukoc Biol. 66:345-9, 1999.
Sakai et al., Genes and Dev., 2:1144, 1988.
Sambrook et al., In: Molecular Cloning: A Laboratory Manual, 2nd Ed., Cold Spring Harbor Press, Cold Spring Harbor, NY, 1989.
Samulski et al., J. Virol., 63:3822-3828, 1989.
Samulski et al., EMBO J., 10:3941-3950, 1991.
Sarver et al., Science, 247:1222-1225, 1990.
Satake et al., J Virology, 62:970, 1988.
Scanlon et al., Proc. Nat'1 Acad. Sci. USA, 88:10591-10595, 1991.
Schaffner et al., J Mol. Biol., 201:81, 1988.
Searle et al., Mol. Cell. Biol., 5:1480, 1985.
Sedlak et al., Proc. Nat 'l. Acad. Sci. USA. 92:7834-8, 1995.
Serrano et al., Nature, 366:704-707, 1993.
Serrano et al., Science, 267:249-252, 1995.
Sharp and Marciniak, Cell, 59:229, 1989.
Shaul and Ben-Levy, EMBO J., 6:1913, 1987.
Shelling and Smith, Gene Therapy, 1:165-169, 1994.
Sherman et al., Mol. Cell. Biol., 9:50, 1989.
Shimizu et al., Nuture 399:483-487, 1999.
Sleigh and Lockett, J. EMBO, 4:3831, 1985.
Soddu and Sacchi, Cytokines Cell Mol. Ther., 4:177-185, 1998.
Solodin et al., Biochemistry 34:13537, 1995.
Solyanik et al., Cell Prolif., 28:263-278, 1995.
Spalholz et al., Cell, 42:183, 1985.
Spandau and Lee, J Virology, 62:427, 1988.
Spandidos and Wilkie, EMBO J., 2:1193, 1983.
Steinman et al., Hum Immunol. 60:562-7, 1999.
Stephens and Hentschel, Biochem. J., 248:1, 1987.
Stokke et al., Cell Prolif., 30(5):197-218, 1997.
Stratford-Perricaudet and Perricaudet, "Gene transfer into animals: the promise of adenovirus," In: Human Gene Transfer, Eds, O. Cohen-Haguenauer and M. Boiron, Editions John Libbey Eurotext, France. p. 51-61, 1991.
Stratford-Perricaudet et al., Hum. Gene Ther., 1:241-256, 1990.
Stuart et al., Nature, 317:828, 1985.
Su et al., Proc. Nat'l Acad. Sci, USA 1998, 95: 14400-14405.
Sullivan and Peterlin, Mol. Cell. Biol., 7:3315, 1987.
Swartzendruber and Lehman, J. Cell. Physiology, 85:179, 1975.
Tacket et al., Vaccine, 17:2826-9, 1999.
Takebe et al., Mol. Cell. Biol., 8:466, 1988.
Tavernier et al., Nature, 301:634, 1983.
Taylor and Kingston, Mol. Cell. Biol., 10:165, 1990a.
Taylor and Kingston, Mol. Cell. Biol., 10:176, 1990b.
Taylor et al., J. Biol. Chem., 264:1 S 160, 1989.
Temin, In: Gene Transfer, Kucherlapati (ed.), New York: Plenum Press, pp. 149-188, 1986.
Templeton et al., Nat. Biotechnol., 15:647-52, 1997.
Thierry et al. Proc. Nat'l Acad Sci. USA, 92(21):9742-6, 1995.
Thiesen et al., J Virology, 62:614, 1988.
Top et al., J. Infect. Dis., 124:155-160, 1971.
Tratschin et al., Mol. Cell. Biol., 5:32581-3260, 1985.
Tratschin et al., Mol. Cell. Biol., 4:2072-2081, 1984.
Treisman, Cell, 42:889, 1985.
Tronche et al., Mol. Biol. Med., 7:173, 1990.
Trudel and Constantini, Genes and Dev., 6:954, 1987.
Tsujimoto and Croce, Proc. Natl. Acad. Sci. USA, 83:5214-8, 1986.
Tsujimoto et al., Science, 228:1440-3, 1985.
Tsukamoto et al., Nature Genetics 9:243, 1995.
Tur-Kaspa el al., Mol. Cell Biol., 6:716-718, 1986.
Tyndall et al., Nuc. Acids. Res., 9:6231, 1981.
Vannice and Levinson, J. Virology, 62:1305, 1988.
Vasseur et al., Proc. Nat'l Acad. Sci. USA., 77:1068, 1980.
Vogelstein and Kinzler, Cell, 70:523-526, 1992.
Wagner et al.. Proc. Nat'I Acad. Sci. 87:3410-3414, 1990.
Walsh et al., Proc. Nat'l Acad. Sci. USA, 89:7257-7261, 1994.
Walsh et al.. J Clin. Invest., 94:1440-1448, 1994.
Wang and Calame, Cell, 47:241, 1986.
Watanabe et al., Exp. Cell Res. 230:76-83, 1997.
Weber et al., Cell, 36:983, 1984.
Wei et al., Gene Therapy, 1:261-268, 1994.
Weinberg, Science, 254:1138-1145, 1991.
Weinberger et al. Mol. Cell. Biol., 8:988, 1984.
White, Genes Dev., 10:1-15, 1996.
Winoto and Baltimore, Cell, 59:649, 1989.
Wong et al., Gene, 10:87-94, 1980.
Wong et al., Gene, 10:87-94, 1980.
Wu and Wu, Adv. Drug Delivery Rev., 12:159-167, 1993.
Wu and Wu, Biochem., 27:887-892, 1988.
Wu and Wu, J. Biol. Chem., 262:4429-4432, 1987.
Yang et al., Proc. Nat'l Acad. Sci. USA, 87:9568-9572, 1990.
Yang et al., J. Virol., 68:4847-4856, 1994.
Yang, J and Huang, L, Gene Therapy 4:950-960, 1997.
Yoder et al., Blood, 82 (Supp.):1:347A, 1994.
Yoneda et al., J. Natl. Canc. Inst., 90:447-454, 1998.
Yutzey et al. Mol. Cell. Biol., 9:1397, 1989.
Zhou et al., Exp. Hematol. (NY), 21:928-933, 1993.
Zhou et al., J. Exp. Med., 179:1867-1875, 1994.
Zhu et al., Science 261:209 1993.

## Claims

1. Use of an effective amount of an expression cassette comprising a nucleic acid sequence encoding a human MDA-7 polypeptide under the control of a promoter operable in eukaryotic cells for the manufacture of a medicament for treating a human patient with cancer wherein the medicament is administered in combination with at least one other anticancer treatment, wherein the anticancer treatment is chemotherapy, wherein the chemotherapy comprises a DNA damaging agent, tamoxifen or Herceptin.

2. The use of claim 1, wherein the expression cassette is administered to the patient before, during, or after the other anti-cancer treatment.

3. The use of claim 1, wherein the expression cassette encodes a full-length human MDA-7 protein.

4. The use of claim 1, wherein the expression cassette further encodes a secretory signal.

5. The use of claim 1, wherein the cancer further comprises a tumor.

6. The use of claim 1, wherein the cancer is melanoma, non-small cell lung, small-cell lung, lung, hepatocarcinoma, retinoblastoma, astrocytoma, glioblastoma, leukemia, neuroblastoma, head, neck, breast, pancreatic, prostate, renal, bone, testicular, ovarian, mesothelioma, cervical, gastrointestinal, lymphoma, brain, colon or bladder.

7. The use of claim 1, wherein the expression cassette is an adenovirus vector.

8. The use of claim 7, wherein the adenovirus vector is administered at between about 10³ and about 10¹⁵ viral particles.

9. The use of claim 1, wherein the expression cassette is administered to the patient in a lipoplex.

10. The use of claim 9, wherein the lipoplex comprises DOTAP and at least one cholesterol, cholesterol derivative, or cholesterol mixture.

11. The use of claim 1, wherein administering is by injection of the expression cassette.

12. The use of claim 11, wherein the injection is local, regional, or distal to the cancer.

13. The use of claim 1, wherein administering is via continuous infusion, intratumoral injection, or intravenous injection. ,

14. The use of claim 1, wherein the promoter is CMV IE, dectin-1, dectin-2, human CD11c, F4/80, SM22, or MHC class II promoter.

15. The use of claim 11, wherein administering comprises multiple injections.

16. The use of claim 1, wherein the expression cassette is administered to the tumor bed prior to or after resection of the tumor.

17. The use of claim 1, wherein the expression cassette is administered to the tumor bed both prior to and after tumor resection.

18. The use of claim 1, wherein the nucleic acid sequence encodes amino acids from about 182 to about 206 of SEQ ID NO: 2.

19. The use of claim 1, wherein the nucleic acid sequence encodes amino acids from about 175 to about 206 of SEQ ID NO: 2.

20. The use method of claim 1, wherein the nucleic acid sequence encodes amino acids from about 150 to about 206 of SEQ ID NO: 2.

21. The use of claim 1, wherein the nucleic acid sequence encodes amino acids from about 100 to about 206 of SEQ ID NO: 2.

22. The use of claim 1, wherein the nucleic acid sequence encodes amino acids from about 49 to about 206 of SEQ ID NO: 2.

23. The use of claim 1, wherein the MDA-7 polypeptide is lacking a signal sequence from the full-length MDA-7 polypeptide sequence.

24. The use of claim 23, wherein the expression cassette further comprises a second nucleic acid sequence encoding a heterologous secretory signal.

25. The use of claim 24, wherein the secretory signal is further defined as a positively charged N-terminal region in combination with a hydrophobic core.

26. The use of claim 1, wherein the DNA damaging agent is adriamycin, fluorouracil (5FU), etoposide (VP-16), camptothecin. actinomycin-D, mitomycin C or cisplatin (CDDP).

27. The use of claim 27, wherein the DNA damaging agent is adriamycin.

28. A use of claim 1, wherein the nucleic acid sequence is administered in an amount effective to inhibit angiogenesis around the tumor.

## Patentansprüche

1. Verwendung einer wirksamen Menge einer Expressionskassette umfassend eine Nukleinsäuresequenz, die ein humanes MDA-7 Polypeptid unter der Kontrolle eines in eukaryotischen Zellen funktionsfähigen Promotors kodiert, zur Herstellung eines Medikaments zur Behandlung eines menschlichen Patienten mit Krebs, wobei das Medikament in Kombination mit mindestens einer anderen Anti-Krebs-Behandlung verabreicht wird, wobei die Anti-Krebs-Behandlung eine Chemotherapie ist, wobei die Chemotherapie ein DNA-schädigendes Mittel, Tamoxifen oder Herceptin umfasst.

2. Verwendung nach Anspruch 1, wobei die Expressionskassette dem Patienten vor, während oder nach der anderen Anti-Krebs-Behandlung verabreicht wird.

3. Verwendung nach Anspruch 1, wobei die Expressionskassette ein humanes MDA-7 Protein in voller Länge kodiert.

4. Verwendung nach Anspruch 1, wobei die Expressionkassette weiterhin ein sekretorisches Signal kodiert.

5. Verwendung nach Anspruch 1, wobei der Krebs weiterhin einen Tumor umfasst.

6. Verwendung nach Anspruch 1, wobei der Krebs ist Melanom, nichtkleinzelliger Lungenkrebs, kleinzelliger Lungenkrebs, Lungenkrebs, Leberkarzinom, Retinoblastom, Astrozytom, Glioblastom, Leukämie, Neuroblastom, Kopf-, Nacken-, Brust-, Pankreas-, Prostata-, Nieren-, Knochen-, Hoden-, Eierstock-, Mesotheliom, Zervikal-, Gastrointestinal-, Lymphom, Gehirn-, Darm- oder Blasenkrebs.

7. Verwendung nach Anspruch 1, wobei die Expressionskassette ein Adenovirus-Vektor ist.

8. Verwendung nach Anspruch 7, wobei der Adenovirus-Vektor mit zwischen etwa 10³ und etwa 10¹⁵ viralen Partikeln verabreicht wird.

9. Verwendung nach Anspruch 1, wobei die Expressionskassette dem Patienten in einem Lipoplex verabreicht wird.

10. Verwendung nach Anspruch 9, wobei der Lipoplex DOTAP und mindestens ein Cholesterin, Cholesterin-Derivat oder eine Cholesterin-Mischung umfasst.

11. Verwendung nach Anspruch 1, wobei das Verabreichen durch Injektion der Expressionkassette erfolgt.

12. Verwendung nach Anspruch 11, wobei die Injektion lokal, regional oder distal zum Krebs erfolgt.

13. Verwendung nach Anspruch 1, wobei das Verabreichen mittels kontinuierlicher Infusion, intratumoraler Injektion oder intravenöser Injektion erfolgt.

14. Verwendung nach Anspruch 1, wobei der Promoter ein CMV IE, Dektin-1, Dektin-2, humaner CD11c, F4/80, SM22 oder ein MHC Klasse 2 Promotor ist.

15. Verwendung nach Anspruch 11, wobei das Verabreichen mehrere Injektionen umfasst.

16. Verwendung nach Anspruch 1, wobei die Expressionskassette vor oder nach Resektion des Tumors in das Tumorbett verabreicht wird.

17. Verwendung nach Anspruch 1, wobei die Expressionskassette sowohl vor als auch nach Tumorresektion in das Tumorbett verabreicht wird.

18. Verwendung nach Anspruch 1, wobei die Nukleinsäuresequenz Aminosäuren von etwa 182 bis etwa 206 der SEQ ID NO: 2 kodiert.

19. Verwendung nach Anspruch 1, wobei die Nukleinsäuresequenz Aminosäuren von etwa 175 bis etwa 206 der SEQ ID NO: 2 kodiert.

20. Verwendungsverfahren nach Anspruch 1, wobei die Nukleinsäuresequenz Aminosäuren von etwa 150 bis etwa 206 der SEQ ID NO: 2 kodiert.

21. Verwendung nach Anspruch 1, wobei die Nukleinsäuresequenz Aminosäuren von etwa 100 bis etwa 206 der SEQ ID NO: 2 kodiert.

22. Verwendung nach Anspruch 1, wobei die Nukleinsäuresequenz Aminosäuren von etwa 49 bis etwa 206 der SEQ ID NO: 2 kodiert.

23. Verwendung nach Anspruch 1, wobei dem MDA-7-Polypeptid eine Signalsequenz der Volllänge-MDA-7-Polypeptidsequenz fehlt.

24. Verwendung nach Anspruch 23, wobei die Expressionskassette weiterhin eine zweite Nukleinsäuresequenz umfasst, die ein heterologes, sekretorisches Signal kodiert.

25. Verwendung nach Anspruch 24, wobei das sekretorische Signal weiterhin definiert ist als eine positiv geladene N-terminale Region in Kombination mit einem hydrophoben Kernbereich.

26. Verwendung nach Anspruch 1, wobei das DNA-schädigende Mittel Adriamycin, Fluorouracil (5FU), Etoposid (VP-16), Camptothecin, Actinomycin-D, Mitomycin C oder Cisplatin (CDDP) ist.

27. Verwendung nach Anspruch 27, wobei das DNA-schädigende Mittel Adriamycin ist.

28. Verwendung nach Anspruch 1, wobei die Nukleinsäuresequenz in einer Menge verabreicht wird, die wirksam ist, Angiogenese um den Tumor herum zu inhibieren.

## Revendications

1. Utilisation d'une quantité efficace d'une cassette d'expression comprenant une séquence d'acides nucléiques codant pour un polypeptide MDA-7 humain sous contrôle d'un promoteur opérable dans des cellules eucaryotes pour la fabrication d'un médicament pour traiter un patient humain atteint de cancer, dans laquelle le médicament est administré en association avec au moins un autre traitement anticancéreux, lequel traitement anticancéreux consiste en une chimiothérapie, cette chimiothérapie comprenant un agent occasionnant des dommages à l'ADN, du tamoxifène ou herceptine.

2. L'utilisation selon la revendication 1, dans laquelle la cassette d'expression est administrée au patient avant, pendant ou après l'autre traitement anti-cancéreux.

3. L'utilisation selon la revendication 1, dans laquelle la cassette d'expression code pour une protéine MDA-7 humaine présentant la totalité de sa longueur.

4. L'utilisation selon la revendication 1, dans laquelle la cassette d'expression code en outre pour un signal sécrétoire.

5. L'utilisation selon la revendication 1, dans laquelle le cancer présente en outre une tumeur

6. L'utilisation selon la revendication 1, dans laquelle le cancer est un mélanome, un cancer bronchique à grande cellules, un cancer bronchique à petite cellules, un cancer du poumon, un hépatocarcinome, un rétinoblastome, un astrocytome, un glioblastome, une leucémie, un neuroblastome, un cancer de la tête, de la nuque, du sein, du pancréas, de la prostate, du rein, des os, des testicules, des ovaires un mésothéliome, un cancer cervical, gastrointestinal, un lymphome, un cancer du cerveau de colon ou de la vessie.

7. L'utilisation selon la revendication 1, dans laquelle la cassette d'expression est un vecteur d'adénovirus.

8. L'utilisation selon la revendication 7, dans laquelle le vecteur d'adénovirus est administré à un niveau compris entre environ 10³ et environ 10¹⁵ particules virales

9. L'utilisation selon la revendication 1, dans laquelle la cassette d'expression est administrée au patient dans un lipoplex.

10. L'utilisation selon la revendication 9, dans laquelle le lipoplex comprend de la DOTAP et au moins du cholestérol, un dérivé du cholestérol ou un mélange de cholestérols,

11. L'utilisation selon la revendication 1, dans laquelle l'administration est réalisée par injection de la cassette d'expression.

12. L'utilisation selon la revendication 11, dans laquelle l'injection est une injection locale, régionale ou distale par rapport au cancer.

13. L'utilisation selon la revendication 1, dans laquelle l'administration est faite par infusion continue, par injection intratumorale ou par injection intraveineuse.

14. L'utilisation selon la revendication 1, dans laquelle le promoteur consiste en CMV IE, dectine-1, dectine-2, CD11c humaine, F4/80, SM22 ou un promoteur MHC classe II.

15. L'utilisation selon la revendication 11, dans laquelle l'administration comprend des injections multiples.

16. L'utilisation selon la revendication 1, dans laquelle la cassette d'expression est administrée au lit tumoral avant ou après résection de la tumeur.

17. L'utilisation selon la revendication 1, dans laquelle la cassette d'expression est administrée au lit tumoral à la fois avant et après résection de la tumeur.

18. L'utilisation selon la revendication 1, dans laquelle la séquence d'acides nucléiques code pour les acides aminés entre environ 182 et environ 206 de la SEQ ID N°2

19. L'utilisation selon la revendication 1, dans laquelle la séquence d'acides nucléiques code pour les acides aminés entre environ 175 et environ 206 de la SEQ ID N°2

20. L'utilisation selon la revendication 1, dans laquelle la séquence d'acides nucléiques code pour les acides aminés entre environ 150 et environ 206 de la SEQ ID N°2

21. L'utilisation selon la revendication 1, dans laquelle la séquence d'acides nucléiques code pour les acides aminés entre environ 100 et environ 206 de la SEQ ID N°2

22. L'utilisation selon la revendication 1, dans laquelle la séquence d'acides nucléiques code pour les acides aminés entre environ 49 et environ 206 de la SEQ ID N°2

23. L'utilisation selon la revendication 1, dans laquelle le polypeptide MDA-7 manque d'une séquence de signal de la séquence de polypeptide MDA-7 présentant la totalité de sa longueur.

24. L'utilisation selon la revendication 23, dans laquelle la cassette d'expression comprend en outre une deuxième séquences nucléiques codant pour un signal sécrétoire hétérologue.

25. L'utilisation selon la revendication 24, dans laquelle le signal sécrétoire est en outre défini comme une région N-terminale chargée positivement en association avec un noyau hydrophobe.

26. L'utilisation selon la revendication 1, dans laquelle l'agent occasionnant des dommages à l'ADN consiste en adriamycine, fluorouracyl (5FU), étoposide (VP-16), camptothécine, actinomycine-D, mitomycine C ou cisplatine (CDDP).

27. L'utilisation selon la revendication 27, dans laquelle l'agent occasionnant des dommages à l'ADN est l'adriamycine,

28. L'utilisation selon la revendication 1, dans laquelle la séquence d'acides nucléiques est administrée en une quantité efficace pour inhiber l'angiogénèse autour de la tumeur.
